# EUROPEAN PATENT APPLICATION

(11) **EP 4 840 956 A1**
(43) Date of publication of application: **18.11.2026**
(21) Application number: 24916744.6
(22) Date of filing: 26.12.2024
(51) Int. Cl.: C12N 15/11, C12N 5/10, A61K 31/713, A61K 48/00, A61P 37/08, C07H 21/02

(54) **PHARMACEUTICAL COMPOSITION FOR INHIBITING NFKBIZ GENE EXPRESSION, AND USE**

(30) Priority: 13.01.2024 CN 202410054022
(71) Applicant: Rehydration Therapeutics Co. Ltd., Shanghai 201811 (CN)
(72) Inventor: ZHANG, Chuan, Shanghai 200240 (CN); HUANG, Yangyang, Shanghai 200240 (CN); JIANG, Kai, Shanghai 200240 (CN); LIU, Yuhe, Shanghai 200240 (CN); CUI, Haonan, Shanghai 200240 (CN); ZHANG, Kaiqing, Shanghai 200240 (CN)
(74) Representative: Castell, Klaus
(86) International application number: PCT/CN2024/142554
(87) International publication number: WO 2025/148697

(57) **Abstract**

The present disclosure belongs to the field of biomedicine, and provides a pharmaceutical composition for inhibiting *NFKBIZ* gene expression, and use thereof. A double-stranded nucleic acid molecule capable of targeting and regulating *NFKBIZ* gene expression is designed. By means of verifying the gene regulation efficiency of nucleic acid molecules, multiple nucleic acid molecules capable of inhibiting *NFKBIZ* gene expression are obtained from screening, and efficient inhibition of IκB-ζ protein expression is achieved at the protein level. In addition, use of a pharmaceutical composition formed by the conjugation of the designed nucleic acid molecules and targeting ligands is verified in ocular disease treatment. The pharmaceutical composition can solve the problems with existing small molecule immunomodulatory drugs of low delivery efficiency and poor therapeutic effect, providing small nucleic acid drugs having tissue-targeted delivery functions, and has the advantages of long duration of efficiency and low side effects. In addition, the pharmaceutical composition has simple ingredients, is easy to synthesize, and has good prospects for translational applications.

## Description

The present disclosure claims priority to Chinese Patent Application No. 202410054022.X, filed with the China National Intellectual Property Administration on January 13, 2024 and entitled "PHARMACEUTICAL COMPOSITION FOR INHIBITING *NFKBIZ* GENE EXPRESSION, AND USE THEREOF", the disclosure of which is incorporated herein by reference in its entirety.

### TECHNICAL FIELD

The present disclosure relates to the technical field of biopharmaceuticals, and specifically relates to a pharmaceutical composition for inhibiting nuclear factor of kappa light polypeptide gene enhancer in B-cells inhibitor, zeta (*NFKBIZ*) gene expression and use thereof.

### BACKGROUND

IκB-ζ (nuclear factor of kappa light polypeptide gene enhancer in B-cells inhibitor, zeta) is a non-classical member of the IkappaB protein family. IκB-ζ is located in the cell nucleus and mainly regulates the expression of secondary response genes in the nuclear factor κB (NF-κB) signaling pathway. The NF-κB signaling pathway plays a key role in various cellular processes, including cell growth, differentiation and apoptosis, particularly in immune response and inflammatory reaction. IκB-ζ is encoded by the *NFKBIZ* gene. The activation or upregulated expression of the *NFKBIZ* gene can promote the expression and secretion of a series of cellular inflammatory factors downstream of the NF-κB signaling pathway. It extensively regulates the functions of various cells such as the mononuclear phagocyte system, NK cells, T cells, B cells and epithelial cells, and has been identified as a key regulatory gene in inflammatory processes and immune regulation. Ocular surface diseases, among the most common in ophthalmology, include dry eye disease, keratitis, conjunctivitis and blepharitis, all of which are associated with inflammation and can be treated by inhibiting the expression of inflammatory factors. Therefore, regulating the *NFKBIZ* gene expression is a potential therapeutic approach for the treatment of such diseases.

Small interfering RNA (siRNA), as an important effector molecule of RNA interference (RNAi) technology, induces sequence-specific degradation of target mRNA *in vivo* by double-stranded RNA (dsRNA), thereby resulting in gene silencing to varying degrees. This mechanism holds great potential for disease treatment. Compared to small-molecule drugs, siRNA has advantages such as abundant targets, low tendency to induce drug resistance, long-acting efficacy, and ease of synthesis and production. In contrast to antibodies, which face the challenges such as high costs, potential systemic toxicity and the formation of anti-drug antibody (ADA), siRNA has the advantages of longer-lasting efficacy and safety. In addition, siRNA drugs have been favored by an increasing number of pharmaceutical companies in recent years due to the higher likelihood of successful development and a faster, more streamlined research and development and manufacturing pipeline. Thus, the use of siRNA technology to target inflammation-related genes, such as *NFKBIZ*, holds promise for immune regulation. However, there are currently few reports on siRNAs targeting the *NFKBIZ* gene, and the knockdown efficiency is low, which fails to meet the requirements for efficient inhibition of *NFKBIZ* expression.

In addition, although several siRNA drugs have been approved for the treatment of liver-related diseases, there is still a need for the development of siRNA therapies for non-liver diseases. The eye is one of the most sophisticated organs in the human body. Due to its unique anatomical and physiological characteristics, topical ocular administration is the most common route of drug delivery, with eye drops, ocular ointments and ocular gels being the main dosage forms. For immune-related diseases, existing small-molecule immunomodulatory drugs in clinical practice have problems in drug delivery, such as poor water solubility, toxic and side effects associated with frequent and repeated administration, low bioavailability with only about 5% to 10% of the administrated dose reaching the target tissue, and poor patient compliance.

### SUMMARY

In view of this, an object of the present disclosure is to provide a pharmaceutical composition for inhibiting *NFKBIZ* gene expression, which is a conjugate formed from a targeting ligand and a nucleic acid molecule with gene regulatory function. By means of targeting an ocular tissue-specific protein using a targeting ligand, the pharmaceutical composition improves the uptake efficiency of the nucleic acid molecule by ocular surface cells, and efficiently downregulates the *NFKBIZ* gene expression, thereby effectively inhibiting the expression of IκB-ζ protein at the protein level. Ultimately, the pharmaceutical composition solves the problem of oligonucleotides drug delivery, improves the bioavailability of drugs, and achieves effective treatment of related diseases.

The present disclosure provides a pharmaceutical composition for inhibiting *NFKBIZ* gene expression, which includes a nucleic acid molecule inhibiting *NFKBIZ* gene expression and a targeting ligand targeting an ocular tissue-specific protein.

In some embodiments, the nucleic acid molecule includes at least one nucleotide sequence of the following items A and B:
A: a sense strand selected from at least one of the following numbered nucleotide sequences: SEQ ID NO: 1 to SEQ ID NO: 18, SEQ ID NO: 25 to SEQ ID NO: 49, SEQ ID NO: 51 to SEQ ID NO: 89, SEQ ID NO: 93 to SEQ ID NO: 108, SEQ ID NO: 113 to SEQ ID NO: 132, SEQ ID NO: 134 to SEQ ID NO: 135, SEQ ID NO: 138, SEQ ID NO: 143 to SEQ ID NO: 152, SEQ ID NO: 154 to SEQ ID NO: 161, SEQ ID NO: 169 to SEQ ID NO: 185, and SEQ ID NO: 421; and
   an antisense strand selected from at least one of the following numbered nucleotide sequences: SEQ ID NO: 211 to SEQ ID NO: 228, SEQ ID NO: 235 to SEQ ID NO: 259, SEQ ID NO: 261 to SEQ ID NO: 299, SEQ ID NO: 303 to SEQ ID NO: 318, SEQ ID NO: 323 to SEQ ID NO: 342, SEQ ID NO: 344 to SEQ ID NO: 345, SEQ ID NO: 348, SEQ ID NO: 353 to SEQ ID NO: 362, SEQ ID NO: 364 to SEQ ID NO: 371, SEQ ID NO: 379 to SEQ ID NO: 395, and SEQ ID NO: 422; and
B: nucleotide sequences having 2-3 consecutive or non-consecutive bases added, deleted or substituted based on the nucleotide sequences in item A, and target the *NFKBIZ* gene to exert an inhibitory effect.

In some embodiments, the sense strand or the antisense strand of the nucleic acid molecule further includes an overhang at an end.

In some embodiments, the overhang is 1-3nt in length.

In some embodiments, the nucleic acid molecule includes any one or more of the following modified nucleotides: deoxynucleotides, 3'-deoxythymidine (dT) nucleotides, 2'-O-methyl modified nucleotides, 2'-fluoro-modified nucleotides, 2'-deoxy-modified nucleotides, locked nucleic acids, conformationally restricted nucleotides, constrained ethyl nucleotides, abasic nucleotides, 2'-amino-modified nucleotides, 2'-O-allyl-modified nucleotides, 2'-C-alkyl-modified nucleotides, 2'-hydroxy-modified nucleotides, 2'-methoxyethyl-modified nucleotides, 2'-O-alkyl-modified nucleotides, morpholino nucleotides, phosphoramidate-modified nucleotides, nucleotides containing unnatural bases, tetrahydropyran-modified nucleotides, 1,5-anhydrohexitol-modified nucleotides, cyclohexenyl-modified nucleotides, nucleotides containing phosphorothioate linkages, nucleotides containing methylphosphonate linkages, nucleotides containing 5'-phosphate groups, and nucleotides containing 5'-phosphate mimics.

In some embodiments, the targeting ligand targeting the ocular tissue-specific protein includes at least one of: an aptamer targeting an ocular tissue-specific protein, a compound modified with boronic acid or a derivative thereof, or a compound modified with phenylboronic acid or a derivative thereof.

In some embodiments, the ocular tissue-specific protein includes at least one of: mucin, integrin, and CD44;
the mucin includes at least one of: MUC-1, MUC-4, and MUC-16;
the integrin includes at least one of: α_{V}β₁, α_{V}β₃, α_{V}β₆, α₅β₁, α₆β₁, α_{V}β₁, a_{V}β₅, α₆β₄, and α₁β₁.

In some embodiments, the aptamer targeting the mucin MUC-1 has the nucleotide sequence of SEQ ID NO: 423;
the aptamer targeting the mucin MUC-16 has the nucleotide sequence of SEQ ID NO: 424;
the aptamer targeting the integrin α_{V}β₃ has the nucleotide sequence of SEQ ID NO: 425; and
the aptamer targeting CD44 has the nucleotide sequence of SEQ ID NO: 426.

In some embodiments, the aptamer further includes a stability-enhancing modification; and
the stability-enhancing modification includes at least one of: phosphorothioate backbone modification, 2'-O-methyl modification, 2'-methoxyethyl modification, 2'-fluoro modification, inverted dT modification, dT modification, and terminal conjugation with polyethylene glycol.

In some embodiments, the compound modified with boronic acid or the derivative thereof, or the compound modified with phenylboronic acid or the derivative thereof, is a linear or branched compound terminally modified with boronic acid or phenylboronic acid or a derivative thereof;
the linear or branched compound is modified with 1-40 moieties of boronic acid or the derivative thereof, or phenylboronic acid or the derivative thereof;
in some embodiments, the compound modified with boronic acid or the derivative thereof, or the compound modified with phenylboronic acid or the derivative thereof, includes any one of formulas 1-10: 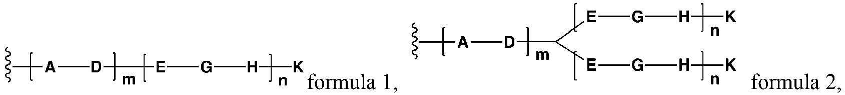 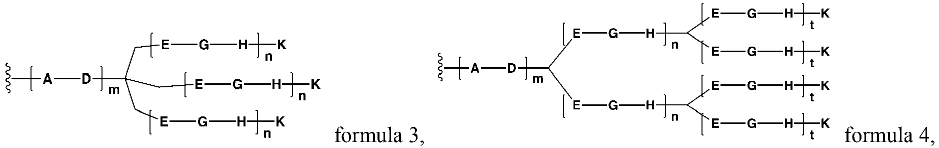 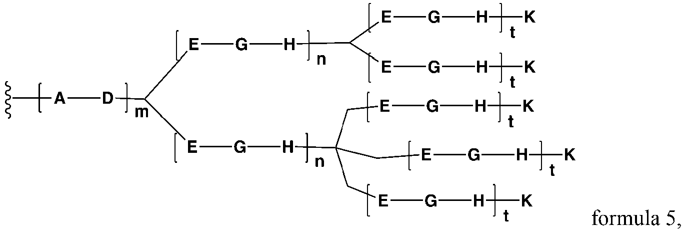 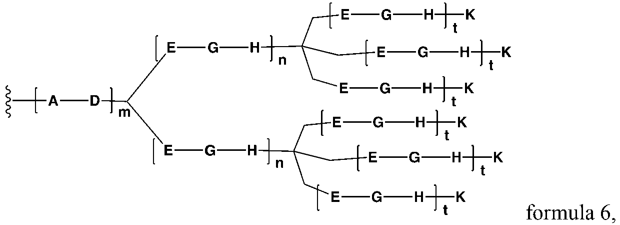 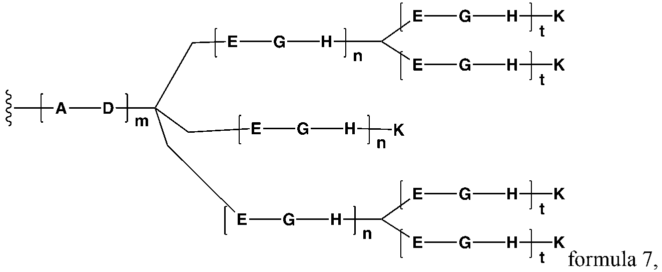 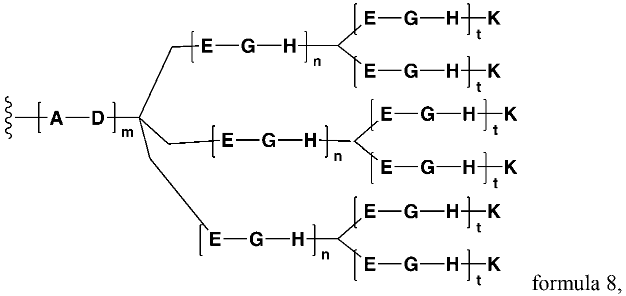 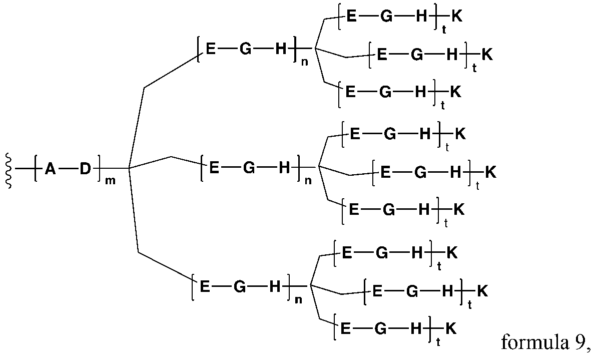 and 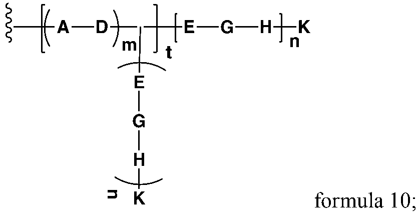
in formula 1 to formula 10, A and G are each independently absent, -(CH₂)ₕ-, or -(CH₂)ₕ in which one or more methylene groups are substituted with an M group; h is 0-15; the M group includes: one or more of -O-, -S-, -C(O)-, -C(O)O-, -C(O)NH-, -CH(T^{C})-, -C(T') (T")-, -NH-, -N(T^{N})-, -S-S-, -C(T')=C(T")-, -C≡C-, 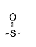 and 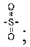
T^{C}, T^{N}, T' and T" in the M group each indicate that any one or more hydrogen atoms on a specified atom, including a carbon atom or a nitrogen atom, are replaced by L, provided that the normal valency of the specified atom is not exceeded and the substitution results in a stable compound; the L group includes any one of: C1-C6 alkyl, C2-C6 alkenyl, C2-C6 alkynyl, cyano, hydroxyl, oxo, carboxyl, cycloalkyl, cycloalkenyl, heterocyclyl, heteroaryl, aryl, ketone, alkoxycarbonyl, aryloxycarbonyl, heteroaryloxycarbonyl or halogen; the halogen includes F, Cl, Br or I; (O) in the A represents a carbonyl oxygen atom;
D, E, and H are each independently absent or one or more of -O-, -S-, -C(O)-, -NH-, -CH₂-, -C(O)NH-, -NHC(O)-, -C(O)O-, -OC(O)-, -OC(O)O-, -OC(O)NH-, -NHC(O)O-, 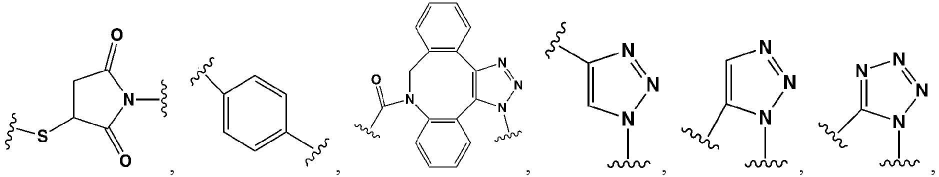 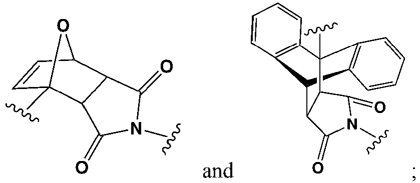 (O) in the D, E, and H groups represents a carbonyl oxygen atom; the 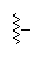 represents an attachment position of the corresponding formula; the A, G, D, E, and H are not simultaneously absent; and
K represents boronic acid or a derivative thereof, or phenylboronic acid and a derivative thereof; m, n and t are independently 1-15.

In some embodiments, the targeting ligand is directly covalently conjugated to an end of the nucleic acid molecule.

In some embodiments, when the targeting ligand is an aptamer, a phosphate group of the aptamer is covalently conjugated to a ribose of the nucleic acid molecule.

In some embodiments, when the targeting ligand is a compound modified with boronic acid or a derivative thereof, or a compound modified with phenylboronic acid or a derivative thereof, the targeting ligand is covalently conjugated to a phosphate group or base at an end of the nucleic acid molecule;
when covalently conjugated to the phosphate group at the end of the nucleic acid molecule, the targeting ligand is conjugated via an X group; the X group represents -O- or -S-.

In some embodiments, the targeting ligand is covalently conjugated to the nucleic acid molecule via an extension sequence linked to a terminus of the nucleic acid molecule.

In some embodiments, the extension sequence includes an extension sequence having only one end linked to a terminus of the nucleic acid molecule and/or an extension sequence that forms a stem-loop structure by being attached to the 3' end of one strand of the nucleic acid molecule at one end, while being linked or not linked to the 5' end of the complementary strand of the nucleic acid molecule at the other end.

In some embodiments, the extension sequence is 1-40nt in length; the extension sequence is a random nucleotide sequence.

In some embodiments, when the targeting ligand is an aptamer, a phosphate group of the aptamer is covalently conjugated to a ribose of the extension sequence.

In some embodiments, when the targeting ligand is a compound modified with boronic acid or a derivative thereof, or a compound modified with phenylboronic acid or a derivative thereof, the targeting ligand is covalently coupled to one or more phosphate groups or bases on the extension sequence;
when covalently conjugated to the phosphate groups of the extension sequence, the targeting ligand is conjugated via an X group; the X group represents -O- or -S-.

The present disclosure provides use of the pharmaceutical composition in the preparation of a drug for preventing and/or treating an ocular disease.

In some embodiments, the ocular disease includes one or more of dry eye disease, keratitis, conjunctivitis and blepharitis.

The present disclosure provides a pharmaceutical composition for inhibiting *NFKBIZ* gene expression, which includes a nucleic acid molecule and a targeting ligand. In the present disclosure, by means of conjugating the nucleic acid molecule and the targeting ligand, the problems existing in commonly used immunomodulatory drugs for ocular diseases, such as poor water solubility, poor compliance due to frequent and repeated administration, toxic and side effects, low bioavailability, and poor patient adherence, can be solved; Meanwhile, in the present disclosure, based on the structural characteristics of ocular tissues, target molecules that specifically recognize ocular surface tissues are selected, which enables targeted and efficient delivery of functional nucleic acids, thereby enabling functional nucleic acids to efficiently exert biological effects. In addition, the pharmaceutical composition for inhibiting *NFKBIZ* gene expression according to the present disclosure has simple ingredients and is easy to synthesize, thus possessing good prospects for translational application.

Further, the present disclosure specifically defines the sequences of the nucleic acid molecules. In the present disclosure, against the *NFKBIZ* gene (GenBank Accession No.: NM_031419.4), 210 pairs of double-stranded ribonucleic acid molecules are obtained by siRNA design methods. Moreover, quantitative polymerase chain reaction (qPCR) and Western blot are further used to verify the gene silencing efficiency of the siRNAs at the gene and protein levels, respectively. The results show that multiple screened siRNAs in the present disclosure are capable of efficiently inhibiting the expression of *NFKBIZ* mRNA, and have been proven to have the characteristic of inhibiting IκB-ζ expression at the protein level. The present disclosure provides a collection of nucleic acid molecules capable of specifically inhibiting the expression of the *NFKBIZ* gene and protein IκB-ζ. Compared to existing small-molecule immunomodulatory drugs, these nucleic acid molecules have advantages such as low tendency to induce drug resistance and long-acting efficacy, providing a foundation for the prevention and treatment of inflammation-related diseases by means of inhibiting the expression of inflammatory factors.

Further, the targeting ligands involved in the present disclosure are specifically defined as two categories: aptamers and boronic acid/phenylboronic acid and derivatives thereof. The conjunction of aptamers to nucleic acid molecules improves the cellular uptake efficiency of the nucleic acid molecules through the targeting of the aptamers to the ocular tissue-specific proteins, thereby improving the inhibitory effect on the *NFKBIZ* gene in ocular surface cells, enhancing the retention and adhesion capabilities of the nucleic acid pharmaceutical composition on the ocular surface, increasing bioavailability, reducing drug loss, and achieving effective treatment of ocular surface diseases. Moreover, in the present disclosure, by conjugating boronic acid/phenylboronic acid and derivatives thereof in different valences to nucleic acid molecules directly or via extension sequences, the intracellular delivery of the nucleic acid molecules can be effectively enhanced, and the inhibitory effect on the *NFKBIZ* gene can be improved, thereby achieving the treatment of ocular surface diseases.

### BRIEF DESCRIPTION OF THE DRAWINGS

FIG. 1 shows a schematic diagram of a pharmaceutical composition including a nucleic acid molecule and a targeting ligand according to the present disclosure.
FIG. 2 shows a schematic diagram of the conjugation of a nucleic acid aptamer to a nucleic acid molecule via an extension sequence, where 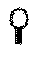 represents a nucleic acid molecule moiety, and the base may be adenine (A), guanine (G), cytosine (C), or uracil (U); 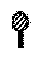 represents an extension sequence moiety, which may be a ribonucleotide molecule or a deoxyribonucleotide molecule, the base may be any one of adenine (A), guanine (G), cytosine (C), and thymine (T), and n represents 1-40 bases in length; and 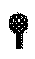 represents an aptamer moiety.
FIG. 3 shows a schematic diagram of the covalent conjugation of a targeting ligand to a nucleic acid molecule with a stem-loop structure.
FIG. 4 shows the qPCR results from screening siRNAs that inhibits the *NFKBIZ* gene expression.
FIG. 5 shows the Wester-blot analysis of the expression of IκB-ζ protein after treated with siRNAs targeting the NFKBIZ gene.
FIG. 6 is a polyacrylamide gel electrophoresis (PAGE) image of the Apt-si*NFKBIZ* nucleic acid pharmaceutical compositionsgene pharmaceutical compositions.
FIG. 7 shows the targeted cellular uptake evaluation of the nucleic acid pharmaceutical compositions including Apt-si*NFKBIZ*-Cy3 by HCEC cells, where panel A shows the detection results of laser scanning confocal microscopy, and panel B shows the detection results of flow cytometry.
FIG. 8 shows the qPCR results of the *NFKBIZ* gene silencing by the Apt-siNFKBIZ nucleic acid pharmaceutical compositions in HCEC cells.
FIG. 9 shows the Western-blot results for the inhibition of the IκBζ protein expression in HCEC cells by the Apt-siNFKBIZ nucleic acid pharmaceutical compositions.
FIG. 10 shows fluorescence images of tissue sections for the ocular surface retention of the Apt-si*NFKBIZ*-M nucleic acid pharmaceutical compositions in mice.
FIG. 11 shows the *in vivo* results of dry eye disease treatment using the Apt-si*NFKBIZ*-M nucleic acid pharmaceutical compositions, where panel A shows sodium fluorescein staining images; panel B is a graph showing sodium fluorescein staining scores; and panel C is a graph showing the results of tear secretion volume.
FIG. 12 shows the synthetic route of the PBA-si*NFKBIZ* nucleic acid pharmaceutical compositions.
FIG. 13 shows the verification results of the cellular uptake capacity of the PBA-si*NFKBIZ*-Cy3 nucleic acid pharmaceutical composition; where panel A shows a laser scanning confocal microscopy image; and panel B is a graph showing the flow cytometry quantification.
FIG. 14 shows the verification of the cellular gene regulatory capacity of the PBA-si*NFKBIZ* nucleic acid pharmaceutical composition by qPCR.
FIG. 15 shows the evaluation results for the effect of *in vivo* dry eye disease treatment using the PBA-si*NFKBIZ*-M nucleic acid pharmaceutical composition, where panel A shows the analysis of the sodium fluorescein scores on the ocular surface of mice on day 0, day 7, and day 14 of the treatment; and panel B shows the results of the phenol red thread test (PRTT) in mice on day 0, day 7, and day 14 of the treatment.
FIG. 16 shows the synthetic route of 2PBA-MAL.
FIG. 17 shows a ¹H NMR spectrum of compound 3.
FIG. 18 shows a ¹³C NMR spectrum of compound 3.
FIG. 19 shows a ¹H NMR spectrum of compound 4.
FIG. 20 shows a ¹³C NMR spectrum of compound 4.
FIG. 21 shows a ¹H NMR spectrum of compound 5.
FIG. 22 shows a ¹³C NMR spectrum of compound 5.
FIG. 23 shows the synthetic route of the 2PBA-si*NFKBIZ*-S conjugate.
FIG. 24 shows the verification of the synthesis of the 2PBA-si*NFKBIZ* nucleic acid pharmaceutical composition by 20% non-denaturing PAGE.
FIG. 25 shows the verification results of the cellular uptake capacity of the 2PBA-si*NFKBIZ*-Cy3 nucleic acid pharmaceutical composition, where panel A shows a laser scanning confocal microscopy image; and panel B is a graph showing the flow cytometer quantification.
FIG. 26 shows the cellular gene regulatory capacity of the 2PBA-si*NFKBIZ* nucleic acid pharmaceutical composition as verified by qPCR.
FIG. 27 shows the evaluation results for the effect of *in vivo* dry eye disease treatment using the 2PBA-si*NFKBIZ*-M nucleic acid pharmaceutical composition, where panel A shows sodium fluorescein staining images of the ocular surface of mice; panel B shows the analysis of the sodium fluorescein scores on the ocular surface of mice on day 0, day 7, and day 14 of the treatment; and panel C shows the results of the PRTT in mice on day 0, day 7, and day 14 of the treatment.
FIG. 28 shows the synthetic route of 4PBA-NH₂.
FIG. 29 shows a ¹H NMR spectrum of compound 7.
FIG. 30 shows a ¹³C NMR spectrum of compound 7.
FIG. 31 shows a ¹H NMR spectrum of compound 8.
FIG. 32 shows a ¹³C NMR spectrum of compound 8.
FIG. 33 shows a ¹H NMR spectrum of compound 11.
FIG. 34 shows a ¹³C NMR spectrum of compound 11.
FIG. 35 shows the synthetic route of the 4PBA-si*NFKBIZ*-S conjugate.
FIG. 36 shows the synthesis of the 4PBA-si*NFKBIZ* nucleic acid pharmaceutical composition as verified by 20% non-denaturing PAGE.
FIG. 37 shows the evaluation of the cellular uptake capacity of the 4PBA-si*NFKBIZ*-FAM nucleic acid pharmaceutical composition, where panel A shows a laser scanning confocal microscopy image; and panel B shows a flow cytometer quantitative graph.
FIG. 38 shows the results of the cellular biological evaluation of the 4PBA-si*NFKBIZ* nucleic acid pharmaceutical composition, where panel A shows the regulation of the *NFKBIZ* gene by the 4PBA-si*NFKBIZ* nucleic acid pharmaceutical composition as detected by qPCR; and panel B shows the regulation of IκB-ζ protein by the 4PBA-si*NFKBIZ* nucleic acid pharmaceutical composition as detected by Western-blot.
FIG. 39 shows the effect of *in vivo* dry eye disease treatment using the 4PBA-si*NFKBIZ*-M nucleic acid pharmaceutical composition, where panel A shows sodium fluorescein staining images of the ocular surface of mice; panel B shows the analysis of the sodium fluorescein scores on the ocular surface of mice on day 0, day 7, and day 14 of the treatment; and panel C shows the results of the PRTT in mice on day 0, day 7, and day 14 of the treatment;
FIG. 40 shows the synthetic route of 6PBA-N₃.
FIG. 41 shows a ¹H NMR spectrum of compound 13.
FIG. 42 shows a ¹H NMR spectrum of compound 15.
FIG. 43 shows a ¹³C NMR spectrum of compound 15.
FIG. 44 shows the synthetic route of the 6PBA-si*NFKBIZ*-S conjugate.
FIG. 45 shows the synthesis of the 6PBA-si*NFKBIZ* nucleic acid pharmaceutical composition as verified by 20% non-denaturing PAGE.
FIG. 46 shows the evaluation of the cellular uptake capacity of the 6PBA-si*NFKBIZ*-FAM nucleic acid pharmaceutical composition, where panel A shows a laser scanning confocal microscopy image; and panel B is a graph showing the flow cytometry quantification.
FIG. 47 shows the results of the cellular biological evaluation of the 6PBA-si*NFKBIZ* nucleic acid pharmaceutical composition, where panel A shows regulation of the *NFKBIZ* gene by the 6PBA-si*NFKBIZ* nucleic acid pharmaceutical composition as detected by qPCR; and panel B shows the regulation of the IκB-ζ protein by the 6PBA-si*NFKBIZ* nucleic acid pharmaceutical composition as detected by Western-blot.
FIG. 48 shows the evaluation results of the effect of *in vivo* disease treatment of the 6PBA-si*NFKBIZ*-M nucleic acid pharmaceutical composition, where panel A shows sodium fluorescein staining cornea images of the ocular surface of mice; panel B shows the analysis of the corneal sodium fluorescein score on the ocular surface of mice on day 0, day 7, and day 14 of the treatment; and panel C shows the results of the PRTT in mice on day 0, day 7, and day 14 of the treatment.
FIG. 49 shows the synthetic route of the PBA₁₀-siNKKBIZ-S conjugate.
FIG. 50 shows the verification of the synthesis of the PBA₁₀-si*NFKBIZ* nucleic acid pharmaceutical composition by 10% non-denaturing PAGE.
FIG. 51 shows the verification results of the cellular uptake capacity of the PBA₁₀-si*NFKBIZ*-FAM nucleic acid pharmaceutical composition at different time points by flow cytometry.
FIG. 52 shows the verification of the gene regulatory capacity of the PBA₁₀-si*NFKBIZ* nucleic acid pharmaceutical composition by qPCR.
FIG. 53 shows the evaluation results of the effect of *in vivo* disease treatment of the PBA₁₀-si*NFKBIZ*-M nucleic acid pharmaceutical composition, where panel A shows sodium fluorescein staining images of the ocular surface of mice; panel B shows the analysis of the sodium fluorescein scores on the ocular surface of mice on day 0, day 7, and day 14 of the treatment; and panel C shows the results of the PRTT in mice on day 0, day 7, and day 14 of the treatment.
FIG. 54 shows the synthetic route of 2PBA-Br.
FIG. 55 shows a ¹H NMR spectrum of compound 18.
FIG. 56 shows a ¹³C NMR spectrum of compound 18.
FIG. 57 shows the synthetic route of the PBA₆-siNKKBIZ-S conjugate.
FIG. 58 shows the synthesis of the PBA₆-si*NFKBIZ* nucleic acid pharmaceutical composition as verified by 20% non-denaturing PAGE.
FIG. 59 shows the evaluation results of the cellular uptake capacity of the PBA₆-si*NFKBIZ*-FAM nucleic acid pharmaceutical composition, where panel A shows a laser scanning confocal microscopy image of the uptake of the PBA₆-si*NFKBIZ*-FAM nucleic acid pharmaceutical composition by HCEC cells; panel B shows the uptake of the PBA₆-si*NFKBIZ*-FAM nucleic acid pharmaceutical composition by HCEC cells as quantified by flow cytometry.
FIG. 60 shows the results of the cellular biological evaluation of the PBA₆-si*NFKBIZ* nucleic acid pharmaceutical composition, where panel A shows the regulation of the *NFKBIZ* gene by the PBA₆-si*NFKBIZ* nucleic acid pharmaceutical composition as detected by qPCR; and panel B shows the regulation of the *NFKBIZ* gene regulation of the PBA₆-si*NFKBIZ* nucleic acid pharmaceutical composition as detected by Western-blot.
FIG. 61 shows the *in vivo* effect of dry eye disease treatment using the PBA₆-si*NFKBIZ*-M nucleic acid pharmaceutical composition, where panel A shows sodium fluorescein staining images of the ocular surface of mice; panel B shows the analysis results of the sodium fluorescein scores on the ocular surface of mice on day 0, day 7, and day 14 of the treatment; and panel C shows the results of the PRTT in mice on day 0, day 7, and day 14 of the treatment.

### DETAILED DESCRIPTION OF THE EMBODIMENTS

The present disclosure provides a pharmaceutical composition for inhibiting *NFKBIZ* gene expression, which includes a nucleic acid molecule inhibiting *NFKBIZ* gene expression and a targeting ligand for targeting an ocular tissue-specific protein.

In the present disclosure, the nucleic acid molecule is a double-stranded nucleic acid molecule designed based on the siRNA design principles against the *NFKBIZ* gene (GenBank Accession No.: NM_031419.4), which is complementary to at least 15 consecutive nucleotides in the sense or antisense strand of the *NFKBIZ* gene. In the present disclosure, the nucleic acid molecule includes at least one of the following nucleotide sequences: A: a sense strand selected from at least one of the following numbered nucleotide sequences: SEQ ID NO: 1 to SEQ ID NO: 18, SEQ ID NO: 25 to SEQ ID NO: 49, SEQ ID NO: 51 to SEQ ID NO: 89, SEQ ID NO: 93 to SEQ ID NO: 108, SEQ ID NO: 113 to SEQ ID NO: 132, SEQ ID NO: 134 to SEQ ID NO: 135, SEQ ID NO: 138, SEQ ID NO: 143 to SEQ ID NO: 152, SEQ ID NO: 154 to SEQ ID NO: 161, SEQ ID NO: 169 to SEQ ID NO: 185, and SEQ ID NO: 421; and an antisense strand selected from at least one of the following numbered nucleotide sequences: SEQ ID NO: 211 to SEQ ID NO: 228, SEQ ID NO: 235 to SEQ ID NO: 259, SEQ ID NO: 261 to SEQ ID NO: 299, SEQ ID NO: 303 to SEQ ID NO: 318, SEQ ID NO: 323 to SEQ ID NO: 342, SEQ ID NO: 348, SEQ ID NO: 344 to SEQ ID NO: 345, SEQ ID NO: 353 to SEQ ID NO: 362, SEQ ID NO: 364 to SEQ ID NO: 371, SEQ ID NO: 379 to SEQ ID NO: 395, and SEQ ID NO: 422; and B: nucleotide sequences having 2-3 consecutive or non-consecutive bases added, deleted or substituted based on the nucleotide sequences in item A, and target the *NFKBIZ* gene to exert an inhibitory effect. Experiments have demonstrated that the nucleic acid molecule of the present disclosure is a double-stranded nucleic acid molecule capable of efficiently targeting the *NFKBIZ* gene sequence. Compared to existing small-molecule immunological drugs, the nucleic acid molecule has advantages such as low tendency to induce drug resistance and long-acting efficacy. The length of the nucleic acid molecule is preferably 15-30bp. In the present disclosure, a total of 210 pairs of nucleic acid molecules are designed. The inhibition of the nucleic acid molecules on the *NFKBIZ* gene is verified by qPCR, and the inhibition of the nucleic acid molecules on the expression of the IκB-ζ protein encoded by the *NFKBIZ* gene is verified by Western-blot. The results show that the nucleic acid molecules of the solutions of the present disclosure have the characteristic of high target gene silencing efficiency compared to other nucleic acid molecules designed in the same batch. The nucleic acid molecule may be synthesized by standard methods known in the art, for example, by using an automated nucleic acid synthesizer. In specific embodiments, the species source of the *NFKBIZ* gene preferably includes human and/or murine origin.

In the present disclosure, an end of the sense or antisense strand of the nucleic acid molecule is preferably further provided with an overhang. The length of the overhang is preferably 1-3nt, more preferably 2nt. The present disclosure imposes no special limitation on the nucleotide sequence of the overhang, and any sequence well-known in the art for an overhang may be employed. In the embodiments of the present disclosure, the sequence of the overhang is preferably a sequence formed by oligomerization of U or T or a sequence that is complementary paired with a sequence downstream of the nucleic acid molecule targeting region on the *NFKBIZ* gene template. The position of the overhang is preferably at one end of the nucleic acid molecule. When an extension sequence is attached to the nucleic acid molecule, the overhang and the extension sequence are preferably attached to different ends of the nucleic acid molecule, respectively.

In the present disclosure, the nucleic acid molecule includes any one or more of the following modified nucleotides: deoxynucleotides, 3'-deoxythymidine (dT) nucleotides, 2'-O-methyl modified nucleotides, 2'-fluoro-modified nucleotides, 2'-deoxy-modified nucleotides, locked nucleic acids, conformationally restricted nucleotides, constrained ethyl nucleotides, abasic nucleotides, 2'-amino-modified nucleotides, 2'-O-allyl-modified nucleotides, 2'-C-alkyl-modified nucleotides, 2'-hydroxy-modified nucleotides, 2'-methoxyethyl-modified nucleotides, 2'-O-alkyl-modified nucleotides, morpholino nucleotides, phosphoramidate-modified nucleotides, nucleotides containing unnatural bases, tetrahydropyran-modified nucleotides, 1,5-anhydrohexitol-modified nucleotides, cyclohexenyl-modified nucleotides, nucleotides containing phosphorothioate linkages, nucleotides containing methylphosphonate groups, nucleotides containing 5'-phosphate groups, and nucleotides containing 5'-phosphate mimics. The modifications are conducive to improving the stability of the nucleic acid molecule.

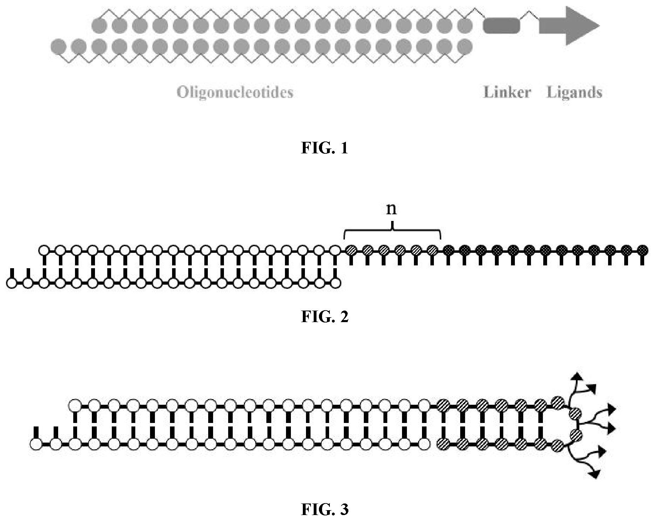

In the present disclosure, the schematic structural diagram of the pharmaceutical composition is shown in FIG. 1. The targeting ligand targeting the ocular tissue-specific protein preferably includes an aptamer targeting an ocular tissue-specific protein, a compound modified with boric acid or a derivative thereof, or a compound modified with phenylboronic acid or a derivative thereof. The ocular tissue-specific protein preferably includes at least one of: mucin, integrin, and CD44. The mucin preferably includes at least one of: MUC-1, MUC-4, and MUC-16. Ocular surface mucins, a group of high-molecular-weight glycoproteins widely present on the ocular surface, are classified into two major categories: secretory mucins and membrane-associated mucins, and play an important role in lubricating the ocular surface and maintaining ocular surface homeostasis. Ocular surface mucins are closely associated with a variety of ocular surface diseases, including dry eye disease, infections, allergies, and immune-related diseases. Mucins can bind to water to form a gel, preventing tear evaporation. They can also interact with a variety of inflammatory factors and play an important role in maintaining the ocular surface epithelial cell barrier. Mucins are classified into two major categories: secretory mucins and membrane-associated mucins. MUC1, MUC4 and MUC16 are the major membrane-associated mucins in the human eye and can be detected in the cornea, conjunctiva and lacrimal glands. The expression levels of *MUC*1 and *MUC*16 mRNA in the conjunctiva and cornea are the same, whereas the expression level of *MUC4* mRNA is the highest in the conjunctival epithelium and relatively low in the corneal epithelium. Moreover, corneal epithelial cells have been shown to express integrins and are a source of inflammatory cytokines in dry eye disease. In addition, inflammatory Th17 cells express a large amount α_{V}β₃ integrin, which is essential to maintain the Th17 inflammatory phenotype, such as the production of IL-17 in dry eye disease and IL-17-induced disruption of the corneal epithelial barrier. In addition, CD44 protein is a transmembrane protein expressed on the surface of corneal epithelial cells, and CD44 protein is involved in the migration and adhesion processes of damage repair. Therefore, in the present disclosure, an ocular surface tissue cell-targeting aptamer and a nucleic acid pharmaceutical composition *(Apt-siNFKBIZ)* for inhibiting the *NFKBIZ* gene are designed based on targeting mucin, integrin, and CD44 protein to improve the efficiency of nucleic acid molecule silencing. The aptamer targeting the mucin MUC-1 (Apt_{MUC1}) preferably has the nucleotide sequence of SEQ ID NO: 423. The aptamer targeting the mucin MUC-16 (Apt_{MUC16}) preferably has the nucleotide sequence of SEQ ID NO: 424. The nucleotide sequence of the aptamer targeting the integrin α_{V}β₃ has the nucleotide sequence of SEQ ID NO: 425. The nucleotide sequence of the aptamer targeting CD44 protein has the nucleotide sequence of SEQ ID NO: 426.

In the present disclosure, the aptamer preferably further includes a stability-enhancing modification. The stability-enhancing modification preferably includes at least one of: phosphorothioate backbone modification, 2'-O-methyl modification, 2'-methoxyethyl modification, 2'-fluoro modification, inverted dT modification, dT modification, and terminal conjugation with polyethylene glycol.

In the present disclosure, the compound modified with boric acid or the derivative thereof, or the compound modified with phenylboronic acid or the derivative thereof, is a linear or branched compound terminally modified with boric acid or phenylboronic acid. The number of boronic acid or the derivative thereof, or phenylboronic acid or the derivative thereof, modified on the linear or branched compound is 1-40, more preferably 2-20, and further more preferably 3-10. The compound modified with boronic acid or the derivative thereof, or the compound modified with phenylboronic acid or the derivative thereof, is as represented by any one of formula 1 to formula 10:

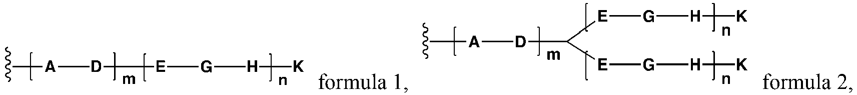

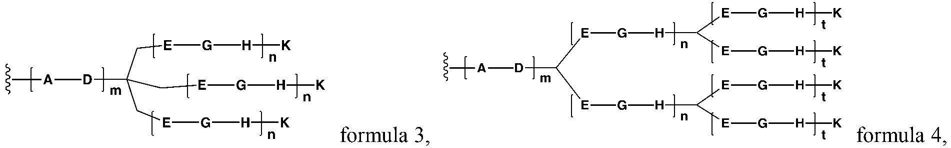

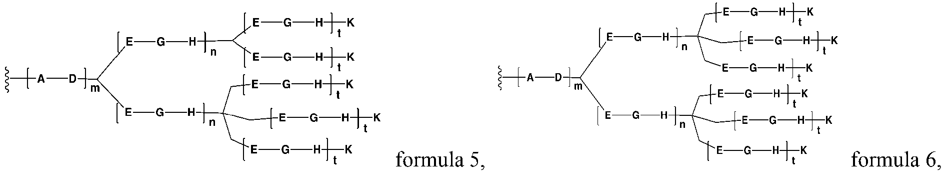

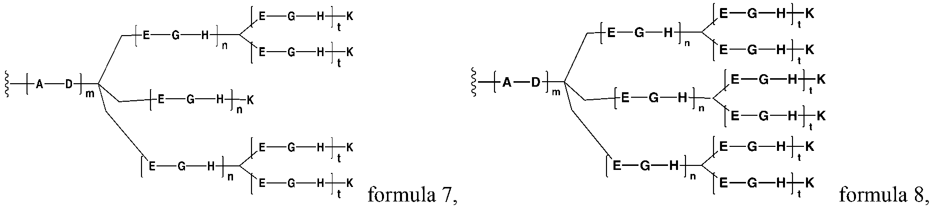

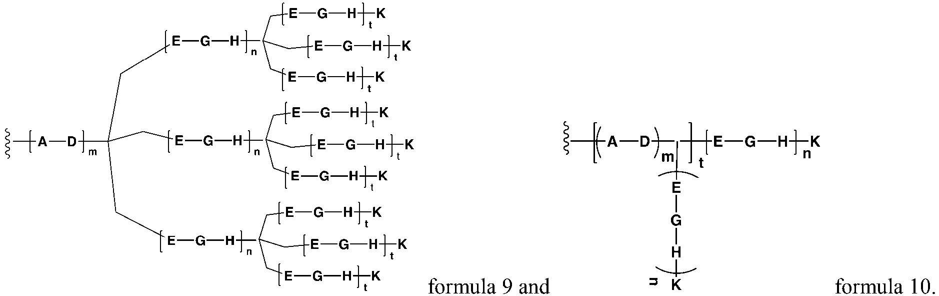

In formula 1 to formula 10, A and G are each independently absent, -(CH₂)ₕ-, or -(CH₂)ₕ- in which one or more methylene groups are substituted with an M group; h is 0-15; the M group includes: one or more of -O-, -S-, -C(O)-, -C(O)O-, -C(O)NH-, -CH(T^{C})-, -C(T') (T")-, -NH-, -N(T^{N})-, -S-S-, -C(T')=C(T")-, -C≡C-, 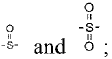
T^{C}, T^{N}, T' and T" in the M group each indicate that any one or more hydrogen atoms on a specified atom, including a carbon atom or a nitrogen atom, are replaced by L, provided that the normal valency of the specified atom is not exceeded and the substitution results in a stable compound; the L group includes any one of: C1-C6 alkyl, C2-C6 alkenyl, C2-C6 alkynyl, cyano, hydroxyl, oxo, carboxyl, cycloalkyl, cycloalkenyl, heterocyclyl, heteroaryl, aryl, ketone, alkoxycarbonyl, aryloxycarbonyl, heteroaryloxycarbonyl or halogen; the halogen includes F, Cl, Br or I; (O) in the A represents a carbonyl oxygen atom;
D, E, and H are each independently absent or one or more of, -O-, -S-, -C(O)-, -NH-, -CH₂-, -C(O)NH-, -NHC(O)-, -C(O)O-, -OC(O)-, -OC(O)O-, -OC(O)NH-, -NHC(O)O-, 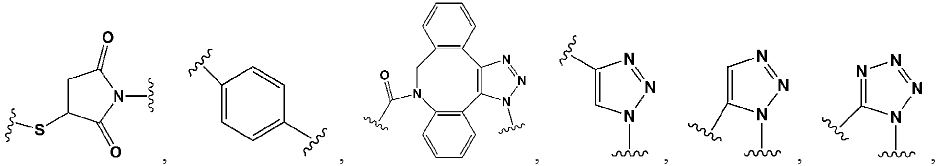 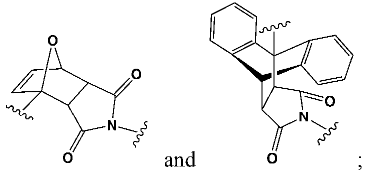 (O) in the D, E, and H groups represents a carbonyl oxygen atom; the 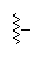 represents an attachment position of the corresponding formula; the A, G, D and H are not simultaneously absent; K represents boronic acid or a derivative thereof, or phenylboronic acid and a derivative thereof; m, n and t are independently 1-15.

In the present disclosure, phenylboronic acid and a derivative thereof, or boronic acid and a derivative thereof, are capable of forming reversible covalent binding to a 1,2-dihydroxy compound or a 1,3-dihydroxy compound, thereby enabling effective recognition of substances such as sialic acid and carbohydrate. A large number of compounds such as carbohydrates and glycoproteins are present on the surface of the cell membrane, and phenylboronic acid can form cyclic boronic esters with polyhydroxy compounds such as sugars, thereby enabling a wide range of applications in carbohydrate and cell recognition. The ocular surface epithelial cells contain a glycocalyx barrier composed of a large number of highly glycosylated glycoproteins, with the main components thereof being transmembrane mucins MUC1, MUC4, MUC16 and galectin-3. The barrier plays roles in lubrication, moistening and barrier on the ocular surface. Thus, in the present disclosure, based on the dynamic covalent bond or non-covalent interaction between phenylboronic acid and a protein glycosyl group, a pharmaceutical composition is designed by terminal grafting onto the sense strand of *siNFKBIZ.* The pharmaceutical composition has good ocular surface retention properties. By means of the specific interaction between phenylboronic acid and an MUC protein expressed on the surface of ocular surface cells, the pharmaceutical composition can effectively promote cellular uptake, thereby more efficiently exerting biological effects.

In the present disclosure, the targeting ligand is preferably directly covalently conjugated to an end of the nucleic acid molecule.

When the targeting ligand is an aptamer, a phosphate group of the aptamer is preferably covalently conjugated to a ribose of the nucleic acid molecule. When the targeting ligand is a compound modified with boronic acid or a derivative thereof, or a compound modified with phenylboronic acid or a derivative thereof, the targeting ligand is preferably covalently conjugated to a phosphate group or base at a terminus of the backbone of the nucleic acid molecule. When the targeting ligand is preferably covalently conjugated to a phosphate group at the terminus of the nucleic acid molecule, the pharmaceutical composition preferably includes at least one of formula 11 to formula 20: 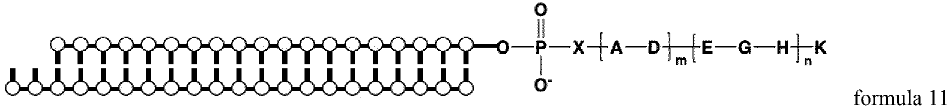 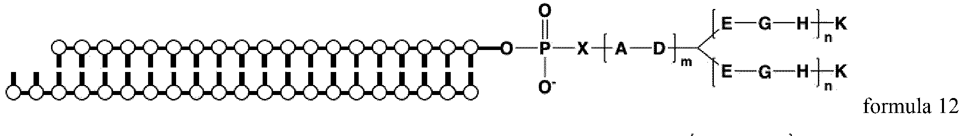 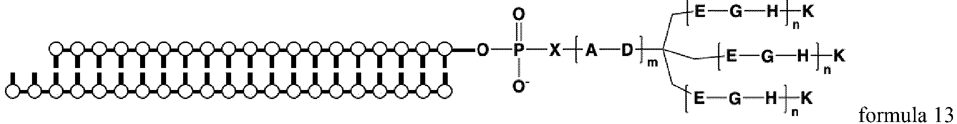 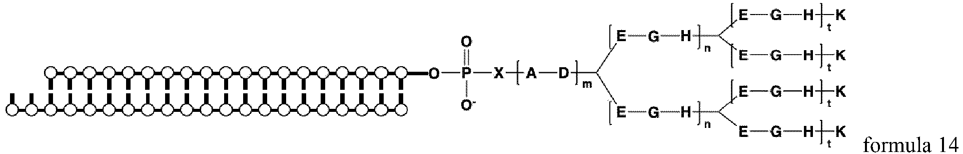 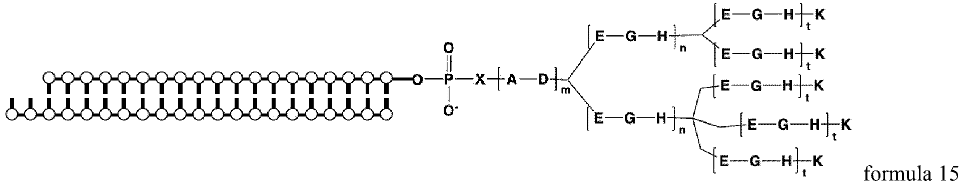 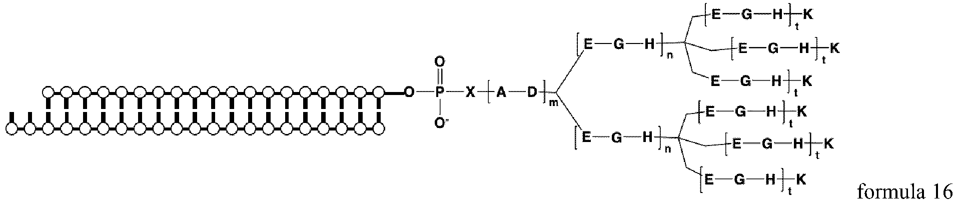 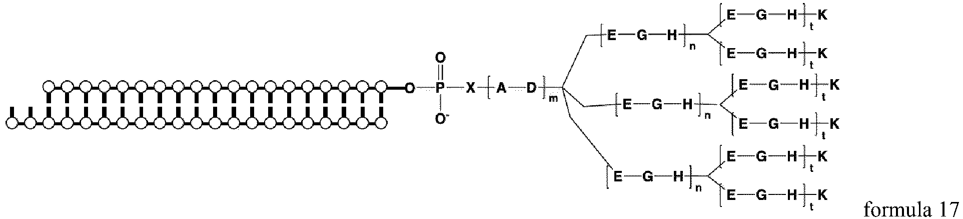 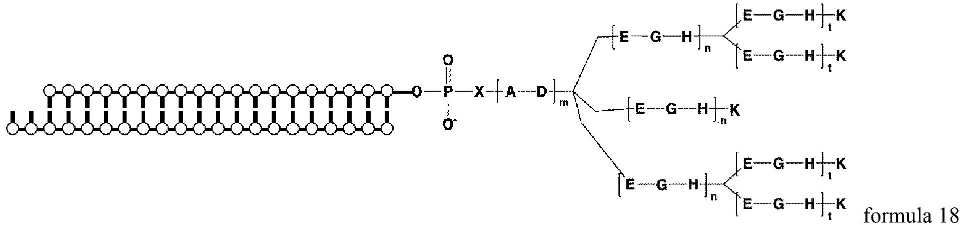 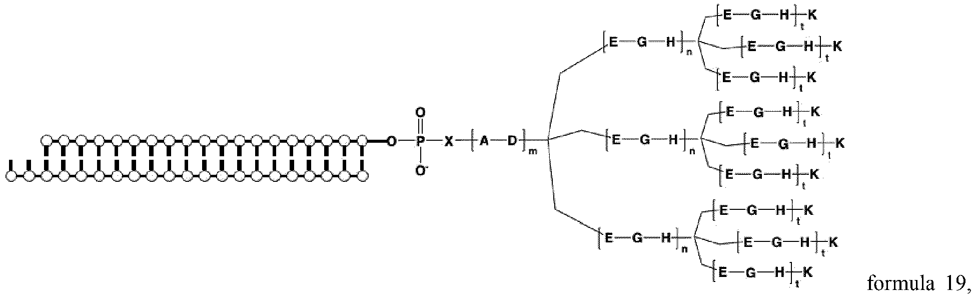 and 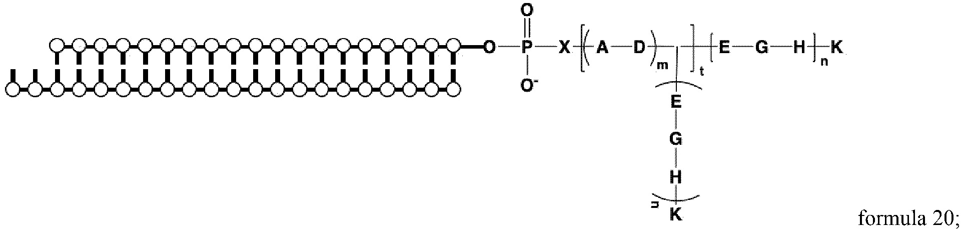 in formula 11 to formula 20, the X group represents -O- or -S-.

In the present disclosure, when the targeting ligand is preferably covalently conjugated to a base at a terminus of a nucleic acid molecule, the pharmaceutical composition preferably includes at least one of formula 21 to formula 30: 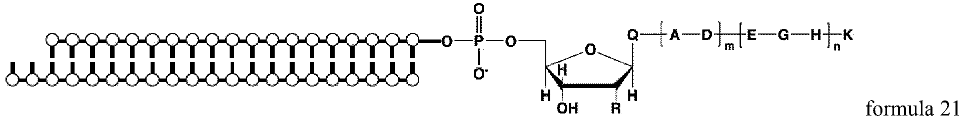 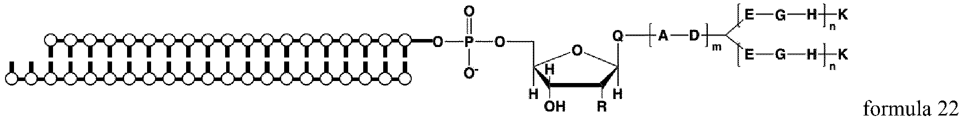 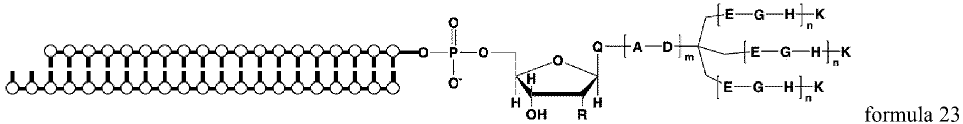 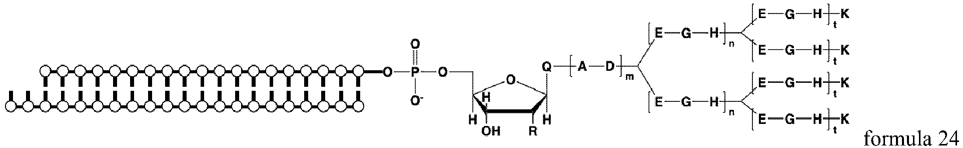 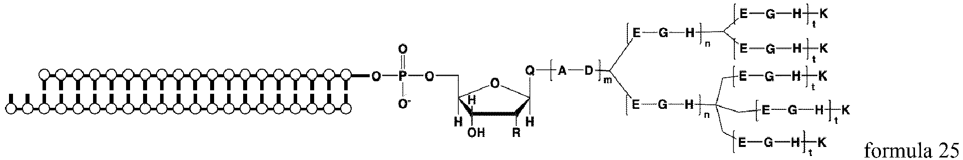 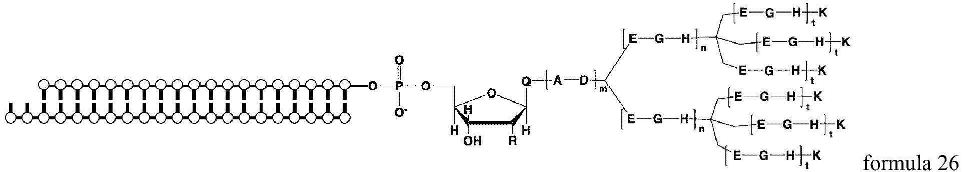 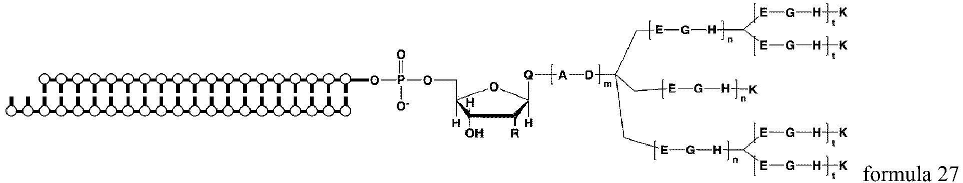 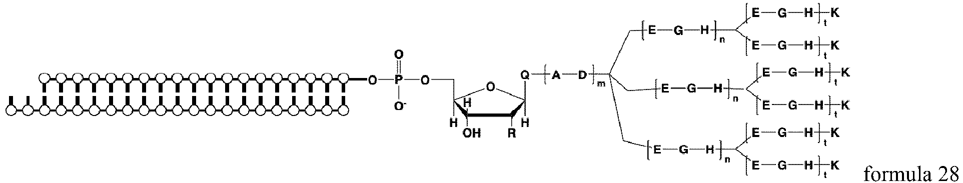 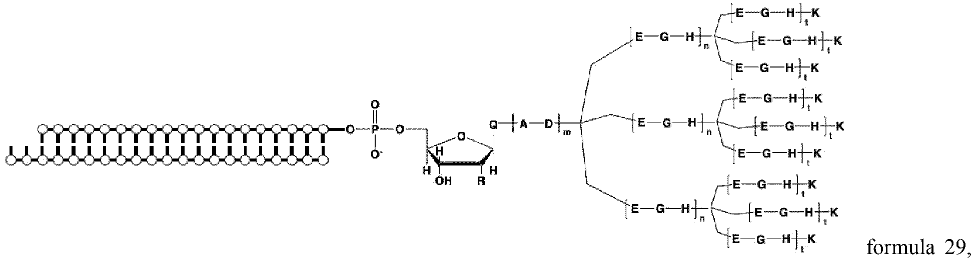 and 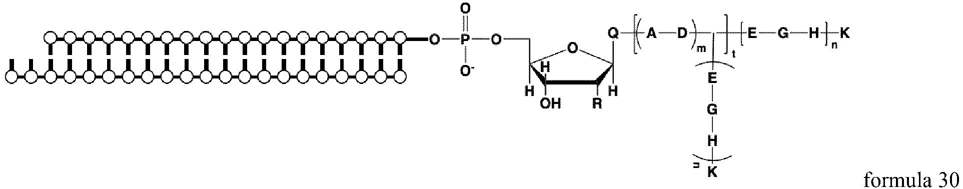
In formula 21 to formula 30, Q represents a base, and R represents -H or -OH;
In formula 11 to formula 30, 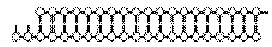 represents a nucleic acid molecule.

In the present disclosure, the targeting ligand is preferably covalently conjugated to the nucleic acid molecule via an extension sequence linked to a terminus of the nucleic acid molecule. The extension sequence preferably includes an extension sequence having only one end attached to a terminus of the nucleic acid molecule and/or an extension sequence having one end attached to the 3' end of one strand of the nucleic acid molecule and the other end attached or not attached to the 5' end of the complementary strand of the nucleic acid molecule, thereby forming a stem-loop structure. The length of the extension sequence is preferably 1-40nt; the extension sequence is preferably a random nucleotide sequence, such as an oligonucleotide sequence, TTTTTT.

In the present disclosure, when the targeting ligand is preferably an aptamer, a phosphate group of the aptamer is preferably covalently conjugated to a ribose of the extension sequence. FIG. 2 shows a schematic diagram of a pharmaceutical composition formed by the covalent conjugation of an aptamer to a nucleic acid molecule via an extension sequence, where the length of the extension sequence is preferably 1-40nt, and the extension sequence can be used to conjugate the aptamer to boric acid or phenylboronic acid or a derivative thereof. The targeting ligand may also form a pharmaceutical composition by the conjugation to the nucleic acid molecule via an extension sequence having a stem-loop structure, where the length of the extension sequence is preferably 10-40nt, the 5' end of the extension sequence is attached to one strand of the nucleic acid molecule, the 3' end of the extension sequence forms a stem-loop structure with or without attachment to the terminus of the complementary strand of the nucleic acid molecule, and the extension sequence is mainly used for conjugating boric acid or phenylboronic acid or a derivative thereof. FIG. 3 shows the formation of a 16-base stem-loop structure at the 3' end of the sense strand of the nucleic acid molecule and the modification with 3 phosphorothioate (PS) backbone modifications at the loop site of the stem-loop structure (3PS-si*NFKBIZ*-S), which enables the conjugation of the stem-loop structure to a bromo group modified on bromo-diphenylboronic acid (2PBA-Br).

In the present disclosure, in a method for preparing the pharmaceutical composition obtained by conjugating an aptamer to a nucleic acid molecule, the aptamer is preferably attached to one strand of the nucleic acid molecule directly or via an extension sequence, then assembled with the complementary strand of the nucleic acid molecule by complementary base pairing, and verified by using non-denaturing PAGE, thereby obtaining a nucleic acid pharmaceutical composition. The present disclosure imposes no special limitation on the attachment method, and any attachment method known in the art may be employed, such as solid-phase nucleic acid synthesis.

In one embodiment of the present disclosure, aptamers with targeting properties for MUC1, MUC16, integrin α_{V}β₃ and CD44 protein are designed, respectively, and pharmaceutical compositions (Apt_{MUC1}-si*NFKBIZ*, Apt_{MUC16}-si*NFKBIZ*, Apt_{αvβ3}-si*NFKBIZ* and Apt_{CD44}-si*NFKBIZ*) formed by attaching the aptamers to nucleic acid molecules inhibiting the *NFKBIZ* gene via extension sequences are prepared, where the pharmaceutical compositions are used for efficient targeted therapy of dry eye disease. Mucins, a group of high-molecular-weight glycoproteins widely present on the ocular surface, are classified into two major categories: secretory mucins and membrane-associated mucins, and play an important role in lubricating the eye and maintaining ocular homeostasis. Ocular mucins are closely associated with a variety of ocular diseases, including dry eye disease, infections, allergies, and immune-related diseases. Mucins can bind to water to form a gel, preventing tear evaporation. They can also interact with a variety of inflammatory factors and play an important role in maintaining the ocular epithelial cell barrier. Corneal epithelial cells have been shown to express integrins and are a source of inflammatory cytokines in dry eye disease. In addition, inflammatory Th17 cells express a large amount of α_{V}β₃ integrin, which is essential to maintain the Th17 inflammatory phenotype, such as the production of IL-17 in dry eye disease and IL-17-induced disruption of the corneal epithelial barrier. CD44 is a transmembrane protein expressed on the surface of corneal epithelial cells, and is involved in the migration and adhesion processes of damage repair. Thus, in the present disclosure, the si*NFKBIZ-*based targeted nucleic acid pharmaceutical compositions (Apt_{MUC1}-si*NFKBIZ*, Apt_{MUC16}-si*NFKBIZ*, Apt_{αvβ3}-si*NFKBIZ* and Apt_{CD44}-si*NFKBIZ*) are constructed based on aptamers targeting MUC1, MUC16, integrin α_{V}β₃ and CD44. The Apt-si*NFKBIZ* compositions overcome various barriers on the ocular surface through hydrophilic aptamers. After ocular surface delivery, the compositions pass through the cornea, the largest static barrier on the ocular surface, allowing for receptor-mediated dsRNA intracellular delivery. The dsRNA passes through the aqueous layer of the tear film to reach the eye, where it is efficiently taken up by corneal epithelial cells. This results in effective adsorption and endocytosis in the ocular surface epithelial tissue, achieving efficient delivery of the functional nucleic acid molecules *siNFKBIZ.* After entering the cells, the nucleic acid molecules (active pharmaceutical ingredients) can better exert immunomodulatory and anti-inflammatory effects. Specifically, the nucleic acid molecules inhibit the production and release of pro-inflammatory cytokines, reduce the release of inflammatory factors (interleukin-1, interleukin-17 and tumor necrosis factor α) and matrix metalloproteinases, and decrease T cell activity to control immune-inflammatory responses and regulate immune homeostasis, thereby effectively alleviating the symptoms and signs of dry eye disease induced by long-term inflammation. Further, the nucleic acid molecules inhibit the inflammatory response of dry eye, promote the restoration of self-tear secretion in dry eye disease patients, break the inflammation vicious cycle of dry eye disease, and inhibit the apoptosis of lacrimal gland cells and goblet cells, thereby achieving the effective treatment of dry eye disease.

In the present disclosure, when the targeting ligand is preferably a compound modified with boronic acid or a derivative thereof, or a compound modified with phenylboronic acid or a derivative thereof, the targeting ligand is covalently conjugated to one or more phosphate groups or bases on the extension sequence. When conjugated to a phosphate group, the compound modified with boronic acid or the derivative thereof, or the compound modified with phenylboronic acid or the derivative thereof is conjugated to the phosphate group via an X group. The X group represents -O- or -S-. When the compound modified with boronic acid or the derivative thereof, or the compound modified with phenylboronic acid or the derivative thereof is monovalent boronic acid or monovalent phenylboronic acid (formula 1), the targeting ligand, when conjugated to a phosphate or base on an extension sequence, includes formula 31 to formula 36: 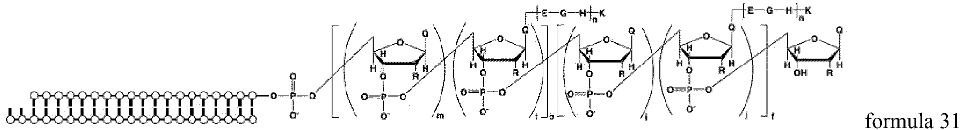 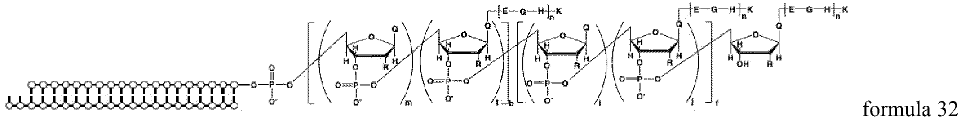 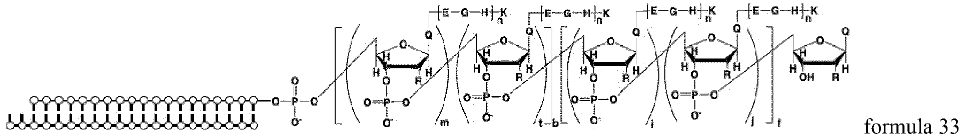 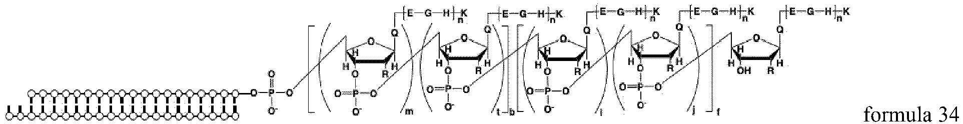 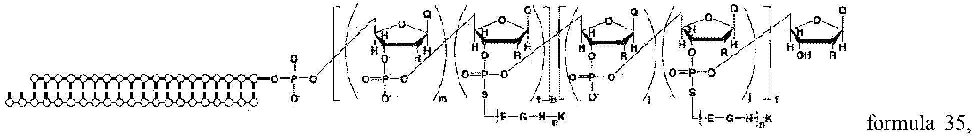 and

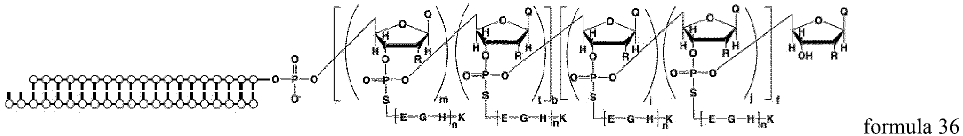

In formula 31 to formula 36, 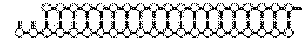 represents a nucleic acid molecule.

In the present disclosure, when the targeting ligand is a compound modified with boronic acid or a derivative thereof, or a compound modified with phenylboronic acid or a derivative thereof, the method for preparing the pharmaceutical composition preferably includes: conjugating a compound modified with boronic acid or a derivative thereof, or a compound modified with phenylboronic acid or a derivative thereof to a phosphate group or base of one strand of a nucleic acid molecule or extension sequence through a chemical reaction to obtain a conjugate; subjecting the conjugate to annealing hybridization with the complementary strand of the nucleic acid molecule to obtain a nucleic acid pharmaceutical composition.

In the present disclosure, the phosphate group or base of one strand of the nucleic acid molecule or extension sequence is preferably subjected to group modification, and then covalently conjugated to a compound modified with boronic acid or phenylboronic acid or a derivative thereof through a chemical reaction to obtain a conjugate. The groups used for the modification include an amino group (3'-NH₂, formula 59), a sulfhydryl group (3'-SH, formula 60), an aldehyde group (5'-CHO, formula 61), dibenzocyclooctyne (3'-DBCO, formula 62), etc.

In the present disclosure, conditions for the annealing hybridization are preferably as follows: the molar ratio of the conjugate to the complementary strand of the nucleic acid molecule is preferably 1 : 1. The temperature for the hybridization annealing is preferably set as follows: the reaction system is placed in a PCR instrument, heated to 65°C and maintained at this temperature for 10 min, and then annealed at a cooling rate of 4°C per minute until the temperature drops to 25°C.

In one embodiment of the present disclosure, a pharmaceutical composition PBA-si*NFKBIZ* is constructed, which is formed by covalently conjugating a single phenylboronic acid (PBA) molecule to a nucleic acid molecule (si*NFKBIZ*) targeting the nuclear factor κB inhibitor ζ (*NFKBIZ*) gene. The specific preparation method preferably includes the steps of:
conjugating 4-carboxyphenylboronic acid to a single strand of a nucleic acid molecule modified with an amino group at the 3' end through an amide chemical reaction to obtain a conjugate;
subjecting the conjugate to hybridization annealing with the complementary strand of the nucleic acid molecule to obtain the pharmaceutical composition PBA-si*NFKBIZ*.

In the present disclosure, the single strand of the nucleic acid molecule modified with an amino group at the 3' end can be directly commissioned to a gene synthesis company for preparation. The condensing agent for the amide chemical reaction is preferably dicyclohexylcarbodiimide (DCC), diisopropylcarbodiimide (DIC), 1-ethyl-(3-dimethylaminopropyl)carbodiimide hydrochloride (EDCI), preferably EDCI. The activating reagent for the amidation reaction is preferably *N*-hydroxysuccinimide (NHS). The temperature of the amide chemical reaction is preferably 20-55°C, more preferably 25°C. The time for the amide chemical reaction is preferably 12-48 h, more preferably 24 h. The amide chemical reaction is preferably performed with shaking, preferably at a speed of 200-4000 rpm, more preferably at 1000 rpm. After the amide chemical reaction, excess 4-carboxyphenylboronic acid and water are preferably removed. The method for removing the 4-carboxyphenylboronic acid preferably includes extraction with ethyl acetate. The method for removing water preferably includes concentration by distillation. The formula of the conjugate is as represented by formula 55: 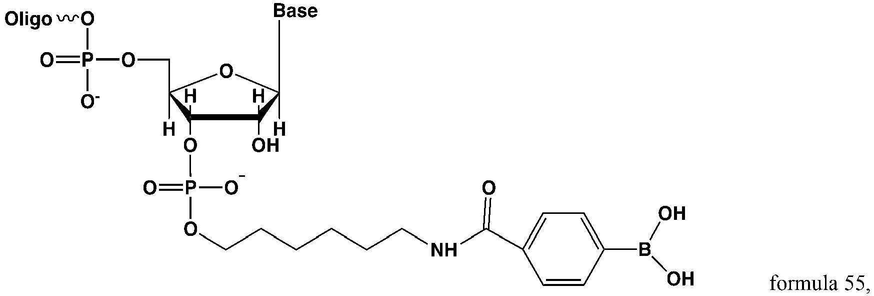 where Oligo 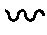 represents an attached nucleic acid molecule, and Base represents any type of base.

In the present disclosure, the conjugate to the complementary strand of the nucleic acid molecule is preferably at a molar ratio of 1 : 1. Conditions for the hybridization annealing are preferably as follows: the reaction system is placed in a PCR instrument, heated to 65°C and maintained at this temperature for 10 min, and then annealed at a cooling rate of 4°C per minute until the temperature drops to 25°C.

In the present disclosure, the PBA-si*NFKBIZ* can be used for the treatment of ocular diseases. In the present disclosure, by means of constructing a PBA-si*NFKBIZ* covalent conjugate, targeted delivery of a nucleic acid drug for inhibiting the *NFKBIZ* gene can be achieved, thereby achieving targeted treatment of dry eye disease. In the present disclosure, based on a dynamic covalent bond interaction between phenylboronic acid and a protein glycosyl group, an *siNFKBIZ* pharmaceutical composition (PBA-siNFKBIZ) is designed and prepared by terminal grafting onto the sense strand of *siNFKBIZ.* The pharmaceutical composition has good ocular surface retention properties. By means of the specific interaction between phenylboronic acid and a sugar chain on an MUC glycoprotein overexpressed on the surface of ocular cells, the pharmaceutical composition can effectively promote cellular uptake, thereby exerting biological effects more efficiently. In addition, small interfering RNA targeting the *NFKBIZ* gene *(siNFKBIZ)* is capable of effectively knocking down the expression of the *NFKBIZ* gene in corneal epithelial cells, thereby reducing the expression of IκB-ζ protein, reducing the level of cellular inflammation, and regulating immune homeostasis. Due to the presence of PBA, the PBA-si*NFKBIZ* covalent conjugate can enhance the interaction thereof with ocular surface tissue cells. Compared to unmodified oligonucleotides, the PBA-si*NFKBIZ* conjugate can be better endocytosed by cells and thus better exert a gene regulatory effect, thereby achieving a good therapeutic effect on dry eye disease.

In one embodiment of the present disclosure, a pharmaceutical composition (2PBA-siNFKBIZ) for inhibiting *NFKBIZ* gene expression is constructed by covalently conjugating two PBA molecules to *siNFKBIZ* through terminal grafting onto the sense strand of *siNFKBIZ.* The specific preparation method preferably includes the steps of:
dissolving a compound of formula 37 and a compound of formula 38 (4-carboxyphenylboronic acid pinacol ester), adding EDCI and DMAP (4-dimethylaminopyridine) for reaction under nitrogen atmosphere to obtain a compound of formula 39;
reacting the compound of formula 39, sodium periodate and ammonium acetate under nitrogen atmosphere to obtain a compound of formula 40;
co-dissolving the compound of formula 40 and 4-maleimidobutyric acid in methanol, adding 4-(4,6-dimethoxy-1,3,5-triazin-2-yl)-4-methylmorpholinium chloride (DMTMM) and N-methylmorpholine (NMM) for reaction to obtain a compound of formula 41;
reacting the compound of formula 41 and one strand of a nucleic acid molecule modified with a sulfhydryl group at the 3' end with shaking to obtain a 2PBA-si*NFKBIZ-*S conjugate; and
subjecting the 2PBA-si*NFKBIZ*-S conjugate and the complementary strand of the nucleic acid molecule to annealing hybridization to obtain the pharmaceutical composition 2PBA-si*NFKBIZ*, where formula 37 is 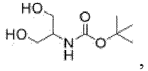 formula 38 is 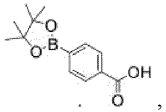 formula 39 is 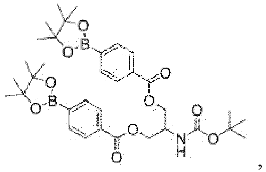 formula 40 is 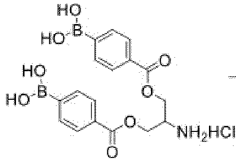 and formula 41 is

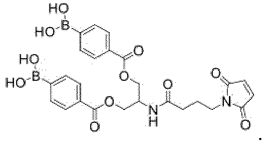

In the present disclosure, the concentration of the compound of formula 37 is preferably 75.00 mg/mL. The concentration of the 4-carboxyphenylboronic acid pinacol ester is preferably 29.00 mg/mL. The concentration of the EDCI is preferably 22.50 mg/mL. The concentration of the DMAP is preferably 0.75 mg/mL. The temperature of the reaction is preferably 20-50°C, more preferably 25°C. The time for the reaction is preferably 4-48 h, more preferably 24 h. After the reaction, distillation under reduced pressure to remove the solvent, followed by separation and purification, is preferably performed. The method for separation and purification is preferably crystallization.

In the present disclosure, the working concentration of the compound of formula 39 is preferably 21.40 mg/mL. The working concentration of the sodium periodate is preferably 28.25 mg/mL. The working concentration of the ammonium acetate is preferably 10.30 mg/mL. The temperature of the reaction is preferably 15-50°C, more preferably 25°C. The time for the reaction is preferably 2-24 h, more preferably 10 h. After the reaction, the organic solvent is removed by distillation under reduced pressure, and after the washing, water is removed to obtain a compound of formula 40.

In the present disclosure, the working concentration of the compound of formula 40 is preferably 14.10 mg/mL. The working concentration of the 4-maleimidobutyric acid is preferably 12.20 mg/mL. The working concentration of the DMTMM is preferably 19.20 mg/mL. The working concentration of the NMM is preferably 68 mg/mL. The temperature of the reaction is preferably 15-50°C, more preferably 25°C. The time for the reaction is preferably 1-10 h, more preferably 2 h. After the reaction, the organic solvent is removed by distillation under reduced pressure, and after the washing, water is removed to obtain a compound of formula 41.

In the present disclosure, the single strand of the nucleic acid molecule modified with a sulfhydryl group at the 3' end is commissioned to a gene synthesis company for preparation. The temperature of the click chemical reaction is preferably 20-55°C, more preferably 40°C. The time for the click chemical reaction is preferably 12-48 h, more preferably 24 h. The click chemical reaction is preferably performed with shaking, preferably at a speed of 200-4000 rpm, more preferably at 1800 rpm. After the click chemical reaction, excess compound of formula 41 and water are preferably removed. The method for removing the compound of formula 41 preferably includes extraction with ethyl acetate. Water is removed by concentrative distillation. The formula of the conjugate is as represented by formula 56. 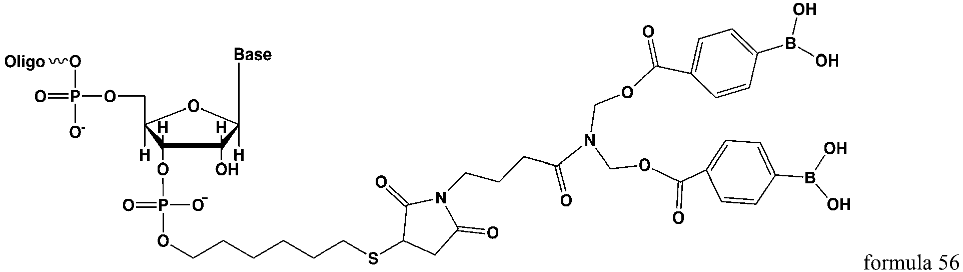 where Oligo 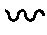 represents an attached nucleic acid molecule, and Base represents any type of base.

In the present disclosure, the conjugate to the complementary strand of the nucleic acid molecule is preferably at a molar ratio of 1 : 1. Concentrations for the hybridization and annealing are preferably set as follows: the reaction system is placed in a PCR instrument, heated to 65°C and maintained at this temperature for 10 min, and then annealed at a cooling rate of 4°C per minute until the temperature drops to 25°C.

In the present disclosure, the 2PBA-si*NFKBIZ* conjugate achieves efficient delivery of *siNFKBIZ* through the targeted recognition of PBA and the enhanced binding ability to membrane proteins on the cell surface provided by the targeting moiety of 2PBA, thereby realizing the treatment of dry eye disease by 2PBA-si*NFKBIZ*. In the present disclosure, the small-molecule target 2PBA is combined with a small interfering RNA (siRNA) drug for the treatment of dry eye disease. In one aspect, PBA is capable of increasing the retention of the nucleic acid drug on the ocular surface due to its binding to mucin; in another aspect, PBA, due to its recognition role with MUC proteins, enables the *siNFKBIZ* nucleic acid drug to overcome the cell membrane barrier and achieve efficient cellular delivery. Furthermore, in the present disclosure, by means of the key role of IκB-ζ in immune regulation, an siRNA drug is designed, which, in combination with efficient delivery of PBA, can effectively inhibit the expression of the *NFKBIZ* gene. Moreover, compared to the PBA-si*NFKBIZ* covalent conjugate prepared by conjugating a single PBA molecule to an siRNA targeting the *NFKBIZ* gene, the 2PBA-si*NFKBIZ* covalent conjugate formed by conjugating two PBA molecules exhibits better binding to mucins, allowing for efficient delivery of the *siNFKBIZ* nucleic acid drug, such that a good therapeutic effect in the treatment of dry eye disease is achieved.

In one embodiment of the present disclosure, a pharmaceutical composition (4PBA-siNFKBIZ) for inhibiting *NFKBIZ* gene expression is constructed by covalently conjugating a PBA tetramer molecule to *siNFKBIZ* through terminal grafting onto the sense strand of *siNFKBIZ.* The specific preparation method includes:
dissolving a compound of formula 42 and 4-carboxyphenylboronic acid pinacol ester, adding DMTMM and NMM, and performing a reaction under nitrogen atmosphere to obtain a compound of formula 43;
mixing the compound of formula 43, DIEA, acetic anhydride and DMAP, performing a reaction under nitrogen atmosphere, and isolating a compound of formula 44;
dissolving the compound of formula 44 and the compound of formula 37, adding EDCI and DMAP, and performing a reaction under nitrogen atmosphere to obtain a compound of formula 45;
mixing a solution of the compound of formula 45 in acetone with an aqueous solution containing sodium periodate and ammonium acetate, and performing a reaction under nitrogen atmosphere to obtain a compound of formula 46;
adding hydrogen chloride-ethyl acetate to a solution of the compound of formula 46 in ethyl acetate, and performing a reaction under nitrogen atmosphere to obtain a compound of formula 47;
subjecting the compound of formula 47 and a single strand of a nucleic acid molecule modified with an aldehyde group at the 5' end to a click chemical reaction to obtain a 4PBA-si*NFKBIZ*-S conjugate; and
subjecting the 4PBA-si*NFKBIZ*-S conjugate and the complementary strand of the nucleic acid molecule to annealing hybridization to obtain the pharmaceutical composition 4PBA-si*NFKBIZ,* where formula 42 is 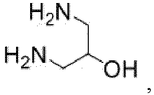 formula 43 is 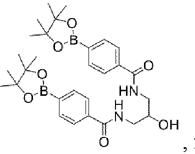 formula 44 is 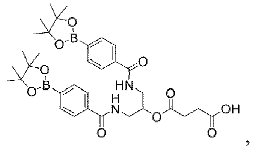 formula 45 is 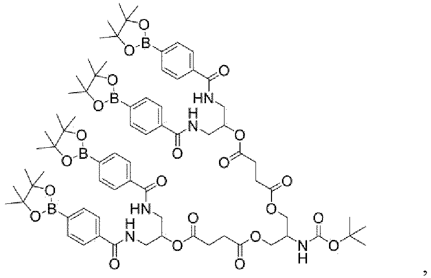 formula 46 is 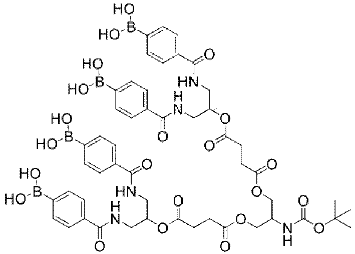 and formula 47 is

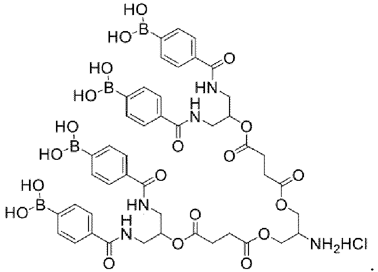

In the present disclosure, the working concentration of the compound of formula 42 is preferably 2.50 mg/mL. The working concentration of the 4-carboxyphenylboronic acid pinacol ester is preferably 20.65 mg/mL. The working concentration of the DMTMM is preferably 23.03 mg/mL. The working concentration of the NMM is preferably 8.43 mg/mL. The temperature of the reaction is preferably 15-45°C, more preferably 25°C. The time for the reaction is preferably 12-48 h, more preferably 20 h.

In the present disclosure, the working concentration of the compound of formula 43 is preferably 17.07 mg/mL. The working concentration of the succinic anhydride is preferably 6.30 mg/mL. The working concentration of the DMAP is preferably 0.33 mg/mL. The reaction conditions are preferably the same as above and will not be described here.

In the present disclosure, the single strand of the nucleic acid molecule modified with an aldehyde group at the 5' end is synthesized by a gene synthesis company. 5'-CHO-si*NFKBIZ*-S is subjected to an electrophilic substitution reaction with the compound of formula 47 to form a Schiff base, which is reduced to obtain a 4PBA-si*NFKBIZ*-S conjugate. The temperature of the electrophilic substitution reaction is preferably 20-55°C, more preferably 40°C. The time for the electrophilic substitution reaction is preferably 12-48 h, more preferably 24 h. The electrophilic substitution reaction is preferably performed with shaking, preferably at a speed of 200-4000 rpm, more preferably at 1800 rpm. After the electrophilic substitution reaction, excess compound of formula 43 and water are preferably removed. The method for removing the compound of formula 47 preferably includes extraction with ethyl acetate. Water is removed by concentrative distillation. The formula of the conjugate is as represented by formula 57: 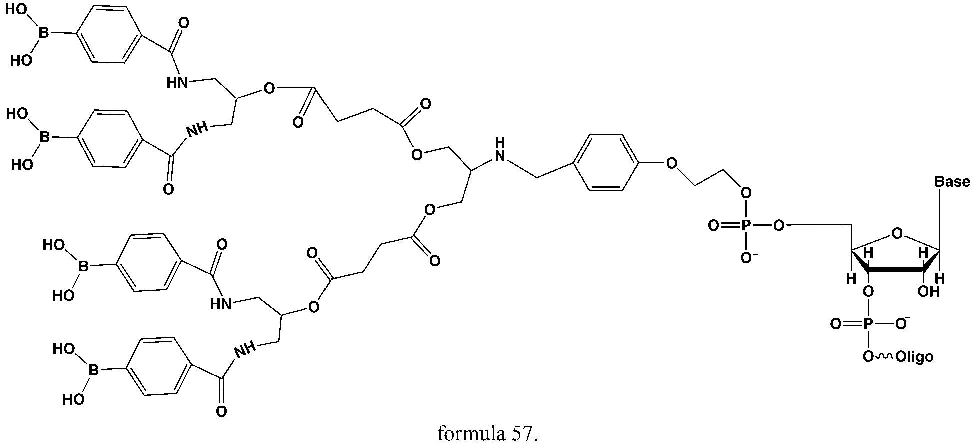 where Oligo 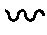 represents an attached nucleic acid molecule, and Base represents any type of base.

In the present disclosure, the pharmaceutical composition (4PBA-siNFKBIZ) enables the use of an *siNFKBIZ* nucleic acid drug in targeted treatment of dry eye disease based on the targeting property of PBA. Boronate esters formed by reacting four phenylboronic acid molecules with 1,2- and 1,3-diol saccharides have strong ocular adhesion, which facilitates the efficient cellular uptake and effectively improves the bioavailability of the nucleic acid drug on the ocular surface. In the present disclosure, 4PBA as a targeting moiety can efficiently target and recognize cells associated with ocular surface tissues. After entering the cells, *siNFKBIZ* can effectively knock down the expression of the inflammation-associated gene *NFKBIZ,* further regulate the protein expression of IκB-ζ, inhibit the inflammatory response of dry eye disease, and block the vicious cycle of inflammation, thereby achieving a better therapeutic effect for dry eye disease.

In one embodiment of the present disclosure, a 3-hydroxy azide compound is first synthesized and reacted with a 2PBA polymer via an esterification reaction to obtain a 6PBA azide-modified compound (6PBA-N₃); next, the compound is successfully grafted onto the end of the sense strand of *siNFKBIZ,* and then annealed with the antisense strand of *siNFKBIZ* for complementation to obtain a pharmaceutical composition (6PBA-si*NFKBIZ*) consisting of a PBA hexamer molecule and an agent inhibiting the expression of the *NFKBIZ* gene. The preparation method for the pharmaceutical composition (6PBA-siNFKBIZ) preferably includes the steps of:
dissolving a compound of formula 48 and azidohexanoic acid, adding DMTMM and NMM, and performing a reaction under nitrogen atmosphere to obtain a compound of formula 49;
dissolving the compound of formula 49 and the compound of formula 44, adding EDCI and DMAP, and performing a reaction under nitrogen atmosphere to obtain a compound of formula 50;
mixing a solution of the compound of formula 50 in acetone with an aqueous solution containing sodium periodate and ammonium carbonate, and performing a reaction under nitrogen atmosphere to obtain a compound of formula 51;
subjecting the compound of formula 51 and one strand of a nucleic acid molecule modified with dibenzocyclooctyne at the 3' end to a reaction with shaking to obtain a 6PBA-si*NFKBIZ*-S conjugate;
subjecting the 6PBA-si*NFKBIZ*-S conjugate and the complementary strand of the nucleic acid molecule to annealing hybridization to obtain the pharmaceutical composition 6PBA-si*NFKBIZ*, where formula 48 is 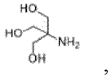 formula 49 is 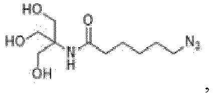 formula 50 is 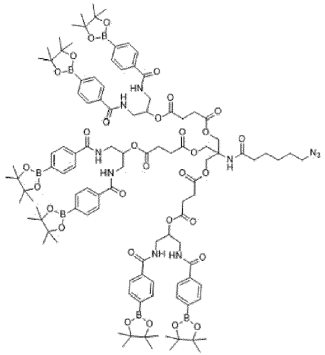 and formula 51 is

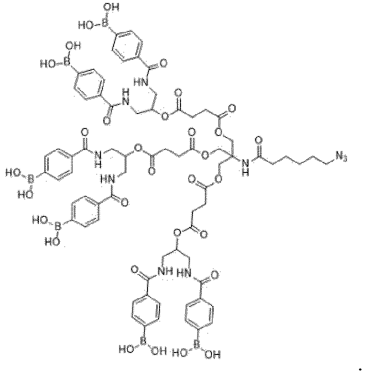

In the present disclosure, the working concentration of the compound of formula 48 is preferably 10.00 mg/mL. The working concentration of the azidohexanoic acid is preferably 23.35 mg/mL. The working concentration of the DMTMM is preferably 34.40 mg/mL. The working concentration of the NMM is preferably 12.40 mg/mL. The reaction conditions are the same as above and will not be described here. After the reaction, the solvent is preferably removed, followed by separation and purification. The method for removal of the solvent is preferably distillation under reduced pressure. The method for separation and purification is preferably silica gel column chromatography. During the separation and purification, the eluent is preferably an ethyl acetate-methanol solution. The volume ratio of ethyl acetate to methanol in the ethyl acetate-methanol solution is preferably 30 : 1.

In the present disclosure, the working concentration of the compound of formula 49 is preferably 3.56 mg/mL. The working concentration of the compound of formula 44 is preferably 35.60 mg/mL. The working concentration of the EDCI is preferably 8.49 mg/mL. The working concentration of the DMAP is preferably 1.00 mg/mL. Conditions for the reaction are the same as above and will not be described here.

In the present disclosure, the preparation of the pharmaceutical composition 6PBA-si*NFKBIZ* from the compound of formula 51 is performed via a click chemical reaction. The single strand of the nucleic acid molecule modified with dibenzocyclooctyne at the 3' end is commissioned to a gene synthesis company for preparation. The temperature of the click chemical reaction is preferably 25-60°C, more preferably 50°C. The time for the click chemical reaction is preferably 12-48 h, more preferably 24 h. The click chemical reaction is preferably performed with shaking, preferably at a speed of 200-4000 rpm, more preferably at 1800 rpm. After the click chemical reaction, excess compound of formula 51 and water are preferably removed. The removal method is preferably extraction with ethyl acetate. Water is removed by concentration by distillation. The formula of the conjugate is as represented by formula 58. 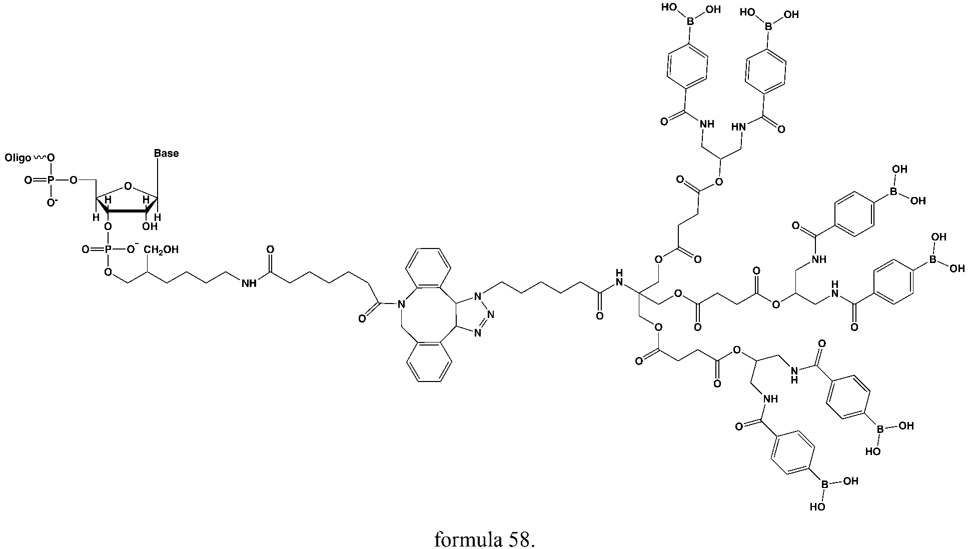 where Oligo 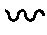 represents an attached nucleic acid molecule, and Base represents any type of base.

In the present disclosure, the pharmaceutical composition 6PBA-si*NFKBIZ* contains a greater number of PBA molecules, which increases the probability of binding to glycosyl groups on the cell membrane surface. This enhances the cell adhesionand facilitates cellular entry via receptor-mediated endocytosis, significantly improving the cellular uptake efficiency of *siNFKBIZ.* The pharmaceutical composition further exerts a gene-silencing effect on the *NFKBIZ* gene in the cells, downregulates the expression of the *NFKBIZ* gene, achieves the downregulation of IκB-ζ, and inhibits cellular inflammation, thereby exerting a therapeutic effect on dry eye disease.

In one embodiment of the present disclosure, a pharmaceutical composition for inhibiting *NFKBIZ* gene expression (PBA₁₀-si*NFKBIZ*) is prepared by grafting 10 phenylboronic acid (PBA) molecules, via phosphorothioate linkages, onto an extension sequence (a DNA strand formed by polymerizing 12 T nucleotides) at the 3' end of the sense strand of a nucleic acid molecule targeting the *NFKBIZ* gene. The preparation method preferably includes the steps of:
artificially synthesizing the sense strand of a nucleic acid molecule with an extension sequence; introducing 10 phosphorothioate modifications into the phosphate backbone of the extension sequence *(10PS-siNFKBIZ-S);*
reacting the 10PS-siNFKBIZwith a 4-bromomethyl-phenylboronic acid solution to obtain a PBA₁₀-si*NFKBIZ*-S conjugate;
subjecting the PBA₁₀-si*NFKBIZ*-S conjugate and the complementary strand of the nucleic acid molecule to annealing hybridization to obtain the pharmaceutical composition PBA₁₀-si*NFKBIZ*,

In the present disclosure, the working concentration of 4-bromomethyl-phenylboronic acid is preferably 60 mM. The working concentration of the 10PS-si*NFKBIZ* is preferably 200 µM. The volume ratio of the *10PS-siNFKBIZ* to the 4-bromomethyl-phenylboronic acid solution is 1: 1. Based on the targeted recognition effect of PBA, the therapeutic effect of the pharmaceutical composition (PBA₁₀-si*NFKBIZ*) in the treatment of dry eye disease is explored. In this embodiment, the 3' end of the sense strand of the *siNFKBIZ* drug is extended by 12 T bases, with phosphorothioate modifications introduced between 10 bases at the end of the extension sequence, and the strand is synthesized using a nucleic acid synthesizer. After PBA is grafted via phosphorothioate linkages, the sense strand is annealed for complementation with the antisense strand of *siNFKBIZ* to obtain PBA₁₀-si*NFKBIZ*. With 10 PBA molecules, the composition exhibits better binding to ocular surface MUC proteins, which enhances the efficiency of interaction with ocular surface proteins, and further increases retention on the ocular surface the intracellular delivery of *siNFKBIZ,* and the biological effects of *siNFKBIZ,* thereby achieving effective treatment of dry eye disease.

In one embodiment of the present disclosure, an *siNFKBIZ* pharmaceutical composition having a stem-loop structure is constructed, into which 3 phosphorothioate modifications are introduced at the stem-loop structure, and 2PBA branched conjugates are grafted onto the stem-loop structure, such that a pharmaceutical composition of *siNFKBIZ* containing six phenylboronic acid molecules (PBA₆-si*NFKBIZ*) is prepared. The preparation method preferably includes the steps of:
subjecting a compound of formula 44 and a compound of formula 52 to an esterification reaction under condensation with EDCI and DMAP to obtain a compound of formula 53;
mixing a solution of the compound of formula 53 in acetone with an aqueous solution containing sodium periodate and ammonium carbonate, and performing a reaction under nitrogen atmosphere to obtain a compound of formula 54 (2PBA-Br);
reacting a solution of the compound of formula 54 with one strand of a modified nucleic acid molecule having 3 phosphorothioate modifications in the stem-loop structure to obtain a PBA₆-si*NFKBIZ*-S conjugate; and
subjecting the PBA₆-si*NFKBIZ*-S conjugate and the complementary strand of the nucleic acid molecule to annealing hybridization to obtain the pharmaceutical composition PBA₆-si*NFKBIZ*, where formula 52 is 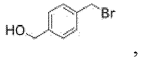 formula 53 is 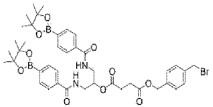 and formula 54 is

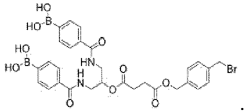

In the present disclosure, the targeted uptake effect of the pharmaceutical composition PBA₆-si*NFKBIZ* in human corneal epithelial cells and the ability thereof to regulate the *NFKBIZ* gene and the IκB-ζ protein are investigated. The results show that PBA₆-si*NFKBIZ* can significantly promote the cellular uptake of the *siNFKBIZ* nucleic acid drug and inhibit the expression of the *NFKBIZ* gene. Further, verification using an animal dry eye disease model demonstrates that the PBA₆-si*NFKBIZ* pharmaceutical composition has good adhesion to the ocular surface, and can better improve the bioavailability of the si*NFKBIZ* nucleic acid drug on the ocular surface, effectively inhibit the expression of the *NFKBIZ* gene, and block the vicious cycle of inflammation on the ocular surface, thereby achieving the treatment of dry eye disease.

The present disclosure provides use of the nucleic acid molecule or the pharmaceutical composition in the preparation of a drug for preventing and/or treating an ocular disease.

In the present disclosure, the ocular disease preferably includes one or more of: dry eye disease, keratitis, conjunctivitis and blepharitis.

The pharmaceutical composition for inhibiting *NFKBIZ* gene expression and the use provided by the present disclosure are described in detail below in conjunction with examples, which, however, should not be construed as limiting the scope of protection of the present disclosure.

### Example 1

### Screen of double-stranded ribonucleic acids that inhibits NFKBIZ gene expression

1. A total of 210 pairs of double-stranded ribonucleic acids (see Table 1) were designed against the *NFKBIZ* gene (GenBank Accession No.: NM_031419.4) by conventional siRNA design methods. In order to preliminarily screen and verify the efficacy of siRNAs, commercial Lipofectamine 2000 was used to transfect human corneal epithelial cells, and the expression of the *NFKBIZ* gene and IκB-ζ protein were verified by qPCR and Western-blot experiments.

**Table 1 siRNA sequences targeting the NFKBIZ gene**

| Nucleic acid molecule No. | SEQ ID NO: | Sense strand sequence | SEQ ID NO: | Antisense strand sequence | Overhang |
|---|---|---|---|---|---|
| 1 | 1 | GAGCCGGUGGCGCAGGU GU | 211 | ACACCUGCGCCACCGGC UC | gc |
| 2 | 2 | UCGGGGUCCUCGAGCGC CC | 212 | GGGCGCUCGAGGACCCC GA | ca |
| 3 | 3 | GGAGCAUGAUUGUGGAC AA | 213 | UUGUCCACAAUCAUGCU CC | ca |
| 4 | 4 | GCAUGAUUGUGGACAAG CU | 214 | AGCUUGUCCACAAUCAU GC | uc |
| 5 | 5 | UGAUUGUGGACAAGCUG CU | 215 | AGCAGCUUGUCCACAAU CA | ug |
| 6 | 6 | GCCGCGGCGGAGAGGGG CU | 216 | AGCCCCUCUCCGCCGCG GC | ug |
| 7 | 7 | CGGCGGGCGGCUGCGGC CU | 217 | AGGCCGCAGCCGCCCGC CG | cg |
| 8 | 8 | CGCUCAACCUGAGCUAC UU | 218 | AAGUAGCUCAGGUUGAG CG | gg |
| 9 | 9 | UGAGCUACUUCUACGGC GC | 219 | GCGCCGUAGAAGUAGCU CA | gg |
| 10 | 10 | GGCUCGCCCGGCUCCGA CU | 220 | AGUCGGAGCCGGGCGAG CC | gg |
| 11 | 11 | UCCUCUGCCUCGUCGGU GU | 221 | ACACCGACGAGGCAGAG GA | ga |
| 12 | 12 | GCGCCGAGCGCCAGCCA GU | 222 | ACUGGCUGGCGCUCGGC GC | gg |
| 13 | 13 | CGCCGAGCGCCAGCCAG UU | 223 | AACUGGCUGGCGCUCGG CG | cg |
| 14 | 14 | CAGCCAGUUGAGCCCCA UA | 224 | UAUGGGGCUCAACUGGC UG | gc |
| 15 | 15 | GCCCCAUAUGGGGUUG GC | 225 | GCCAACCCCCAUAUGGG GC | uc |
| 16 | 16 | GCAGCAGAGAGGCCCCU UU | 226 | AAAGGGGCCUCUCUGCU GC | cu |
| 17 | 17 | AGGUGUUCGGUAAAGA AC | 227 | GUUCUUUACCCGAACAC CU | ug |
| 18 | 18 | UUCGGGUAAAGAACUCA GU | 228 | ACUGAGUUCUUUACCCG AA | ca |
| 19 | 19 | CGGGUAAAGAACUCAGU GA | 229 | UCACUGAGUUCUUUACC CG | aa |
| 20 | 20 | GGGUAAAGAACUCAGUG AA | 230 | UUCACUGAGUUCUUUAC CC | ga |
| 21 | 21 | GGUAAAGAACUCAGUGA AG | 231 | CUUCACUGAGUUCUUUA CC | cg |
| 22 | 22 | AGAACUCAGUGAAGGAA CU | 232 | AGUUCCUUCACUGAGUU CU | uu |
| 23 | 23 | ACUCAGUGAAGGAACUC CU | 233 | AGGAGUUCCUUCACUGA GU | uc |
| 24 | 24 | GUUGCACAUCCGAAGUC AU | 234 | AUGACUUCGGAUGUGCA AC | ag |
| 25 | 25 | GCACAUCCGAAGUCAUA AA | 235 | UUUAUGACUUCGGAUGU GC | aa |
| 26 | 26 | AAGUCAUAAACAGAAGG CU | 236 | AGCCUUCUGUUUAUGAC UU | cg |
| 27 | 27 | AGUCAUAAACAGAAGGC UU | 237 | AAGCCUUCUGUUUAUGA CU | uc |
| 28 | 28 | GGCCAAGCUGUGGAUGA UU | 238 | AAUCAUCCACAGCUUGG CC | ag |
| 29 | 29 | GCCAAGCUGUGGAUGAU UU | 239 | AAAUCAUCCACAGCUUG GC | ca |
| 30 | 30 | GCUGUGGAUGAUUUUAA GA | 240 | UCUUAAAUCAUCCACA GC | uu |
| 31 | 31 | GGAUGAUUUUAAGACAC AA | 241 | UUGUGUCUUAAAAUCAU CC | ac |
| 32 | 32 | GACACAAGGUGUGAACA UA | 242 | UAUGUUCACACCUUGUG UC | uu |
| 33 | 33 | CAUAGAACAGUUCAGAG AA | 243 | UUCUCUGAACUGUUCUA UG | uu |
| 34 | 34 | AGUUCAGAGAAUUGAAG AA | 244 | UUCUUCAAUUCUCUGAA CU | gu |
| 35 | 35 | GCCCGAUUCGUUGUCUG AU | 245 | AUCAGACAACGAAUCGG GC | cc |
| 36 | 36 | CGGGGAGAGCAUGGAAG AU | 246 | AUCUUCCAUGCUCUCCC CG | gg |
| 37 | 37 | GGGAGAGCAUGGAAGAU GU | 247 | ACAUCUUCCAUGCUCUC CC | cg |
| 38 | 38 | GGAAGAUGUUCAUCUCA AU | 248 | AUUGAGAUGAACAUCUU CC | au |
| 39 | 39 | AGAUGUUCAUCUCAAUG AA | 249 | UUCAUUGAGAUGAACAU CU | uc |
| 40 | 40 | GAUGUUCAUCUCAAUGA AC | 250 | GUUCAUUGAGAUGAACA UC | uu |
| 41 | 41 | GAUCUGCUUCAGAACAU UA | 251 | UAAUGUUCUGAAGCAGA UC | ag |
| 42 | 42 | GCUUCAGAACAUUAUCA AC | 252 | GUUGAUAAUGUUCUGAA GC | ag |
| 43 | 43 | AGAACAUUAUCAACAUU AA | 253 | UUAAUGUUGAUAAUGUU CU | ga |
| 44 | 44 | UUAUCAACAUUAAGAAU GA | 254 | UCAUUCUUAAUGUUGAU AA | ug |
| 45 | 45 | CCCUGAACACAGUUCAA GU | 255 | ACUUGAACUGUGUUCAG GG | aa |
| 46 | 46 | CUCAGAAAUGCCAACCA UU | 256 | AAUGGUUGGCAUUUCUG AG | gu |
| 47 | 47 | GGGCUCCCAACAAAUGA UA | 257 | UAUCAUUUGUUGGGAGC CC | cu |
| 48 | 48 | GGCUUCCCUGUACCAGU AU | 258 | AUACUGGUACAGGGAAG CC | ug |
| 49 | 49 | GCCACACUACACCCACA AA | 259 | UUUGUGGGUGUAGUGUG GC | ug |
| 50 | 50 | CACAAACCAACUCUGGA AU | 260 | AUUCCAGAGUUGGUUUG UG | gg |
| 51 | 51 | CCCCCGCUUAUGAACCA AA | 261 | UUUGGUUCAUAAGCGGG GG | ac |
| 52 | 52 | GGUCCAGAAUCACAGUU UU | 262 | AAAACUGUGAUUCUGGA CC | au |
| 53 | 53 | CAGUUUUGCCCAAACCA AA | 263 | UUUGGUUUGGCAAAAC UG | ug |
| 54 | 54 | GCCCAAACCAAAGCUUA GU | 264 | ACUAAGCUUUGGUUUGG GC | aa |
| 55 | 55 | CCCAAACCAAAGCUUAG UU | 265 | AACUAAGCUUUGGUUUG GG | ca |
| 56 | 56 | CCAGUGUUCAGCAGCAA AA | 266 | UUUUGCUGCUGAACACU GG | ag |
| 57 | 57 | AUGAUGCCCAGCAGCGC CU | 267 | AGGCGCUGCUGGGCAUC AU | gc |
| 58 | 58 | GGGGCACGAGAUGGCCU CU | 268 | AGAGGCCAUCUCGUGCC CC | ac |
| 59 | 59 | GCACGAGAUGGCCUCUG AC | 269 | GUCAGAGGCCAUCUCGU GC | cc |
| 60 | 60 | UCACUGCCAUUCUCAAA CA | 270 | UGUUUGAGAAUGGCAGU GA | ag |
| 61 | 61 | CAGUUAGGGAAAUCACU UU | 271 | AAAGUGAUUUCCCUAAC UG | ug |
| 62 | 62 | GUUAGGGAAAUCACUUU UU | 272 | AAAAAGUGAUUUCCCUA AC | ug |
| 63 | 63 | GGGAAAUCACUUUUUCA GU | 273 | ACUGAAAAGUGAUUUC CC | ua |
| 64 | 64 | GGAGCAGGAAGAAAGCA AA | 274 | UUUGCUUUCUUCCUGCU CC | ac |
| 65 | 65 | CAAGACCAGUUUCUUUC AA | 275 | UUGAAAGAAACUGGUCU UG | gg |
| 66 | 66 | GAUGCAGAUGGUGACAC GU | 276 | ACGUGUCACCAUCUGCA UC | cu |
| 67 | 67 | GUGACACGUUCCUUCAU AU | 277 | AUAUGAAGGAACGUGUC AC | ca |
| 68 | 68 | CCUAUGUUCUUGCAAGA AA | 278 | UUUCUUGCAAGAACAUA GG | aa |
| 69 | 69 | UUCACAUGCUGGAUAUU AA | 279 | UUAAUAUCCAGCAUGUG AA | gu |
| 70 | 70 | UCACAUGCUGGAUAUUA AA | 280 | UUUAAUAUCCAGCAUGU GA | ag |
| 71 | 71 | AGAGCACAAUGGACAGA GU | 281 | ACUCUGUCCAUUGUGCU CU | uu |
| 72 | 72 | CUUUCAGGUGGCAGUGG CU | 282 | AGCCACUGCCACCUGAA AG | gc |
| 73 | 73 | GCUGCCAAUCAGCAUCU CA | 283 | UGAGAUGCUGAUUGGCA GC | ca |
| 74 | 74 | GGCACAGGUGAACACCA CA | 284 | UGUGGUGUUCACCUGUG CC | cc |
| 75 | 75 | CAGGUGAACACCACAGA CU | 285 | AGUCUGUGGUGUUCACC UG | ug |
| 76 | 76 | GGGGAAGAACACCUCUG CA | 286 | UGCAGAGGUGUUCUUCC CC | ag |
| 77 | 77 | GGGCCACUCCCAGGUGC UU | 287 | AAGCACCUGGGAGUGGC CC | uu |
| 78 | 78 | CCUUCACUGUGCAGUCA UA | 288 | UAUGACUGCACAGUGAA GG | gg |
| 79 | 79 | CCAGAGAAAUCAACAGC CU | 289 | AGGCUGUUGAUUUCUCU GG | ag |
| 80 | 80 | GAGCUUUUACUGAAGAA UA | 290 | UAUUCUUCAGUAAAAGC UC | cu |
| 81 | 81 | AGCUUUUACUGAAGAAU AA | 291 | UUAUUCUUCAGUAAAAG CU | cc |
| 82 | 82 | GAAUAAGAGUCUGGUUG AU | 292 | AUCAACCAGACUCUUAU UC | uu |
| 83 | 83 | GUGCCUAAUUCAAAUGG GA | 293 | UCCCAUUUGAAUUAGGC AC | uu |
| 84 | 84 | GCCGCACAGCCCUGCAU UU | 294 | AAAUGCAGGGCUGUGCG GC | ca |
| 85 | 85 | GCUGAAGAAGCAAAUCU GG | 295 | CCAGAUUUGCUUCUUCA GC | ug |
| 86 | 86 | GGAACUCAUUCGCCUCU UU | 296 | AAAGAGGCGAAUGAGUU CC | ag |
| 87 | 87 | UUUUGUGAAUGCAAAGG CU | 297 | AGCCUUUGCAUUCACAA AA | ga |
| 88 | 88 | UUUGUGAAUGCAAAGGC UU | 298 | AAGCCUUUGCAUUCACA AA | ag |
| 89 | 89 | GCCCUCCAUGUUGCUGC CA | 299 | UGGCAGCAACAUGGAGG GC | ag |
| 90 | 90 | CCAGCUUGCAGUAUCGG UU | 300 | AACCGAUACUGCAAGCU GG | ca |
| 91 | 91 | GUAUCGGUUGACACAAU UA | 301 | UAAUUGUGUCAACCGAU AC | ug |
| 92 | 92 | GGAGCAGACCCAAGUAC UC | 302 | GAGUACUUGGGUCUGCU CC | cu |
| 93 | 93 | GAACGAACAGCCAGUGC AU | 303 | AUGCACUGGCUGUUCGU UC | uc |
| 94 | 94 | GUUCCCGAUGGCCCUGU GG | 304 | CCACAGGGCCAUCGGGA AC | ca |
| 95 | 95 | GUGGGAGAACAGAUCCG AC | 305 | GUCGGAUCUGUUCUCCC AC | ag |
| 96 | 96 | GCAGAGAGCUCCACCGU AU | 306 | AUACGGUGGAGCUCUCU GC | ug |
| 97 | 97 | AGAGAGCUCCACCGUAU UA | 307 | UAAUACGGUGGAGCUCU CU | gc |
| 98 | 98 | AUAGACCAUUUGCCUUA UA | 308 | UAUAAGGCAAAUGGUCU AU | gu |
| 99 | 99 | CCUUAUAUUGGCAAAUG UA | 309 | UACAUUUGCCAAUAUAA GG | ca |
| 100 | 100 | GUAAGUUGUUUCUAUGA AA | 310 | UUUCAUAGAAACAACUU AC | au |
| 101 | 101 | AGUUGUUUCUAUGAAAC AA | 311 | UUGUUUCAUAGAAACAA CU | ua |
| 102 | 102 | GAAACAAACAUAUUUAG UU | 312 | AACUAAAUAUGUUUGUU UC | au |
| 103 | 103 | CAUAUUUAGUUCACUAU UA | 313 | UAAUAGUGAACUAAAUA UG | uu |
| 104 | 104 | AUAUUUAGUUCACUAUU AU | 314 | AUAAUAGUGAACUAAAU AU | gu |
| 105 | 105 | AUAUAGUGGGUUAUAUU AA | 315 | UUAAUAUAACCCACUAU AU | aa |
| 106 | 106 | UAUAGUGGGUUAUAUUA AA | 316 | UUUAAUAUAACCCACUA UA | ua |
| 107 | 107 | AUAGUGGGUUAUAUUAA AA | 317 | UUUUAAUAUAACCCACU AU | au |
| 108 | 108 | GGGUUAUAUUAAAAGAA AA | 318 | UUUCUUUUAAUAUAAC CC | ac |
| 109 | 109 | AUAUUAAAAGAAAAGAA GA | 319 | UCUUCUUUUCUUUUAAU AU | aa |
| 110 | 110 | UAUUAAAAGAAAAGAAG AA | 320 | UUCUUCUUUUCUUUUAA UA | ua |
| 111 | 111 | UUAAAAGAAAAGAAGAA AA | 321 | UUUUCUUCUUUUCUUUU AA | ua |
| 112 | 112 | UAAAAGAAAAGAAGAAA AA | 322 | UUUUUCUUCUUUCUUU UA | au |
| 113 | 113 | AAAAGAAAAGAAGAAAA AU | 323 | AUUUUUCUUCUUUUCUU UU | aa |
| 114 | 114 | AAAGAAAAGAAGAAAAA UA | 324 | UAUUUUUCUUCUUUUCU UU | ua |
| 115 | 115 | AAGAAAAGAAGAAAAAU AU | 325 | AUAUUUUUCUUCUUUUC UU | uu |
| 116 | 116 | GAUCUAGACAUCUGAAU UU | 326 | AAAUUCAGAUGUCUAGA UC | cc |
| 117 | 117 | GAAUUUGAUCUCAAUGG UA | 327 | UACCAUUGAGAUCAAAU UC | ag |
| 118 | 118 | GUAGCUUUUGAGUAGGA AA | 328 | UUUCCUACUCAAAAGCU AC | ug |
| 119 | 119 | UUUUGAGUAGGAAAGGA CU | 329 | AGUCCUUUCCUACUCAA AA | gc |
| 120 | 120 | GAGUAGGAAAGGACUUU GA | 330 | UCAAAGUCCUUUCCUAC UC | aa |
| 121 | 121 | GGAAAGGACUUUGAUUU GU | 331 | ACAAAUCAAAGUCCUUU CC | ua |
| 122 | 122 | GUGGCACAAAACAUUAU UA | 332 | UAAUAAUGUUUGUGCC AC | aa |
| 123 | 123 | GGCACAAAACAUUAUUA AU | 333 | AUUAAUAAUGUUUUGUG CC | ac |
| 124 | 124 | GCACAAAACAUUAUUAA UA | 334 | UAUUAAUAAUGUUUGU GC | ca |
| 125 | 125 | ACAAAACAUUAUUAAUA UA | 335 | UAUAUUAAUAAUGUUUU GU | gc |
| 126 | 126 | AGCUAUUGACAGUUUCA AA | 336 | UUUGAAACUGUCAAUAG CU | au |
| 127 | 127 | UACUGGCGUUGGACAGG CU | 337 | AGCCUGUCCAACGCCAG UA | ac |
| 128 | 128 | GCGUUGGACAGGCUUCA GU | 338 | ACUGAAGCCUGUCCAAC GC | ca |
| 129 | 129 | GGCUUCAGUCAUUGGAC UA | 339 | UAGUCCAAUGACUGAAG CC | ug |
| 130 | 130 | UGGACUAGAUGAAAGGU GU | 340 | ACACCUUUCAUCUAGUC CA | au |
| 131 | 131 | GGACUAGAUGAAAGGUG UC | 341 | GACACCUUUCAUCUAGU CC | aa |
| 132 | 132 | GUAUAUAAUUGUUAUUU UU | 342 | AAAAAUAACAAUUAUAU AC | ag |
| 133 | 133 | UUAUUUUUGUCCUUAAA AA | 343 | UUUUUAAGGACAAAAAU AA | ca |
| 134 | 134 | GUACAUACUUGGUUGUU AA | 344 | UUAACAACCAAGUAUGU AC | aa |
| 135 | 135 | GGUUGUUAACAUGGUCA UA | 345 | UAUGACCAUGUUAACAA CC | aa |
| 136 | 136 | UGGUCAUAUUUGAAAUG UA | 346 | UACAUUUCAAAUAUGAC CA | ug |
| 137 | 137 | GGUCAUAUUUGAAAUGU AU | 347 | AUACAUUUCAAAUAUGA CC | au |
| 138 | 138 | CAUAAAAUAGAAAAGAA CA | 348 | UGUUCUUUCUAUUUUA UG | ga |
| 139 | 139 | AUAAAAUAGAAAAGAAC AA | 349 | UUGUUCUUUUCUAUUUU AU | gg |
| 140 | 140 | UAGAAAAGAACAAGUGA AU | 350 | AUUCACUUGUUCUUUUC UA | uu |
| 141 | 141 | AGUGAAUUGUUGCUAUU UA | 351 | UAAAUAGCAACAAUUCA CU | ug |
| 142 | 142 | GUGAAUUGUUGCUAUUU AA | 352 | UUAAAUAGCAACAAUUC AC | uu |
| 143 | 143 | GUUGCUAUUUAAAAAA UU | 353 | AAUUUUUUUAAAUAGCA AC | aa |
| 144 | 144 | GCUAUUUAAAAAAAUUU UA | 354 | UAAAAUUUUUUAAAUA GC | aa |
| 145 | 145 | UUAAAAAAAUUUUACAA UU | 355 | AAUUGUAAAAUUUUUUU AA | au |
| 146 | 146 | UAAAAAAAUUUUACAAU UC | 356 | GAAUUGUAAAAUUUUUU UA | aa |
| 147 | 147 | ACAAUUCUUACUAAGGA GU | 357 | ACUCCUUAGUAAGAAUU GU | aa |
| 148 | 148 | AGGAGUUUUAUUGUGU AA | 358 | UUACACAAUAAAAACUC CU | ua |
| 149 | 149 | ACUAAGUCUUUGUAGAU AA | 359 | UUAUCUACAAAGACUUA GU | ga |
| 150 | 150 | CUAAGUCUUUGUAGAUA AA | 360 | UUUAUCUACAAAGACUU AG | ug |
| 151 | 151 | GCUGAAAGAUAUAUUCG AA | 361 | UUCGAAUAUAUCUUUCA GC | ca |
| 152 | 152 | AGAUAUAUUCGAAUUGU AA | 362 | UUACAAUUCGAAUAUAU CU | uu |
| 153 | 153 | GAUAUAUUCGAAUUGUA AA | 363 | UUUACAAUUCGAAUAUA UC | uu |
| 154 | 154 | GCAUUGAAAUUAUACAU UA | 364 | UAAUGUAUAAUUUCAAU GC | au |
| 155 | 155 | CAUUGAAAUUAUACAUU AU | 365 | AUAAUGUAUAAUUUCAA UG | ca |
| 156 | 156 | UACAUUAUUUGUAGGGA AU | 366 | AUUCCCUACAAAUAAUG UA | ua |
| 157 | 157 | UAUUUGUAGGGAAUUGC AU | 367 | AUGCAAUUCCCUACAAA UA | au |
| 158 | 158 | GAAUUGCAUGCUUUUUU UU | 368 | AAAAAAAAGCAUGCAAU UC | cc |
| 159 | 159 | GGCUAAUUUUUAUUUU UU | 369 | AAAAAAUAAAAAAUUAG CC | ag |
| 160 | 160 | GGCUGGCAGUUGCAUGG AA | 370 | UUCCAUGCAACUGCCAG CC | gg |
| 161 | 161 | GCAGUUGCAUGGAAGAG AA | 371 | UUCUCUUCCAUGCAACU GC | ca |
| 162 | 162 | GGAAGAGAACACCUCUU UA | 372 | UAAAGAGGUGUUCUCUU CC | au |
| 163 | 163 | GGCUUACCCUCUAGAAU UU | 373 | AAAUUCUAGAGGGUAAG CC | au |
| 164 | 164 | CUCUAGAAUUUCUAAUU UA | 374 | UAAAUUAGAAAUUCUAG AG | gg |
| 165 | 165 | UGAAAUUUUGUUUUUU UA | 375 | UAAAAAAACAAAAAUUU CA | ac |
| 166 | 166 | CCUUUAUUGAAACAACA AA | 376 | UUUGUUGUUUCAAUAAA GG | ua |
| 167 | 167 | ACAAAAAGUCAGUAUUG AA | 377 | UUCAAUACUGACUUUUU GU | ug |
| 168 | 168 | CCUGUUUCUGUUGUCA AA | 378 | UUUGACAACAGAAAACA GG | aa |
| 169 | 169 | CCAUGAUGUUUAUAUA UA | 379 | UAUAUAUAAAACAUCAU GG | ca |
| 170 | 170 | GAAAAGUUUUAUUUUU UA | 380 | UAAAAAAUAAAACUUUU UC | ug |
| 171 | 171 | AAAAAGUUUUAUUUUUU AA | 381 | UUAAAAAAUAAAACUUU UU | cu |
| 172 | 172 | AAAGUUUUAUUUUUUAA UA | 382 | UAUUAAAAAUAAAACU UU | uu |
| 173 | 173 | AAGUUUUAUUUUUUAAU AA | 383 | UUAUUAAAAAAUAAAAC UU | uu |
| 174 | 174 | GGCUUUGAUUACACACU AA | 384 | UUAGUGUGUAAUCAAAG CC | aa |
| 175 | 175 | ACACUAAGUUUUUGUAA UA | 385 | UAUUACAAAAACUUAGU GU | gu |
| 176 | 176 | ACUAAGUUUUGUAAUA AA | 386 | UUUAUUACAAAAACUUA GU | gu |
| 177 | 177 | CUAAGUUUUGUAAUAA AU | 387 | AUUUAUUACAAAAACUU AG | ug |
| 178 | 178 | GUAAUAAAUUUGACUCA UU | 388 | AAUGAGUCAAAUUUAUU AC | aa |
| 179 | 179 | AAAUUUGACUCAUUAAA AA | 389 | UUUUUAAUGAGUCAAAU UU | au |
| 180 | 180 | UAAAAACCUUUUUUUU UA | 390 | UAAAAAAAAAGGUUUU UA | au |
| 181 | 181 | AAAAACCUUUUUUUUU AA | 391 | UUAAAAAAAAAAGGUUU UU | aa |
| 182 | 182 | AAAACCUUUUUUUUUUA AA | 392 | UUUAAAAAAAAAAGGUU UU | ua |
| 183 | 183 | AAACCUUUUUUUUUUAA AA | 393 | UUUUAAAAAAAAAAGGU UU | uu |
| 184 | 184 | ACCUUUUUUUUUUAAAA AA | 394 | UUUUUUAAAAAAAAAAG GU | uu |
| 185 | 185 | CCUUUUUUUUUAAAAA AA | 395 | UUUUUUUAAAAAAAAAA GG | uu |
| 186 | 186 | CUUUUUUUUUUAAAAAA AA | 396 | UUUUUUUUAAAAAAAAA AG | gu |
| 187 | 187 | UUUUUUUUUAAAAAAA AA | 397 | UUUUUUUUAAAAAAAA AA | gg |
| 188 | 188 | UUUUUUUUAAAAAAAA AA | 398 | UUUUUUUUUAAAAAAA AA | ag |
| 189 | 189 | UUUUUUUUAAAAAAAAA AA | 399 | UUUUUUUUUUAAAAAA AA | aa |
| 190 | 190 | UUUUUUUAAAAAAAAA AA | 400 | UUUUUUUUUUUAAAAA AA | aa |
| 191 | 191 | UUUUUUAAAAAAAAAAAA AA | 401 | UUUUUUUUUUUUAAAA AA | aa |
| 192 | 192 | UUUUUAAAAAAAAAAAA AA | 402 | UUUUUUUUUUUUUUAAA AA | aa |
| 193 | 193 | UUUUAAAAAAAAAAAAA AA | 403 | UUUUUUUUUUUUUUAA AA | aa |
| 194 | 194 | UUUAAAAAAAAAAAAA AG | 404 | CUUUUUUUUUUUUUUUA AA | aa |
| 195 | 195 | UUAAAAAAAAAAAAAA GA | 405 | UCUUUUUUUUUUUUUUU AA | aa |
| 196 | 196 | UAAAAAAAAAAAAAAG AA | 406 | UUCUUUUUUUUUUUUUUU UA | aa |
| 197 | 197 | AAAAAAAAAAAAAAAGA AA | 407 | UUCUUUUUUUUUUUUU UU | aa |
| 198 | 198 | AAAAAAAAAAAAAAGAA AA | 408 | UUUUCUUUUUUUUUUUU UU | ua |
| 199 | 199 | AAAAAAAAAAAAGAAA AU | 409 | AUUUCUUUUUUUUUU UU | uu |
| 200 | 200 | AAAAAAAAAAAGAAAA UC | 410 | GAUUUCUUUUUUUUU UU | uu |
| 201 | 201 | AAAAAAAAAAGAAAAU CU | 411 | AGAUUUCUUUUUUUUU UU | uu |
| 202 | 202 | AGAAAAUCUCAUUAGUG AA | 412 | UUCACUAAUGAGAUUUU CU | uu |
| 203 | 203 | GUACUUAAAUUCUUUUU AA | 413 | UUAAAAAGAAUUUAAGU AC | uc |
| 204 | 204 | AAUUCUUUUUAAAAAGA UA | 414 | UAUCUUUUUAAAAAGAA UU | ua |
| 205 | 205 | CGUUAGUUAUAAUCACC UU | 415 | AAGGUGAUUAUAACUAA CG | ug |
| 206 | 206 | AACUGAUGGUUUUAAAU AA | 416 | UUAUUUAAAACCAUCAG UU | uu |
| 207 | 207 | ACUGAUGGUUUUAAAUA AA | 417 | UUUAUUUAAACCAUCA GU | uu |
| 208 | 208 | GAUGGUUUUAAAUAAAC UA | 418 | UAGUUUAUUUAAAACCA UC | ag |
| 209 | 209 | GGUUUUAAAUAAACUAA UU | 419 | AAUUAGUUUAUUUAAAA CC | au |
| 210 | 210 | AUAAACUAAUUCACUAA UA | 420 | UAUUAGUGAAUUAGUUU AU | uu |

### 2. Screen of double-stranded ribonucleic acids that inhibits NFKBIZ gene expression based on qPCR

Human corneal epithelial cells (HCECs) were seeded at a density of 3.5 × 10⁴ cells/well in a 6-well plate and cultured overnight before transfection. Lipofectamine 2000 (Invitrogen GmbH, Karlsruhe, Germany) was incubated with siRNA in OptiMEM medium according to the manufacturer's instructions, and then added to the 6-well plate. A Lipofectamine 2000 blank control group was set up. Six hours after transfection, the Opti-MEM medium was replaced with complete medium. 48 h after medium replacement, total RNA was extracted using the Beyotime RNA extraction kit, and reverse transcription was performed with the TAKARA reverse transcription kit (Code No. RR036A). The reverse transcription reaction solution was prepared according to the following components (the preparation was performed on ice).

**Table 2 Reverse transcription system**

| Reagent | Volume/amount | Final concentration |
|---|---|---|
| 5× PrimeScript RT Master Mix Perfect Real Time | 4 µL | 1× |
| Total RNA | 1000 ng | |
| RNase free H₂O | up to 20 µL | |

The specific reaction procedures are as follows: First, the reaction was performed at 37°C for 15 min (reverse transcription reaction), then at 85°C for 5 s (reverse transcriptase inactivation reaction), then the reaction was cooled to 4°C, and finally the sample was taken out and stored at -20°C. The qPCR reaction was performed according to the TAKARA real-time quantitative PCR kit (Code No. RR820A) with GAPDH as the internal reference gene. The primers are as follows: GAPDH forward primer: AGAAGGCTGGGGCTCATTTG (SEQ ID NO: 427); and GAPDH reverse primer: AGGGGCCATCCACAGTCTTC (SEQ ID NO: 428).

NKFBIZ forward primer: ACACCCACAAACCAACTCTGG (SEQ ID NO: 429);
*NFKBIZ* reverse primer: GGCAAAACTGTGATTCTGGACC (SEQ ID NO: 430). The PCR reaction solution was prepared according to the following components (the preparation was performed on ice).

**Table 3 qPCR reaction system**

| Reagent | Volume | Volume | Final concentration |
|---|---|---|---|
| TB GreenPremix Ex TaqII (Tli RNaseH Plus) (2×) | 10 µl | 25 µl | 1× |
| PCR forward primer (10 µM) | 0.8 µl | 2 µl | 0.4 µM × 1 |
| PCR reverse primer (10 µM) | 0.8 µl | 2 µl | 0.4 µM × 1 |
| ROX Reference Dye (50×) or ROX Reference Dye II (50×) × 2 | 0.4 µl | 1 µl | 1× |
| DNA template × 3 | 2 µl | 4 µl | |
| Sterile water | 6 µl | 16 µl | |
| Total | 20 µl × 4 | 50 µl × 4 | |

The specific reaction procedures are as follows: First stage: Pre-denaturation; Number of cycles: 1; 95°C for 30 s; Second stage: PCR reaction, number of cycles: 40; 95°C for 3 s; 60°C for 30 s; Melting curve stage.

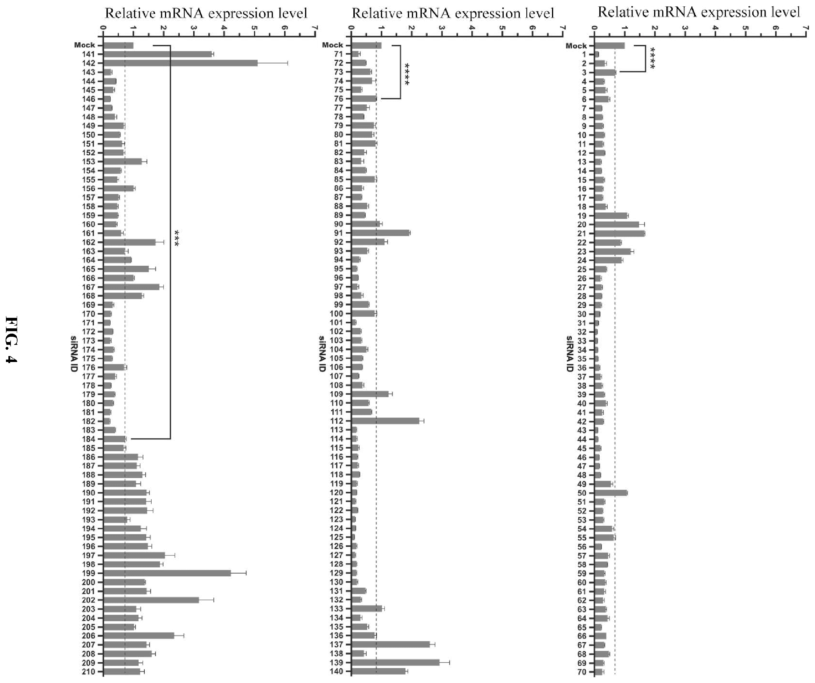

The results are shown in FIG. 4. The Mock group represents the baseline *NFKBIZ* mRNA expression level in cells without siRNA treatment. Using this group as the normalization reference, siRNA sequences that significantly inhibited *NFKBIZ* gene expression compared to the Mock group (sequences with mRNA expression levels lower than or equal to the dashed line in FIG. 4) were selected as preferred sequences. Through quantitative analysis of significant differences, ID1-18, ID25-49, ID51-89, ID93-108, ID113-132, ID134-135, ID143-152, and ID169-185 were found to be capable of significantly inhibiting the mRNA expression of the *NFKBIZ* gene.

### 3. Verification of the biological effects of double-stranded ribonucleic acids inhibiting NFKBIZ gene expression based on Western-blot

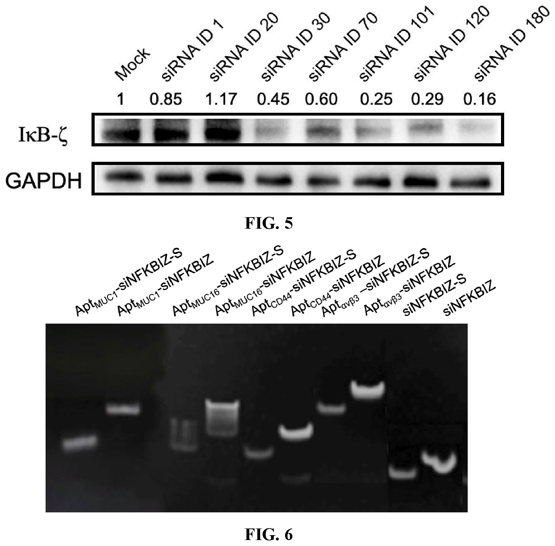

To better verify the inhibitory effect of siRNAs on the *NFKBIZ* gene, seven siRNA sequences were selected to detect the expression level of the IκB-ζ protein. Human corneal epithelial cells (HCECs) were seeded at a density of 3.5 × 10⁴ cells/well in a 6-well plate and cultured overnight before transfection. Lipofectamine 2000 was incubated with siRNA in Opti-MEM medium according to the manufacturer's instructions, then added to the 6-well plate. A lipofectamine 2000 blank control group was set up. Six hours after transfection, the OptiMEM medium was replaced with complete medium. Forty-eight hours after medium replacement, total protein was extracted for detection. The results are shown in FIG. 5. It was shown that the selected siRNA ID1, siRNA ID30, siRNA ID70, siRNA ID101, siRNA ID120, and siRNA ID180 were all capable of inhibiting the expression of IκB-ζ at the protein level, which was consistent with the qPCR results. This indicates that these siRNAs are not only capable of inhibiting the expression of the *NFKBIZ* gene at the mRNA level, but also inhibiting the expression of IκB-ζ protein, thereby exerting an anti-inflammatory effect.

### Example 2

### Targeted delivery of siNFKBIZ-based nucleic acid pharmaceutical compositions by aptamers for the treatment of dry eye disease

Aptamers are short single-stranded DNA or RNA molecules that can selectively bind to specific targets, including proteins, peptides, carbohydrates, small molecules, toxins, etc. Compared to other targeting ligands (such as antibodies), aptamers have lower toxicity and immunogenicity. In addition, aptamers hold broad application prospects in the field of oligonucleotide delivery due to their advantages such as ease of synthesis, chemical modification and conjugation. Mucins are a key factor in tear film quality and tear film stability. On the ocular surface, mucins are secreted by conjunctival goblet cells and lacrimal glands, with MUC1, MUC4 and MUC16 being the major transmembrane mucins highly expressed at the ocular surface. Corneal epithelial cells have been shown to express integrins and are a source of inflammatory cytokines in dry eye disease. In addition, inflammatory Th17 cells express a large amount of α_{V}β₃ integrin, which is essential to maintain the Th17 inflammatory phenotype, such as the production of IL-17 in dry eye disease and IL-17-induced disruption of the corneal epithelial barrier. To achieve efficient delivery of siRNA, in this example, an siNFKBIZ-based nucleic acid pharmaceutical composition was developed based on aptamers targeting MUC1, MUC16, integrin α_{V}β₃ and CD44 protein for the treatment of dry eye disease, an ocular surface disease.

### 1. Assembly of pharmaceutical compositions for targeted delivery of siNFKBIZ based on aptamers

First, in this example, two aptamers targeting MUC proteins, namely the aptamer targeting MUC1 protein (Apt_{MUC1} aptamer) and the aptamer targeting MUC16 protein (Apt_{MUC16}), as well as the aptamer targeting integrin α_{V}β₃ (Apt_{αvβ3} aptamer) and the aptamer targeting CD44 protein (Apt_{CD44} aptamer) were selected. The aptamers were each conjugated to the sense strand (si*NFKBIZ* sense, SEQ ID NO: 1) of siRNA targeting the *NFKBIZ* gene via the deoxyribonucleic acid linker TTTTT, and then assembled with the antisense strand (si*NFKBIZ* antisense, SEQ ID NO: 211) of siRNA that inhibits the *NFKBIZ* gene through complementary base pairing. The assembly was verified by non-denaturing PAGE. The results in FIG. 6 showed that Apt-si*NFKBIZ* sense was successfully hybridized with si*NFKBIZ* antisense.

The sequences of the si*NFKBIZ* used in this example are as follows:
The sequence of small interfering RNA targeting the human *NFKBIZ* gene (si*NFKBIZ*) includes:
Sense: 5'-GAGCCGGUGGCGCAGGUGU-3' (SEQ ID NO: 1); and
Antisense: 5'-ACACCUGCGCCACCGGCUCgc-3' (SEQ ID NO: 431).

The sequence of small interfering RNA targeting the mouse *NFKBIZ* gene (si*NFKBIZ*-M) includes:
Sense: 5'-GCGUCAAUGUACCAGUAUU-3' (SEQ ID NO: 421); and
Antisense: 5'-AAUACUGGUACAUUGACGCCU-3' (SEQ ID NO: 422).

The sequences of the aptamers are as follows:
MUC1 aptamer: GCAGTTGATCCTTTGGATACCCTGG (SEQ ID NO: 423);
MUC16 aptamer: CTCACTATAGGGAGACAAGAATAAACGCTCAA (SEQ ID NO: 424);
α_{V}β₃ aptamer: GGGAGACAAGAATAAACGCTCAATTCAACGCT GTGAAGGGCTTATACGAGCGGATTACCCTTCGACAGGAGGCTCACAAAAGGC (SEQ ID NO: 425); and
CD44 aptamer: ACCGGGCGTACACCGTCGCGGCACATGTCTGA (SEQ ID NO: 426).

### 2. Verification of cellular uptake efficiency of siNFKBIZ-based nucleic acid pharmaceutical compositions with aptamer-targeting function

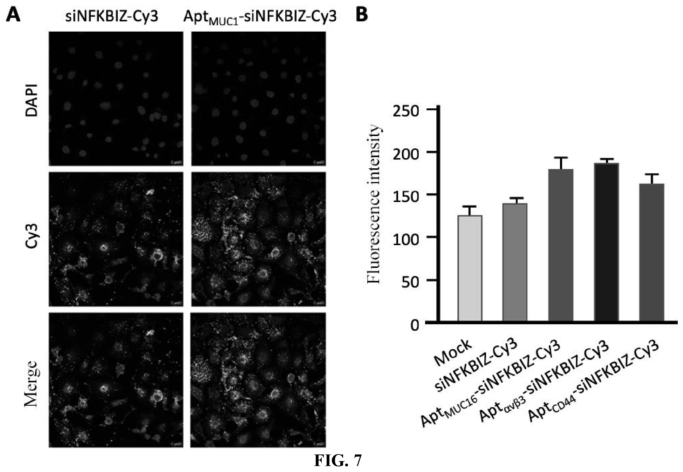

To verify the uptake efficiency of the Apt_{MUC1}-si*NFKBIZ* -based nucleic acid pharmaceutical composition at the cellular level, confocal laser scanning microscopy was performed. The specific method was as follows: Human corneal epithelial cells (HCECs) were seeded at a density of 2 × 10⁴ cells/well in a confocal dish and cultured overnight before drug addition. The Cy3-labeled Apt_{MUC1}-si*NFKBIZ* nucleic acid pharmaceutical composition (Apt_{MUC1}-si*NFKBIZ*-Cy3) and Cy3-labeled si*NFKBIZ* (si*NFKBIZ*-Cy3) were separately prepared using Opti-MEM medium at an equivalent Cy3 concentration of 0.5 µM, followed by incubation with HCEC cells. After 6 h, the cells were washed with PBS and fixed with 4% paraformaldehyde. Next, the cells were stained with DAPI, and finally observed using a laser scanning confocal microscope. The results are shown in A in FIG. 7. The results showed that the fluorescence intensity of cells in the Apt_{MUC1}-si*NFKBIZ* pharmaceutical composition incubation group was significantly higher than that in the free si*NFKBIZ* group. This indicates that aptamers targeting MUC1 can significantly enhance the intracellular delivery efficiency of si*NFKBIZ*, thereby enabling si*NFKBIZ* to exert a gene silencing effect.

Next, the targeted uptake efficiency of the Apt_{MUC16}-, Apt_{αvβ3}- and Apt_{CD44}-conjugated si*NFKBIZ* pharmaceutical compositions at the cellular level was verified by flow cytometry. The specific method was as follows: HCEC cells were seeded at 3.0 × 10⁵ cells/well in a 12-well plate and cultured overnight in DMEM. Cy3-labeled si*NFKBIZ* (si*NFKBIZ*-Cy3) and the Apt_{MUC16}-, Apt_{αvβ3}-, and Apt_{CD44}-conjugated pharmaceutical compositions were added to wells at an equivalent Cy3 concentration of 0.5 µM. The cells were incubated in Opti-MEM medium at 37°C for 6 h, and finally collected using trypsin for flow cytometric analysis (BD FACSCalibur, USA). The results are shown in panel B of FIG. 7. Apt_{MUC16}, Apt_{αvβ3} and Apt_{CD44} were all capable of significantly increasing the cellular uptake efficiency of siRNA, thereby enabling efficient delivery of siRNA into cells to exert a gene silencing effect.

### 3. Biological verification of siNFKBIZ-based nucleic acid pharmaceutical compositions having aptamer-targeting function

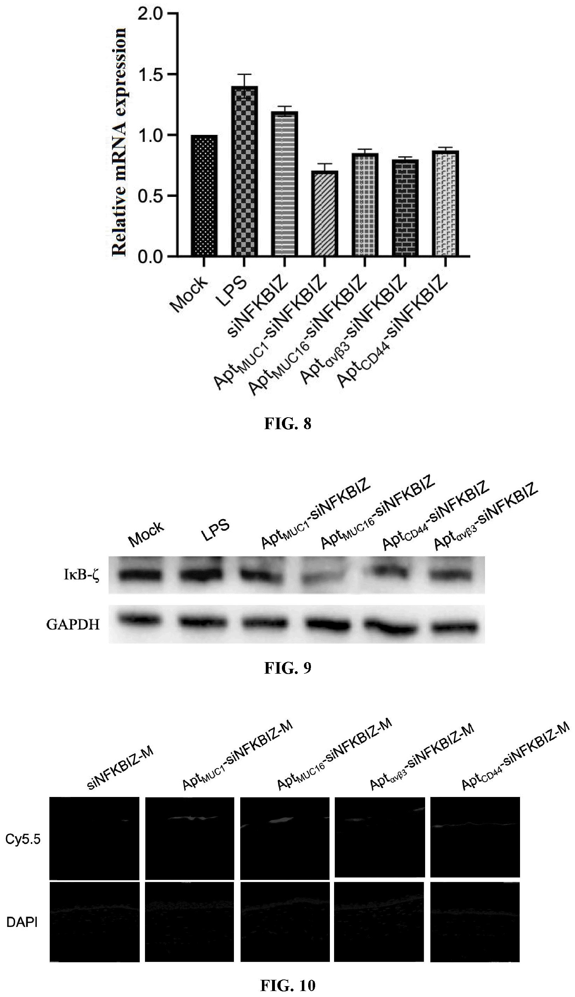

To verify the biological effects of the targeted delivery of si*NFKBIZ* by aptamers, the expression of the *NFKBIZ* gene and IκB-ζ protein was verified by qPCR and Western-blot experiments. HCEC cells were seeded at a density of 1 × 10⁵ cells/well in a 6-well plate and cultured for adherence overnight. Then, the cells were stimulated with 100 ng/mL LPS for 12 h. After removing the supernatant, 1 mL of Opti-MEM medium containing each of si*NFKBIZ* and the Apt_{MUC-1}-si*NFKBIZ*, Apt_{MUC16}-si*NFKBIZ*, Apt_{αvβ3}-si*NFKBIZ* and Apt_{CD44}-si*NFKBIZ* pharmaceutical compositions was added (at an equivalent si*NFKBIZ* concentration of 500 nM). After co-incubation at 37°C for 6 h, the medium was replaced with DMEM, and incubation was continued for 48 h. The cells were collected to extract total RNA for qPCR experiment (to evaluate the expression of *NFKBIZ* mRNA in HCEC cells) and total protein for Western-blot experiment. The results of the qPCR experiment are shown in FIG. 8. LPS stimulated the expression of the *NFKBIZ* gene in HCEC cells, which was upregulated by approximately 1.4 times; the si*NFKBIZ* nucleic acid drug alone could downregulate the expression of the *NFKBIZ* gene to around 1.2 times for the *NFKBIZ* gene. Apt_{MUC1}-si*NFKBIZ*, Apt_{MUC16}-si*NFKBIZ*, Apt_{αvβ3}-si*NFKBIZ* and Apt_{CD44}-si*NFKBIZ* drugs were all capable of significantly inhibiting *NFKBIZ* expression, achieving a gene inhibition rate of 40-50%. The Western-blot results are shown in FIG. 9. It could be seen from the results that Apt_{MUC-1}-si*NFKBIZ*, Apt_{MUC16}-si*NFKBIZ*, Apt_{αvβ3}-si*NFKBIZ* and Apt_{CD44}-si*NFKBIZ* pharmaceutical compositions all had a better inhibitory effect on the expression of IκB-ζ protein compared to the stimulation group. In summary, the aptamer-and-si*NFKBIZ*-based nucleic acid pharmaceutical compositions are capable of efficiently delivering si*NFKBIZ* nucleic acid drug, achieving downregulation of the *NFKBIZ* gene, and further inhibiting the expression of IκB-ζ protein, thereby inhibiting the inflammatory response.

### 4. Evaluation of ocular surface retention effect of Apt-siNFKBIZ-M nucleic acid pharmaceutical compositions

The retention effect of the pharmaceutical compositions for inhibiting *NFKBIZ* gene expression on the ocular surface was verified by animal experiments. All animal experiments in this example complied with the Statement of the Association for Research in Vision and Ophthalmology. All animal experiments were performed according to the guidelines established by the Ethics Committee of Shanghai Jiao Tong University. Thirty specific pathogen-free (SPF) C57BL/6 mice (60 eyes), aged 6-8 weeks, were selected and housed in an environmentally controlled room with adequate food and water. The mice were randomly divided into 6 groups, and benzalkonium chloride was instilled twice daily to establish a dry eye disease mouse model. After C57BL/6 mice were intraperitoneally anaesthetized, 5 µL of each of the following was instilled into the right eye: Apt_{MUC1}-si*NFKBIZ*-Cy5.5, Apt_{MUC16}-si*NFKBIZ*-Cy5.5, Apt_{αvβ3}-si*NFKBIZ*-Cy5.5 and Apt_{CD44}-si*NFKBIZ*-Cy5.5 nucleic acid pharmaceutical compositions, and free siRNA-Cy5.5 eye drops. After 30 min, the mice were anesthetized again, and the corneas were excised for frozen section imaging. The results are shown in FIG. 10. Compared to the si*NFKBIZ*-Cy5.5 treatment group, the Apt-siNFKBIZ nucleic acid pharmaceutical composition according to the present disclosure had stronger fluorescence on the ocular surface. This indicates the pharmaceutical compositions have better ocular surface retention and adhesion capacities *in vivo*, which reduces drug loss and improves bioavailability, suggesting that the Apt-siNFKBIZ nucleic acid pharmaceutical composition is indeed a promising drug for topical ocular application.

### 5. Therapeutic effect of Apt-siNFKBIZ nucleic acid pharmaceutical compositions in dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 40 specific pathogen-free (SPF) C57BL/6 mice (80 eyes), aged 6-8 weeks, were selected from Shanghai Slac Laboratory Animal Co., Ltd. and housed in an environmentally controlled room with adequate food and water. The mice were randomly divided into 8 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, cyclosporine CsA eye drops (Shenyang Xingqi Pharmaceutical Co., Ltd.), si*NFKBIZ*-M, and Apt_{MUC1}-si*NFKBIZ*-M, Apt_{MUC16}-si*NFKBIZ*-M, Apt_{αvβ3}-si*NFKBIZ*-M and Apt_{CD44}-si*NFKBIZ*-M pharmaceutical compositions (at an equivalent siRNA dose of 0.05 mg/kg) into both eyes twice daily (once in the morning and once in the evening) for 14 consecutive days. Tear secretion detection and corneal sodium fluorescein staining evaluation were performed on day 0, day 7 and day 14. The specific procedures were as follows: Basic tear secretion detection (Schirmer's I Test): On day 0, day 7 and day 14 of administration, the basal tear secretion test was performed using Zone-Quick phenol red threads. After the mice were intraperitoneally anaesthetized with 1.25% alfaxalone (0.2 mL/10 g), a 1 mm phenol red-soaked cotton tip was inserted into the lateral inferior conjunctival fornix of the mice and retained for 30 s. The color of the phenol-soaked cotton changed from yellow to red, indicating tear secretion, and the wetted length (mm) was read and recorded. Corneal sodium fluorescein staining score: 2 µL of 0.5% sodium fluorescein (w/v) was instilled onto the ocular surface, followed by manually closing the eyelids 3-4 times. Excess sodium fluorescein was then gently removed. Fluorescein-stained cornea images were captured under cobalt blue light using a slit lamp, and the corresponding scoring was performed by the same experienced ophthalmologist. The cornea was divided into 5 quadrants, with each quadrant scored on a 0-3 scale: Grade 0: absent; Grade 1: less than 30 punctate stains; Grade 2: more than 30 punctate stains but non-diffuse; and Grade 3: Severe diffuse staining or positive plaques. The sum of scores from all quadrants was taken as the final score. All affected eyes in each group were examined and analyzed.

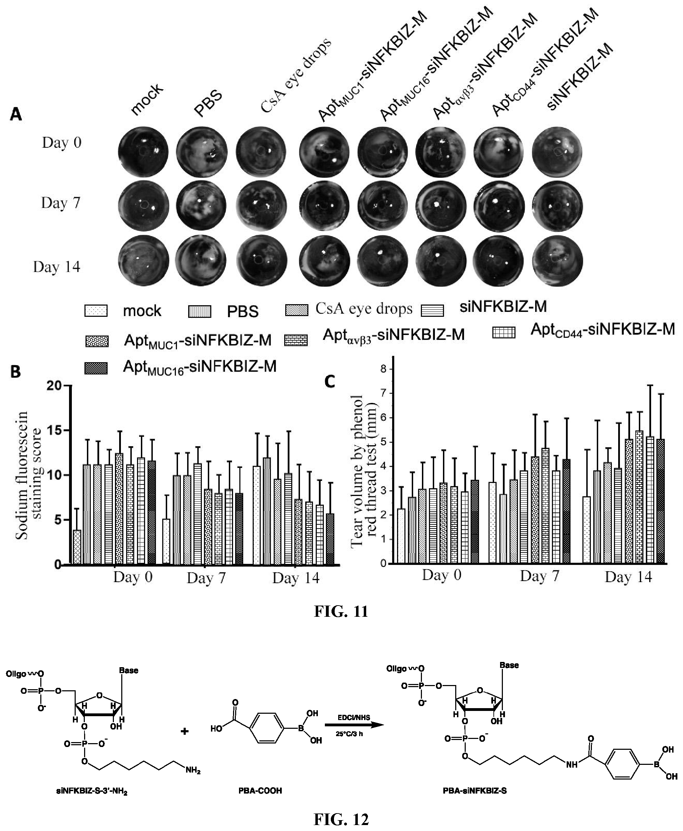

The experimental results are shown in FIG. 11. The treatment groups (CsA eyedrops, si*NFKBIZ*-M, and Apt_{MUC1}-si*NFKBIZ*-M, Apt_{MUC16}-si*NFKBIZ*-M, Apt_{αvβ3}-si*NFKBIZ*-M and Apt_{CD44}-si*NFKBIZ*-M pharmaceutical compositions) were all capable of effectively alleviating corneal damage. After one week of treatment, the corneal sodium fluorescein staining score of the mice in the cyclosporine eyedrops group decreased from 12 to around 8, and the results of the PRTT showed an increase from 3 mm to around 3.8 mm. The ocular score of the mice in the si*NFKBIZ*-M treatment group decreased from 12 to around 10, and the results of the PRTT showed an increase from 3 mm to around 3.5 mm. The corneal sodium fluorescein score of the mice in the Apt-si*NFKBIZ*-M treatment groups were all significantly reduced from about 12 to 6, indicating that Apt-si*NFKBIZ*-M was capable of significantly improving corneal damage. The results of the phenol red thread test (PRTT) showed an increase from about 3 mm to around 5 mm, indicating that Apt-si*NFKBIZ*-M was capable of significantly promoting tear secretion. It can be seen from the above experimental results that the aptamers can effectively promote the intracellular delivery of the si*NFKBIZ* nucleic acid drugs, thereby exerting the biological functions of si*NFKBIZ*, and achieving effective treatment of dry eye disease.

### Example 3

Targeted delivery of nucleic acid molecules by monophenylboronic acid

### 1. Synthesis and characterization of PBA-siNFKBIZ nucleic acid pharmaceutical composition

### 1.1 Synthesis of composition of 4-carboxyphenylboronic acid (PBA-COOH) and NFKBIZ small interfering RNA (PBA-siNFKBIZ)

The small interfering RNAs targeting the *NFKBIZ* gene used in this example were all purchased from Suzhou Biosyntech Co., Ltd. An amino (-NH₂) modification was introduced at the 3' end of the small interfering RNAs, enabling efficient conjugation with the carboxyl group on PBA-COOH via an amide bond reaction. The amino modification at the 3' end is represented as 3'-NH₂ in all the examples below. The modified structure is represented by formula 59: 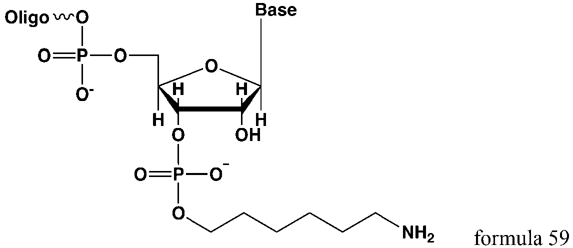 where **Oligo** 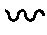 represents an attached nucleic acid molecule, and Base represents any type of base.

The sequences of the *siNFKBIZ* nucleic acid drugs used in this example are as follows:
The sequence of small interfering RNA targeting the human *NFKBIZ* gene *(siNFKBIZ)* includes:
Sense: 5'-AGAACAUUAUCAACAUUAA-3' (SEQ ID NO: 43); and
Antisense: 5'-UUAAUGUUGAUAAUGUUCUga-3' (SEQ ID NO: 432).

The sequence of small interfering RNA targeting the mouse *NFKBIZ* gene (si*NFKBIZ*-M) includes:
Sense: 5'-GCGUCAAUGUACCAGUAUU-3' (SEQ ID NO: 421); and
Antisense: 5'-AAUACUGGUACAUUGACGCCU-3' (SEQ ID NO: 422).

The synthetic route of PBA-siNFKBIZ is shown in FIG. 12.

The specific synthesis method was as follows: 0.22 mg of PBA-COOH (1300 nmol), 0.40 mg of EDCI (2600 nmol) and 0.30 mg of NHS (2600 nmol) were dissolved in 1.3 mL of DMSO. Then, 0.13 mL of the solution was taken, and 5 OD (26 nmol) of the sense strand (si*NFKBIZ*-S-3'-NH₂) of *siNFKBIZ* modified with an amino at the 3' end was added. The mixture was reacted with shaking at 25°C for 24 h. After 5 mL of water was added, EDCI/NHS and excess PBA-COOH in the reaction were removed by extraction with dichloromethane, and then the aqueous solution was concentrated to dryness to obtain a PBA-siNFKBIZ-S conjugate molecule. Then, PBA-si*NFKBIZ*-S and the antisense strand *(siNFKBIZ-A)* of *siNFKBIZ* were mixed at a molar ratio of 1 : 1 via quantitative operations. The mixture was incubated at 65°C for 10 minutes, and then annealed for 10 min to 25°C to obtain the PBA-si*NFKBIZ* pharmaceutical composition.

### 2. Verification of cellular uptake capacity of PBA-siNFKBIZ nucleic acid pharmaceutical composition

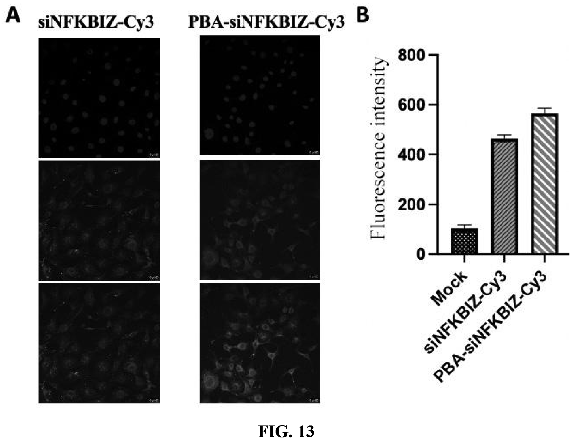

HCEC cells were seeded in a 24-well culture plate at a density of 4 × 10⁴ cells/well, with a clean coverslip placed in each well. After overnight culture, the cells were respectively cultured with PBA-si*NFKBIZ*-Cy3 and si*NFKBIZ*-Cy3 (at an equivalent Cy3 concentration of 0.5 µM) for 6 h. Subsequently, the medium was removed, and the cells were washed 3 times with PBS and fixed with 4% paraformaldehyde at room temperature for 15 min. Then, the cell nuclei were stained with DAPI for 15 min. After washing with PBS, the cells were observed using a laser scanning confocal microscope. The results are shown in panel A of FIG. 13. The green fluorescence intensity of cells in the PBA-si*NFKBIZ*-Cy3 group was higher than that in the si*NFKBIZ*-Cy3 treatment group.

HCEC cells were seeded at 4.0 × 10⁵ cells/well in a 12-well plate and cultured overnight in DMEM. PBA-si*NFKBIZ*-Cy3 was added to the cells at a final Cy3 concentration of 0.5 µM per well, and the mixture was incubated in Opti-MEM at 37°C for 6 h. si*NFKBIZ*-Cy3 was used as a control. The cells were collected by trypsinization and used for flow cytometry analysis. The results are shown in panel B of FIG. 13. The mean fluorescence intensity of the PBA-si*NFKBIZ*-Cy3 group was about 1.2 times that of the si*NFKBIZ* group. In summary, the above results indicate that the modification with PBA can enhance the uptake capacity of the si*NFKBIZ* nucleic acid drug and improve the intracellular efficiency.

### 3. Evaluation of gene regulatory capacity of PBA-siNFKBIZ nucleic acid pharmaceutical composition

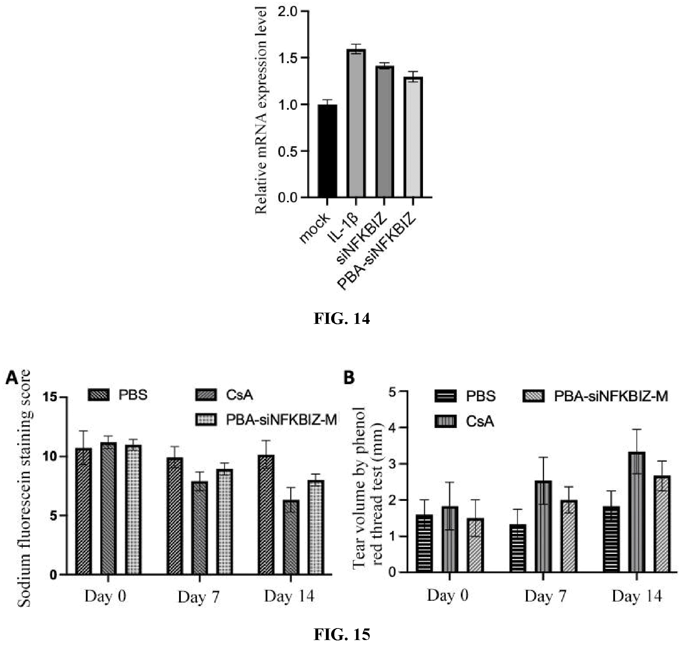

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing si*NFKBIZ* and the PBA-si*NFKBIZ* nucleic acid pharmaceutical composition for 6 h. After that, the media were replaced with complete DMEM medium, and the cells were further cultured for 48 h. Next, the cells were lysed and mRNA was extracted using RNAiso reagent, and the concentration of mRNA was measured by a Nanodrop spectrophotometer. cDNA was obtained using a reverse transcription kit, and further the qPCR experiment was performed. The results are shown in FIG. 14. After stimulation with IL-1β, the expression of the *NFKBIZ* gene in HCEC cells was significantly upregulated to around 1.6 times that of normal cells, while the free si*NFKBIZ* nucleic acid drug could downregulate the expression to around 1.4 times that of normal cells. In contrast, the PBA-si*NFKBIZ* nucleic acid pharmaceutical composition was capable of inhibiting the expression of the *NFKBIZ* gene, and the expression level after inhibition decreased to around 1.26 times that of the normal unstimulated group (Mock group). The above results indicate that the PBA-si*NFKBIZ* nucleic acid pharmaceutical composition can promote the cellular uptake of the si*NFKBIZ-*based nucleic acid drug to a certain extent, thereby exerting a regulatory effect on the *NFKBIZ* gene, and achieving a gene inhibitory effect.

### 4. Therapeutic effect of PBA-siNFKBIZ nucleic acid pharmaceutical composition on dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 15 specific pathogen-free (SPF) C57BL/6 mice (30 eyes), aged 6-8 weeks, were selected from Shanghai Slac Laboratory Animal Co., Ltd. and housed in an environmentally controlled room with adequate food and water. The mice were randomly divided into 3 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, cyclosporine CsA eye drops (Shenyang Xingqi Pharmaceutical Co., Ltd.), PBA-si*NFKBIZ*-M (at an equivalent siRNA dose of 0.05 mg/kg) into both eyes twice daily (once in the morning and once in the evening) for 14 consecutive days. Tear secretion detection and corneal sodium fluorescein staining assessment were performed on day 0, day 7 and day 14. The specific procedures were performed according to Example 2.

The results are shown in FIG. 15. The sodium fluorescein staining results showed that the scores of all treatment groups decreased. Among them, the score of the CsA eye drop treatment group significantly decreased on day 7, and decreased from the initial 11 to around 6 on day 14. The results of the PRTT showed an increase from 1.8 mm to around 3.3 mm. The sodium fluorescein staining score of the mice in the PBA-si*NFKBIZ*-M group decreased after 14 days of treatment (from 11 to about 8.0), and the tear secretion volume also increased (from 1.5 mm to 3 mm). Compared to the PBS treatment group, the CsA treatment group and the PBA-si*NFKBIZ*-M treatment group were both capable of improving the signs of dry eye disease.

### Example 4

### Targeted delivery of siNFKBIZ-based nucleic acid pharmaceutical compositions by biphenylboronic acid for the treatment of dry eye disease

### 1. Synthesis and characterization of 2PBA-siNFKBIZ nucleic acid pharmaceutical composition

### 1.1 Synthesis of 2PBA-MAL

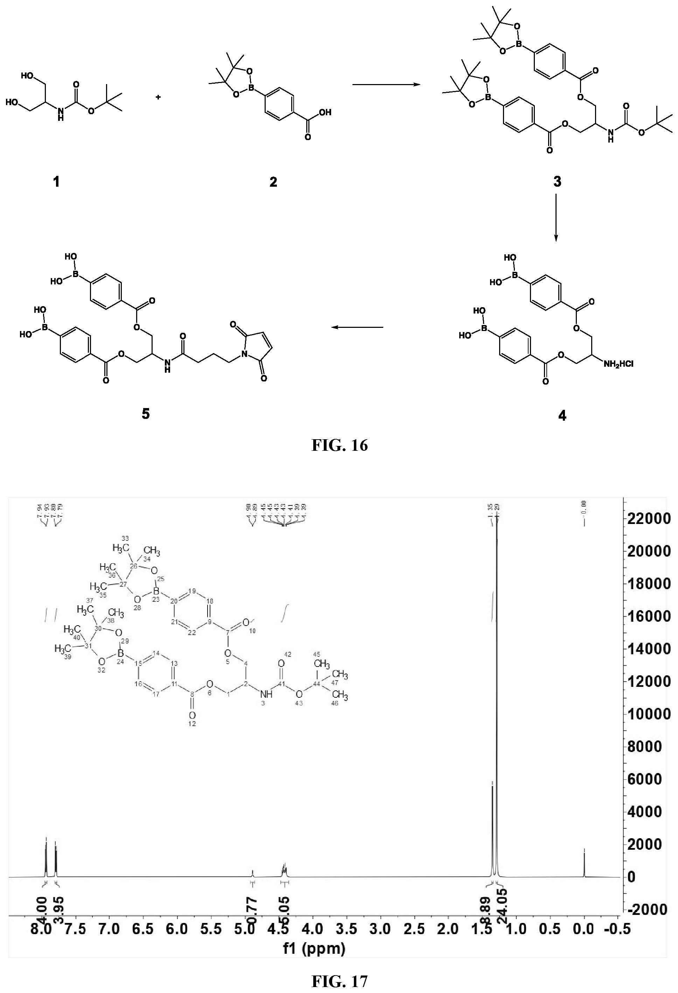

The synthetic route of 2PBA-MAL in this example is shown in FIG. 16.

### 1.2 Synthesis of compound 4

Compound 1 (1,000 mg) and compound 2 (1,440 mg) were added to a 100 mL flask, and 40 mL of dichloromethane was added with stirring until dissolved. EDCI (900 mg) and DMAP (30 mg) were added, and the reaction was performed at room temperature for 20 h under nitrogen atmosphere. After the reaction was completed, dichloromethane was removed by distillation under reduced pressure. 30 mL of water was added, and the mixture was extracted twice with 30 mL of ethyl acetate. The organic phase was collected, washed once with 30 mL of saturated sodium bicarbonate solution, and then once with 30 mL of saturated sodium chloride solution. The organic phase was dried over anhydrous sodium sulfate. After the solvent was concentrated to dryness by rotary evaporation, 10 mL of ethyl acetate was added and shaken. n-Hexane was added slowly with stirring until a large amount of white solid precipitated. The mixture was filtered under vacuum. The filter cake was washed twice with n-hexane to obtain compound 3 (859 mg), yield: about 84%.

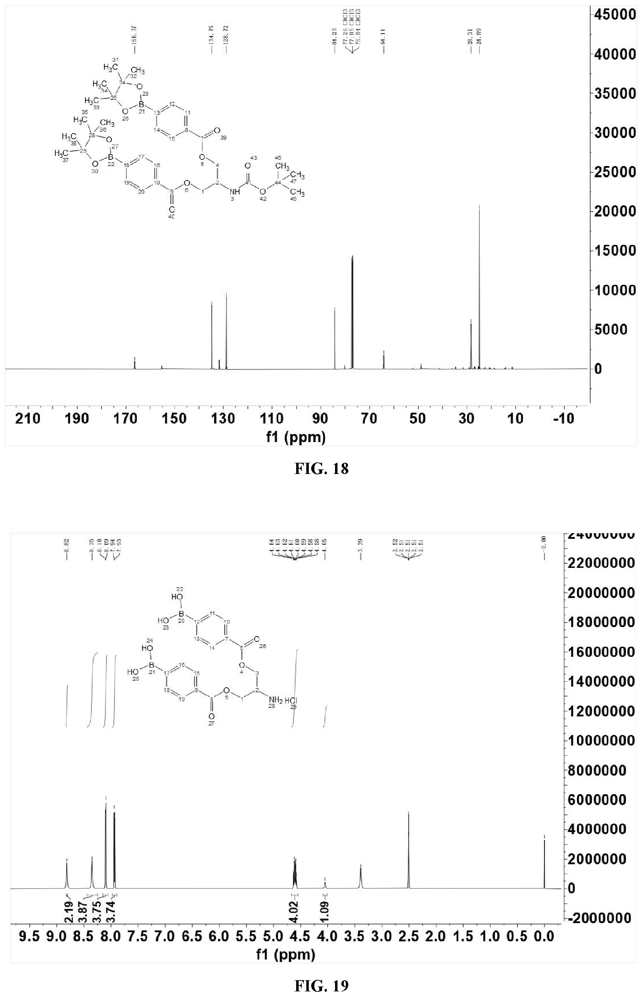

The ¹H NMR spectrum of compound 3 is shown in FIG. 17. The solvent used for the nuclear magnetic spectroscopy test was CDCl₃, and the assignment of the proton peaks was as follows: ¹H NMR (600 MHz, Chloroform-d) δ 7.94 (d, J = 7.9 Hz, 4H), 7.79 (d, J = 7.9 Hz, 4H), 4.89 (d, J = 6.8 Hz, 1H), 4.47 - 4.36 (m, 5H), 1.35 (s, 9H), 1.29 (s, 24H). The ¹H NMR spectrum of compound 3 is shown in FIG. 18. The solvent used for the nuclear magnetic spectroscopy test was CDCl₃, and the assignment of the characteristic carbons contained in the product was as follows: ¹³C NMR (151 MHz, Chloroform-d) δ 166.37, 134.75, 128.72, 84.23, 64.11, 28.31, 24.89.

### 1.3 Synthesis of compound 4

Compound 4 (859 mg) was added to a dry 100 mL round-bottom flask, and 20 mL of acetone was added with stirring until dissolved. Sodium periodate (1130 mg) and ammonium acetate (412 mg) were dissolved in 20 mL of water, and the solution was then added to the acetone solution. The reaction was performed at room temperature for 10 h under nitrogen atmosphere. After the reaction was completed, acetone was removed by distillation under reduced pressure. 20 mL of water was added, and the mixture was washed twice with 20 mL of ethyl acetate. The organic phase was washed once with 20 mL of saturated sodium chloride solution. Subsequently, the organic phase was collected, dried over anhydrous sodium sulfate, and then distilled under reduced pressure to obtain a colorless oily liquid. 20 mL of ethyl acetate and 2 mL of methanol were added for redissolution. 7 mL of a 4M hydrogen chloride-ethyl acetate solution was added in an ice bath, and the reaction was stirred at room temperature for 4 h until a large amount of white solid precipitated, which was filtered under vacuum. The filter cake was washed twice with ethyl acetate to give 424 mg of a white solid, i.e., compound 4, yield: about 76%.

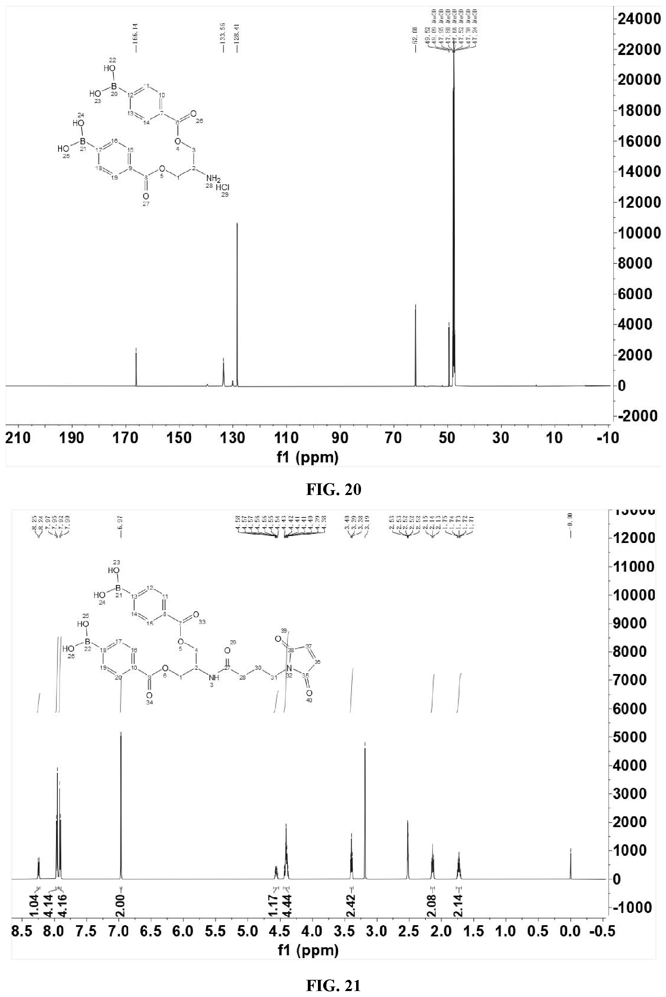

The ¹H NMR spectrum of compound 4 is shown in FIG. 19. The solvent used for the nuclear magnetic spectroscopy test was CDCl₃, and the assignment of the proton peaks was as follows: ¹H NMR (700 MHz, DMSO-d6) δ 8.82 (s, 2H), 8.35 (s, 4H), 8.10 (d, J = 8.3 Hz, 4H), 7.94 (d, J = 8.2 Hz, 4H), 4.61 (qd, J = 11.9, 5.1 Hz, 4H), 4.05 (s, 1H). The ¹³C NMR spectrum of compound 3 is shown in FIG. 20. The solvent used for the nuclear magnetic spectroscopy test was CDCl₃, and the assignment of the characteristic carbons contained in the product was as follows: 13C NMR (151 MHz, Methanol-d4) δ 166.14, 133.56, 128.41, 62.00, 49.52.

### 1.4 Synthesis of compound 5

Compound 4 (424 mg) and 4-maleimidobutyric acid (366 mg) were placed in a dry 100 mL round-bottom flask, and 30 mL of methanol was added with stirring until dissolved. Next, DMTMM (557 mg) and NMM (203 mg) were added, and the reaction was performed at room temperature for 2 h. After the reaction was completed, methanol was removed by distillation under reduced pressure. 20 mL of water was added, and the mixture was washed twice with 20 mL of ethyl acetate. The organic phase was washed once with 20 mL of saturated sodium chloride solution. The organic phase was collected, dried over anhydrous sodium sulfate, and then distilled under reduced pressure to obtain a colorless oily liquid. Purification was performed by reversed-phase column chromatography to obtain 481 mg of a white solid, i.e., compound 7, yield: about 87%.

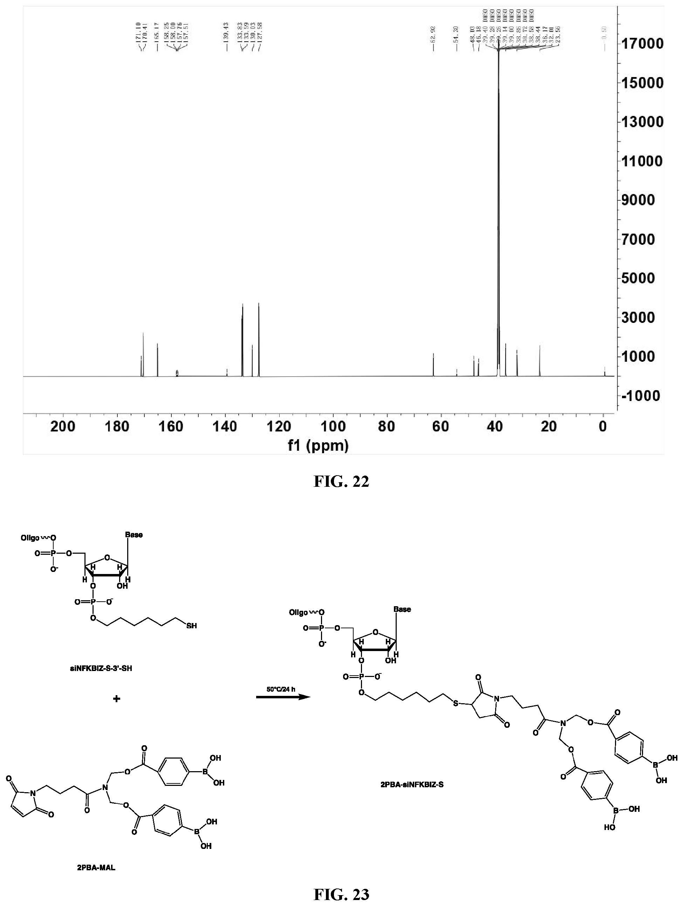

The ¹H NMR spectrum of compound 5 is shown in FIG. 21. The solvent used for the nuclear magnetic spectroscopy test was DMSO-d6, and the assignment of the proton peaks was as follows: ¹H NMR (600 MHz, DMSO-d6) δ 8.25 (d, J = 8.3 Hz, 1H), 7.96 (d, J = 8.3 Hz, 4H), 7.91 (d, J = 8.2 Hz, 4H), 6.97 (s, 2H), 4.61 - 4.52 (m, 1H), 4.45 - 4.36 (m, 4H), 3.39 (t, J = 7.0 Hz, 2H), 2.14 (t, J = 7.5 Hz, 2H), 1.73 (p, J = 7.2 Hz, 2H). The ¹³C NMR spectrum of compound 5 is shown in FIG. 22. The solvent used for the nuclear magnetic spectroscopy test was DMSO-d6, and the assignment of the characteristic carbons contained in the product was as follows: ¹³C NMR (151 MHz, DMSO-d6) δ 170.41, 165.17, 133.83, 133.59, 130.03, 127.58, 62.92, 38.44, 36.17, 32.01, 23.56.

### 1.5 Synthesis of 2PBA-siNFKBIZ nuecleic acid pharmaceutical composition

The small interfering RNAs targeting the *NFKBIZ* gene used in this example were all purchased from Suzhou Biosyntech Co., Ltd. A sulfhydryl (-SH) modification was introduced at the 3' end of the small interfering RNAs, enabling efficient conjugation with the maleimide group modified on 2PBA-MAL via a click chemical reaction. The sulfhydryl modification at the 3' end is represented as 3'-SH in all the examples below. The modified structure is represented by formula 60: 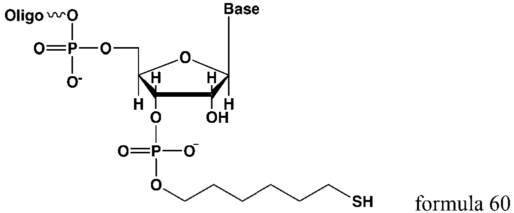 where **Oligo** 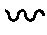 represents an attached nucleic acid molecule, and Base represents any type of base.

The sequences of the *siNFKBIZ* nucleic acid drugs used in this example are as follows:
The sequence of small interfering RNA targeting the human *NFKBIZ* gene *(siNFKBIZ)* includes:
Sense: 5'-GCCCGAUUCGUUGUCUGAU-3' (SEQ ID NO: 35); and
Antisense: 5'-AUCAGACAACGAAUCGGGCcc-3' (SEQ ID NO: 433).

The sequence of small interfering RNA targeting the mouse *NFKBIZ* gene (si*NFKBIZ*-M) includes:
Sense: 5'-GCGUCAAUGUACCAGUAUU-3' (SEQ ID NO: 421); and
Antisense: 5'-AAUACUGGUACAUUGACGCCU-3' (SEQ ID NO: 422).

The synthetic route of the 2PBA-si*NFKBIZ*-S conjugate is shown in FIG. 23.

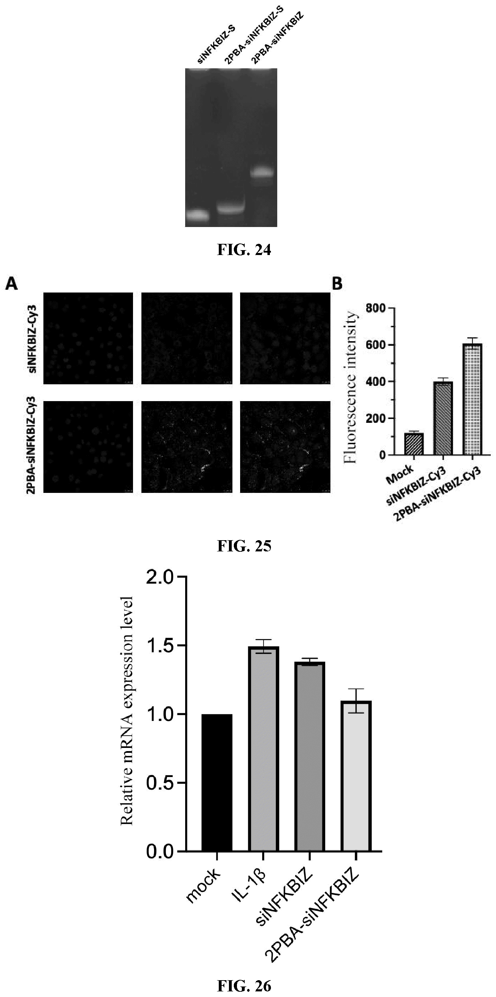

The synthesis method was as follows: 0.72 mg of 2PBA-MAL (1300 nmol) prepared in Example 4.1 was dissolved in 1.3 mL of DMSO. Then, 0.13 mL of the solution was taken, and 5 OD (26 nmol) of the sense strand (si*NFKBIZ*-S) of si*NFKBIZ*-3'-SH was added. The mixture was reacted with shaking at 50°C for 24 h. After 5 mL of water was added, excess 2PBA-MAL in the reaction was removed by extraction with ethyl acetate, and then the aqueous solution was concentrated to dryness to obtain a 2PBA-si*NFKBIZ*-S conjugate molecule. Then, 2PBA-si*NFKBIZ*-S and the antisense strand *(siNFKBIZ-A)* of *siNFKBIZ* were mixed at a molar ratio of 1 : 1 via quantitative operations. The mixture was incubated at 65°C for 10 min, and then annealed for 10 min to 25 °C to obtain the 2PBA-si*NFKBIZ* pharmaceutical composition. The successful conjugation of 2PBA to *siNFKBIZ* was verified by 20% non-denaturing PAGE, shown in FIG. 24.

### 2. Verification of cellular uptake capacity of 2PBA-siNFKBIZ nucleic acid pharmaceutical composition

HCEC cells were seeded in a 24-well culture plate at a density of 4 × 10⁴ cells/well, with a clean coverslip placed in each well. After overnight culture, the cells were respectively cultured with 2PBA-si*NFKBIZ*-Cy3 and siRNA-Cy3 (at an equivalent Cy3 concentration of 0.5 µM) for 6 h. Subsequently, the medium was removed, and the cells were washed 3 times with PBS and fixed with 4% paraformaldehyde at room temperature for 15 min. Then, the cells were stained with DAPI for 15 min. After washing with PBS, the cells were observed using a laser scanning confocal microscope. The results are shown in panel A of FIG. 25. The fluorescence intensity of cells in the 2PBA-si*NFKBIZ*-Cy3 group was higher than that in the si*NFKBIZ*-Cy3 group.

HCEC cells were seeded at 4.0 × 10⁵ cells/well in a 12-well plate and cultured overnight in DMEM. PBA-si*NFKBIZ*-Cy3 was added to the cells at a final Cy3 concentration of 0.5 µM per well, and the mixture was incubated in Opti-MEM at 37°C for 6 h. si*NFKBIZ*-Cy3 was used as a control. The cells were collected using trypsin and used for flow cytometry analysis. The results are shown in panel B of FIG. 25. The mean fluorescence intensity of the 2PBA-si*NFKBIZ*-Cy3 group was about 1.5 times that of the si*NFKBIZ*-Cy3 group. In summary, the above results indicate that modification with 2PBA can further enhance the uptake capacity of the si*NFKBIZ* drug and improve the intracellular delivery efficiency.

### 3. Evaluation of gene regulatory capacity of 2PBA-siNFKBIZ nucleic acid pharmaceutical composition

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing si*NFKBIZ* and the 2PBA-si*NFKBIZ* pharmaceutical composition for 6 h. After that, the media were replaced with complete medium. The cells were further cultured at 37°C for 48 h. Next, the mRNA was extracted and the concentration of the mRNA was measured. cDNA was obtained by reverse transcription and the qPCR experiment was performed. The results are shown in FIG. 26. After stimulation with IL-1β, the expression of the *NFKBIZ* gene in HCEC cells was significantly upregulated to around 1.5 times that of normal cells, while the si*NFKBIZ* drug alone could downregulate the expression to around 1.4 times that of normal cells, indicating no significant difference. In contrast, the 2PBA-si*NFKBIZ* pharmaceutical composition was capable of inhibiting the expression of the *NFKBIZ* gene to around 1.1 times that of normal cells. The above results indicate that the 2PBA-si*NFKBIZ* nucleic acid pharmaceutical composition can effectively promote the intracellular delivery of the si*NFKBIZ* nucleic acid drug, thereby exerting a regulatory effect on the *NFKBIZ* gene, and achieving a good gene inhibitory effect.

### 4. Therapeutic effect of 2PBA-siNFKBIZ nucleic acid pharmaceutical composition on dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 15 specific pathogen-free (SPF) C57BL/6 mice (30 eyes), aged 6-8 weeks, were selected from Shanghai Slac Laboratory Animal Co., Ltd. and housed in an environmentally controlled room with adequate food and water. The mice were randomly divided into 6 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, cyclosporine A (CsA) eye drops (Shenyang Xingqi Pharmaceutical Co., Ltd.), 2PBA-si*NFKBIZ* (at an equivalent siRNA dose of 0.05 mg/kg) into both eyes twice daily (once in the morning and once in the evening) for 14 consecutive days. Tear secretion detection and corneal sodium fluorescein staining assessment were performed on day 0, day 7 and day 14. The specific procedures were performed according to Example 2.

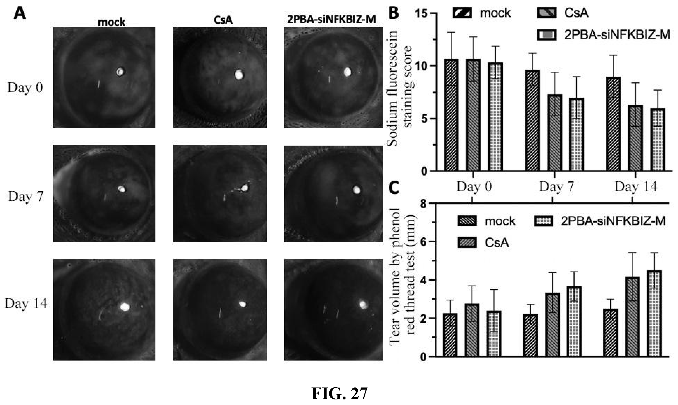

The results are shown in FIG. 27. The sodium fluorescein score of the mice in the CsA eye drops treatment group decreased from 10 to around 5, and the results of the PRTT showed an increase from 2 mm to about 4 mm. The sodium fluorescein ocular score of the mice in the 2PBA-si*NFKBIZ*-M treatment group decreased to around 4.8, and the results of the PRTT showed an increase from 2 mm to around 4.5 mm. It can be seen from the results of the animal experiments that 2PBA-si*NFKBIZ*-M has a good therapeutic effect for dry eye disease in mice. This can be attributed to two reasons. First, PBA can bind to ocular proteins, thereby promoting the intracellular delivery of si*NFKBIZ*-M. Second, after internalization into cells, si*NFKBIZ*-M can effectively exert a regulatory effect on the *NFKBIZ* gene, thereby inhibiting inflammation, and achieving a good therapeutic effect on dry eye disease.

### Example 5

### Tetraphenylboronic acid-mediated targeted delivery of siNFKBIZ-based nucleic acid pharmaceutical compositions for the treatment of dry eye disease

### 1. Synthesis and characterization of 4PBA-siNFKBIZ nucleic acid pharmaceutical composition

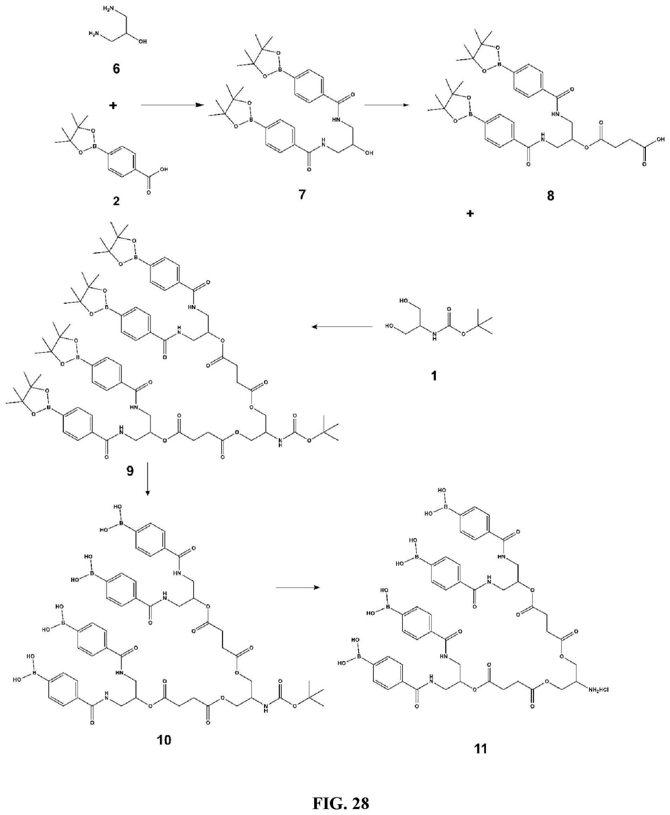

The synthetic route of 4PBA-NH₂ in this example is shown in FIG. 28.

### 1.1 Synthesis of compound 7

Compound 6 (100 mg) and compound 2 (826 mg) were added to a 100 mL flask, and 40 mL of methanol was added with stirring until dissolved. DMTMM (921 mg) and NMM (337 mg) were added, and the reaction was performed at room temperature for 20 h under nitrogen atmosphere. After the reaction was completed, methanol was removed by distillation under reduced pressure. 10 mL of ethyl acetate was added and shaken. The mixture was left to stand to precipitate a white solid, which was filtered under vacuum to obtain compound 7 (512 mg), yield: about 84%.

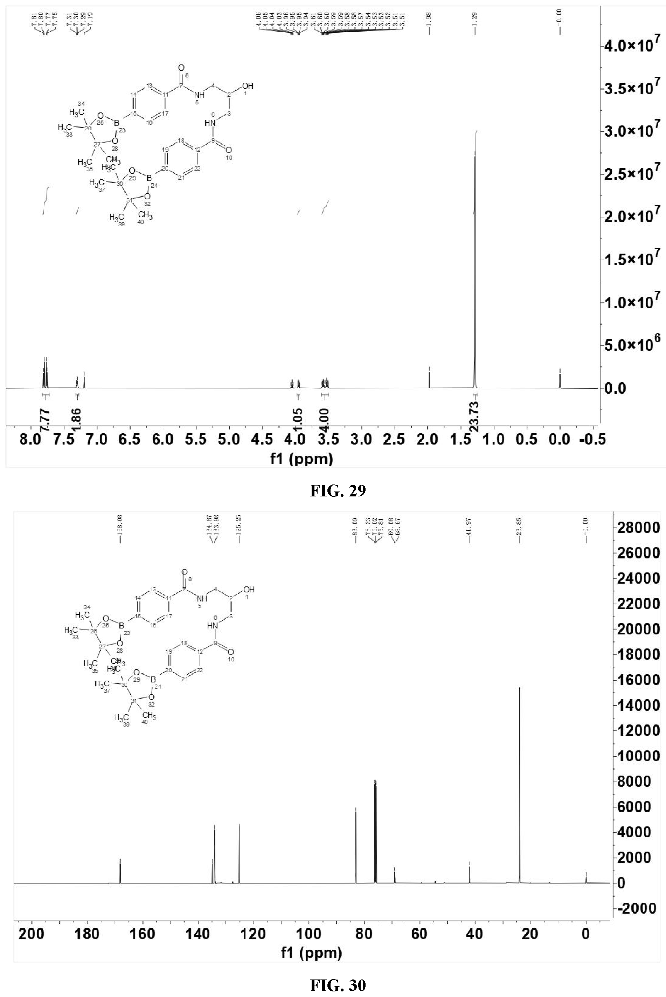

The ¹H NMR spectrum of compound 7 is shown in FIG. 29. The solvent used for the nuclear magnetic spectroscopy test was CDCl₃, and the assignment of the proton peaks was as follows: ¹H NMR (700 MHz, Chloroform-d) δ 7.82 - 7.73 (m, 8H), 7.30 (t, J = 6.4 Hz, 2H), 3.95 (q, J = 5.0 Hz, 1H), 3.61 - 3.50 (m, 4H), 1.29 (s, 24H). The ¹³C NMR spectrum of compound 7 is shown in FIG. 30. The solvent used for the nuclear magnetic spectroscopy test was CDCl₃, and the assignment of the characteristic carbons contained in the product was as follows: ¹³C NMR (151 MHz, Chloroform-d) δ 168.08, 134.87, 133.98, 131.74, 125.25, 83.09, 69.08, 41.97, 23.85.

### 1.2 Synthesis of compound 8

Compound 7 (512 mg) was placed in a 100 mL flask, and 30 mL of dichloromethane was added with stirring until dissolved. Then, DMAP (10 mg) and DIEA (240 mg) were added. Acetic anhydride (190 mg) was added in an ice bath, and the reaction was performed at room temperature for 20 h under nitrogen atmosphere. After the reaction was completed, dichloromethane was removed by distillation under reduced pressure. Then, 50 mL of water was added, and the mixture was washed twice with 20 mL of ethyl acetate. The organic phase was washed once with 20 mL of saturated sodium chloride solution. The organic phase was dried over anhydrous sodium sulfate, and then distilled under reduced pressure to obtain a colorless oily liquid. 10 mL of ether was added and shaken. The mixture was left to stand to precipitate a white solid, which was filtered under vacuum. The filter cake was dried to obtain 550 mg of a white solid, i.e., compound 8, yield: about 91%.

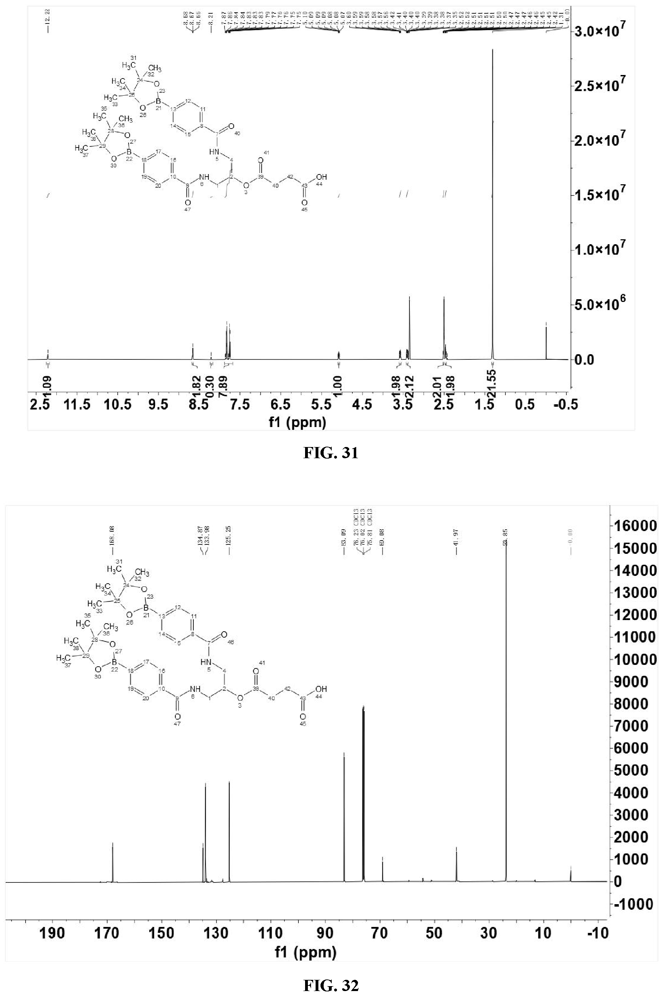

The ¹H NMR spectrum of compound 8 is shown in FIG. 31. The solvent used for the nuclear magnetic spectroscopy test was CDCl₃, and the assignment of the proton peaks was as follows: ¹ H NMR (700 MHz, DMSO-d6) δ 12.22 (s, 1H), 8.67 (t, J = 6.0 Hz, 2H), 7.88 - 7.69 (m, 8H), 5.09 (tt, J = 6.7, 4.9 Hz, 1H), 3.58 (dt, J = 13.9, 5.1 Hz, 2H), 3.43 - 3.38 (m, 2H), 2.51 (s, 2H), 2.48 - 2.44 (m, 2H), 1.31 (s, 22H). The ¹³C NMR spectrum of compound 8 is shown in FIG. 32. The solvent used for the nuclear magnetic spectroscopy test was CDCl₃, and the assignment of the characteristic carbons contained in the product was as follows: ¹³C NMR (151 MHz, Chloroform-d) δ 174.55, 170.86, 167.66, 134.79, 133.97, 131.68, 125.34, 83.13, 70.50, 38.17, 28.39, 28.20, 23.83.

### 1.3 Synthesis of compound 9

Compound 1 (100 mg) and compound 8 (1019 mg) were added to a 100 mL flask, and 40 mL of ethyl acetate was added with stirring until dissolved. EDCI (300 mg) and DMAP (10 mg) were added, and the reaction was performed at room temperature for 20 h under nitrogen atmosphere. After the reaction was completed, dichloromethane was removed by distillation under reduced pressure. 30 mL of water (pH = 3) was added, and the mixture was washed twice with 30 mL of ethyl acetate. The organic phase was collected, washed once with 30 mL of saturated sodium bicarbonate solution, and then once with 30 mL of saturated sodium chloride solution. The organic phase was collected, dried over anhydrous sodium sulfate, and then distilled under reduced pressure to obtain a white foamy solid, which was directly used for the subsequent reaction.

### 1.4 Synthesis of compound 10

Compound 9 (647 mg) was added to a dry 100 mL round-bottom flask, and 10 mL of acetone was added with stirring until dissolved. Sodium periodate (761 mg) and ammonium acetate (278 mg) were dissolved in 10 mL of water, and the solution was then added to the acetone solution. The reaction was performed at room temperature for 10 h under nitrogen atmosphere. After the reaction was completed, acetone was removed by distillation under reduced pressure. 20 mL of water was added, and the mixture was washed twice with 20 mL of ethyl acetate. The organic phase was washed once with 20 mL of saturated sodium chloride solution. The organic phase was collected, dried over anhydrous sodium sulfate, and then distilled under reduced pressure to obtain a colorless oily liquid, i.e., compound 10, which was directly used for the subsequent reaction.

### 1.5 Synthesis of compound 11

Compound 10 was added to a dry 100 mL round-bottom flask, and 30 mL of ethyl acetate was added with stirring until dissolved. 4M hydrogen chloride-ethyl acetate solution (10 mL) was added in an ice bath, and the reaction was performed for 3 h under nitrogen atmosphere. After the reaction was completed, the hydrogen chloride-ethyl acetate solution was removed by distillation under reduced pressure. Purification was performed by reversed-phase column chromatography to obtain 392 mg of a white solid, i.e., compound 11, yield: about 83%.

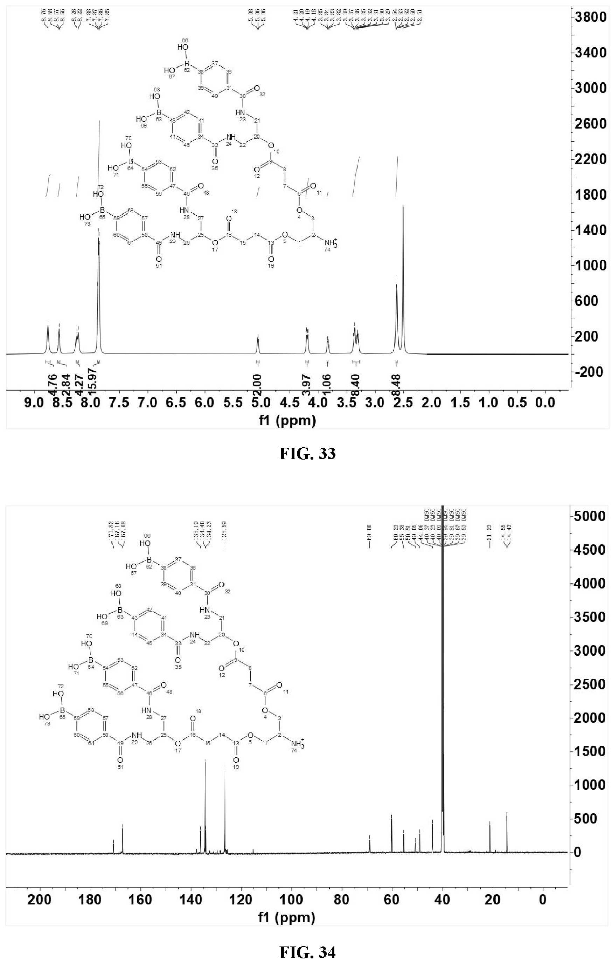

The ¹H NMR spectrum of compound 11 is shown in FIG. 33. The solvent used for the nuclear magnetic spectroscopy test was DMSO-d6, and the assignment of the proton peaks was as follows: ¹H NMR (600 MHz, DMSO-d6) δ 8.76 (s, 5H), 8.57 (d, J = 6.2 Hz, 3H), 8.24 (d, J = 20.8 Hz, 4H), 7.87 (q, J = 6.4, 5.1 Hz, 16H), 5.07 (t, J = 6.0 Hz, 2H), 4.22 - 4.17 (m, 4H), 3.84 (d, J = 5.0 Hz, 1H), 3.40 - 3.28 (m, 8H), 2.64 - 2.60 (m, 8H). The ¹³C NMR spectrum of compound 11 is shown in FIG. 34. The solvent used for the nuclear magnetic spectroscopy test was DMSO-d6, and the assignment of the characteristic carbons contained in the product was as follows: ¹³C NMR (151 MHz, DMSO-d6) δ 170.82, 167.16, 167.08, 136.19, 134.40, 134.23, 126.59, 69.00, 60.23, 55.38, 50.81, 49.05, 44.06, 21.23, 14.55.

### 1.5 Synthesis of 4PBA-siNFKBIZ nucleic acid pharmaceutical composition

The small interfering RNAs targeting the *NFKBIZ* gene used in this example were all purchased from Suzhou Biosyntech Co., Ltd. An aldehyde group (5'-CHO-) modification was introduced at the 5' end of the small interfering RNAs, enabling efficient conjugation with the carboxyl groups modified on 4PBA-NH₂ via electrophilic substitution reaction. The aldehyde group modification at the 5' end is represented as 5'-CHO- in all the examples below. The modified structure is represented by formula 61: 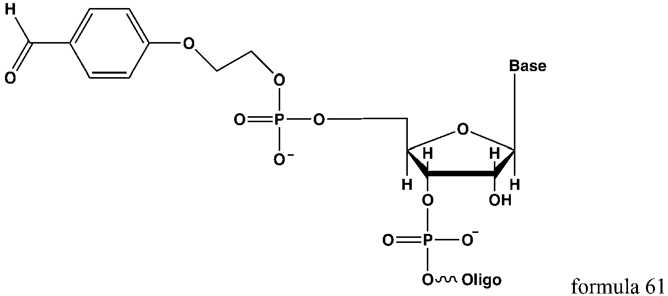 where **Oligo** 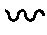 represents an attached nucleic acid molecule, and Base represents any type of base.

The sequences of the si*NFKBIZ* nucleic acid drugs used in this example are as follows:
The sequence of small interfering RNA targeting the human *NFKBIZ* gene (si*NFKBIZ*) includes:
Sense: 5'-UUAUCAACAUUAAGAAUGA-3' (SEQ ID NO: 44); and
Antisense: 5'-UCAUUCUUAAUGUUGAUAA-3' (SEQ ID NO: 254).

To further increase the stability of RNA, stabilizing modifications were performed in this example. The specific sequences are as follows:
Sense: 5'-UmUmAmUmCfAmAfCfAfUmUmAmAmGmAmAmUmsGmsAm-3' (SEQ ID NO: 44); and
Antisense: 5'-UmsCfsAmUmUmCfUmUfAfAmUmGmUmUfGmAfUmsAmsAm-3' (SEQ ID NO: 254).

The sequence of small interfering RNA targeting the mouse *NFKBIZ* gene (si*NFKBIZ*-M) that has a stabilizing modification includes:
Sense: 5'-GmCmGmUmCfAmAfUfGfUmAmCmCmAmGmUmAmsUmsUm-3' (SEQ ID NO: 421); and
Antisense: 5'-AmsAmsUmAmCmUfGmGfUfAmCmAmUmUfGmAfCmsGmsCm-3' (SEQ ID NO: 422),
where m represents a 2'-O-methylation modification of the ribose backbone, s represents a phosphorothioate modification of the backbone, and f represents a 2'-fluoro modification of the ribose backbone.

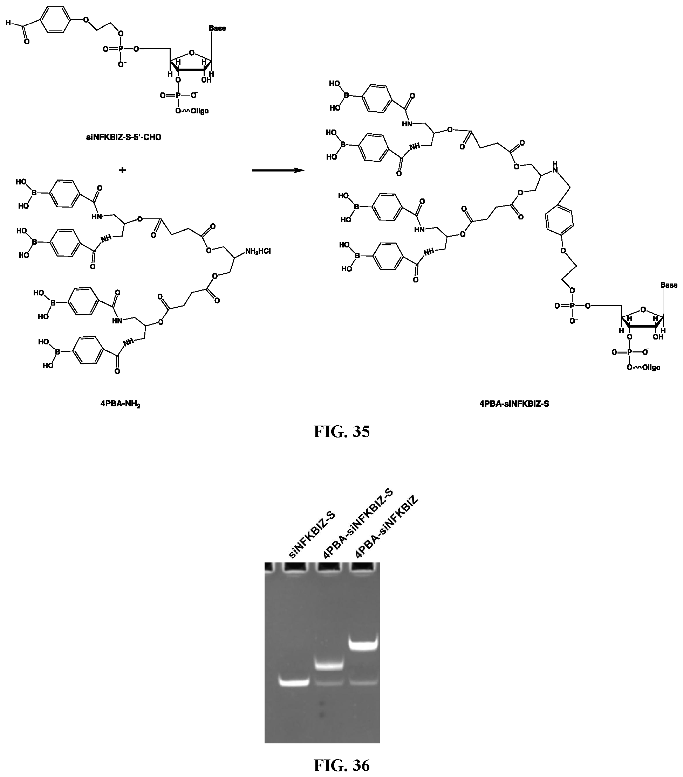

The synthetic route of 4PBA-si*NFKBIZ*-S is shown in FIG. 35.

The specific synthesis method was as follows: 0.15 mg of 4PBA-NH₂ (1300 nmol) and 0.14 mg of pic-BH₃ (1300 nmol) were dissolved in 0.13 mL of DMSO. Then, 0.13 mL of the solution was taken, and 5 OD (26 nmol) of the sense strand (si*NFKBIZ*-S) of 5'-CHO-si*NFKBIZ* and 5 µL of acetic acid were added. The mixture was reacted with shaking at room temperature for 2 h. After the reaction was completed, 1 mL of absolute ethanol and 13 µL of 10× PBS were added, and the mixture was left to stand at -20°C overnight. After centrifugation at 12,000 rpm for 15 min, the supernatant was discarded and the precipitate was reconstituted with water to obtain a 4PBA-si*NFKBIZ*-S conjugate molecule. Then, PBA-si*NFKBIZ*-S and the antisense strand (si*NFKBIZ-*A) of si*NFKBIZ* were mixed at a molar ratio of 1 : 1 via quantitative operations. The mixture was incubated at 65°C for 10 min, and then annealed for 10 min to 25°C to obtain the 4PBA-si*NFKBIZ* pharmaceutical composition. The successful conjugation of 4PBA to si*NFKBIZ* was verified by 20% non-denaturing PAGE, shown in FIG. 36. It could be seen from the figure that the grafting efficiency of 4PBA onto DBCO-si*NFKBIZ* was relatively high, with a yield of over 90%.

### 2. Verification of cellular uptake capacity of 4PBA-siNFKBIZ pharmaceutical composition

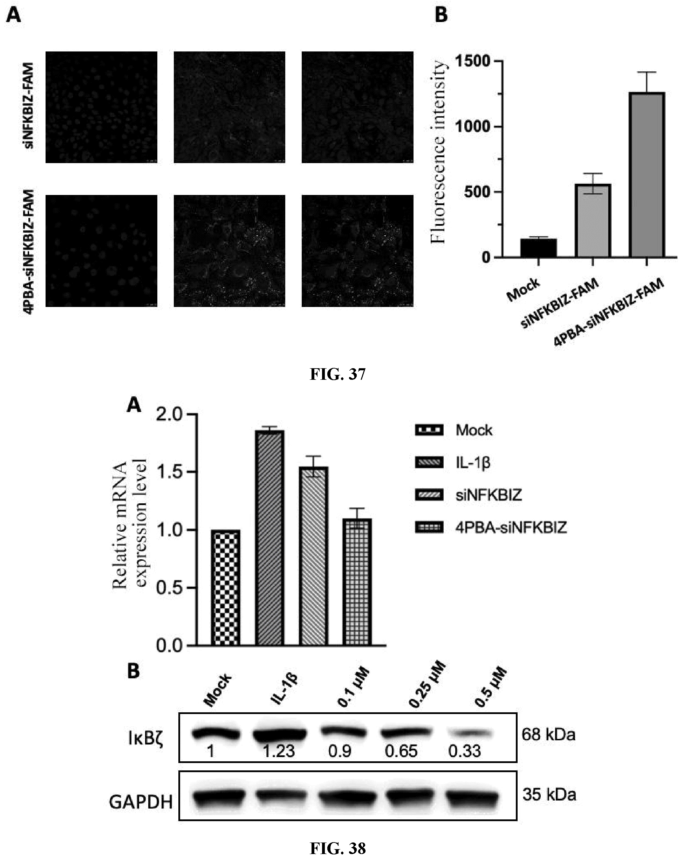

HCEC cells were seeded in a 12-well culture plate at a density of 4 × 10⁴ cells/well, with a clean coverslip placed in each well. After overnight culture, the cells were respectively cultured with 4PBA-si*NFKBIZ*-FAM and siRNA-FAM (at an equivalent FAM concentration of 0.5 µM) for 6 h. Subsequently, the medium was removed, and the cells were washed 3 times with PBS and fixed with 4% paraformaldehyde at room temperature for 15 min. Then, the cell nuclei were stained with DAPI for 15 min. After washing with PBS, the cells were observed using a laser scanning confocal microscope. The results are shown in panel A of FIG. 37. The green fluorescence intensity of cells in the 4PBA-si*NFKBIZ*-FAM group was significantly higher than that in the si*NFKBIZ*-FAM group.

HCEC cells were seeded at 4.0 × 10⁵ cells/well in a 12-well plate and cultured overnight in DMEM. 4PBA-si*NFKBIZ*-FAM was added to the cells at a final FAM concentration of 0.5 µM per well, and the mixture was incubated in Opti-MEM at 37°C for 6 h. si*NFKBIZ*-FAM was used as a control. The cells were collected using trypsin and used for flow cytometry analysis. The results are shown in panel B of FIG. 37. The mean fluorescence intensity of the 4PBA-si*NFKBIZ*-FAM group was about 2.3 times that of the si*NFKBIZ*-FAM group. In summary, the above results indicate that the modification with 4PBA can further enhance the uptake capacity of the si*NFKBIZ* oligonucleotide drug and improve the intracellular delivery efficiency.

### 3. Evaluation of gene regulatory capacity of 4PBA-siNFKBIZ pharmaceutical composition

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing si*NFKBIZ* and the 4PBA-si*NFKBIZ* pharmaceutical composition for 6 h. After that, the media were replaced with complete medium. The cells were further cultured at 37°C for 48 h. Next, the mRNA was extracted and the concentration of the mRNA was measured. cDNA was obtained by reverse transcription and the qPCR experiment was performed. The results are shown in panel A of FIG. 38. After stimulation with IL-1β, the expression of the *NFKBIZ* gene in HCEC cells was significantly upregulated to around 1.8 times that of normal cells, while the si*NFKBIZ* nucleic acid drug alone could downregulate the expression to around 1.5 times that of normal cells. In contrast, the 4PBA-si*NFKBIZ* pharmaceutical composition was capable of inhibiting the expression of the *NFKBIZ* gene to around 1.0 times that of normal cells.

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing the 4PBA-si*NFKBIZ* pharmaceutical composition at different concentrations for 6 h. After that, the media were replaced with complete medium, and the cells were further cultured for 48 h. Next, total protein was extracted from the cells using RIPA buffer containing a protease inhibitor cocktail, the protein concentration was determined, and the Western-blot experiment was performed. The results are shown in panel B of FIG. 38. The above results indicate that the 4PBA-si*NFKBIZ* pharmaceutical composition can effectively promote the intracellular delivery of the si*NFKBIZ* nucleic acid drug, thereby inhibiting the expression of the *NFKBIZ* gene, and effectively inhibiting the expression of IκBζ protein.

### 4. Therapeutic effect of 4PBA-siNFKBIZ pharmaceutical composition on dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 15 specific pathogen-free (SPF) C57BL/6 mice (30 eyes), aged 6-8 weeks, were selected from Shanghai Slac Laboratory Animal Co., Ltd. and housed in an environmentally controlled room with adequate food and water. The mice were randomly divided into 3 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, cyclosporine CsA eye drops (Shenyang Xingqi Pharmaceutical Co., Ltd.), 4PBA-si*NFKBIZ*-M (at an equivalent siRNA dose of 0.05 mg/kg) into both eyes twice daily (once in the morning and once in the evening) for 14 consecutive days. Tear secretion detection and corneal sodium fluorescein staining assessment were performed on day 0, day 7 and day 14. The specific procedures were performed according to Example 2.

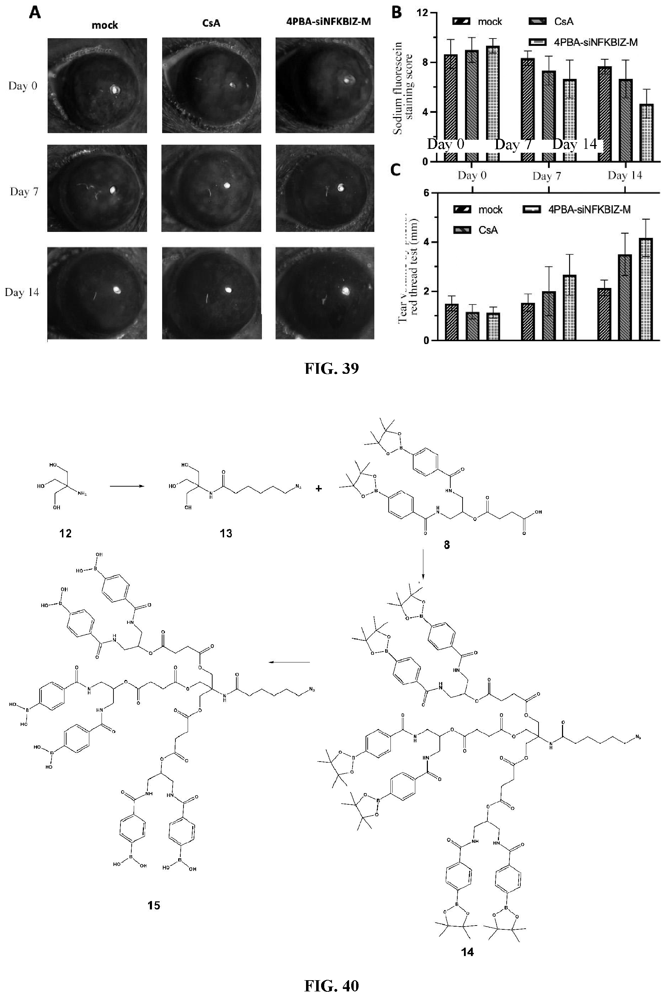

The results are shown in FIG. 39. The sodium fluorescein staining results that, after 14 days of treatment, compared to the PBS group, the treatment groups showed less ocular staining and a significant improvement in the degree of corneal damage. Among them, the 4PBA-si*NFKBIZ*-M treatment group showed a significant reduction in ocular damage, with the score decreasing from the initial 9 to around 4, while the CsA group had a score reduced to around 5. Tear secretion results showed that after treatment with the 4PBA-si*NFKBIZ*-M pharmaceutical composition, the tear secretion volume increased from approximately 1.5 mm to around 4 mm. In summary, the 4PBA-si*NFKBIZ*-M pharmaceutical composition can increase the retention of the si*NFKBIZ*-M drug on the ocular surface, enhance drug bioavailability, and promote the cellular uptake of the si*NFKBIZ* nucleic acid drug, which inhibits the expression of the *NFKBIZ* gene, blocks the vicious cycle of inflammation, and ameliorates ocular surface corneal damage, thereby achieving effective treatment of dry eye disease.

### Example 6

### Hexaphenylboronic acid-mediated targeted delivery of siNFKBIZ nucleic acid pharmaceutical compositions for the treatment of dry eye disease

### 1. Synthesis and characterization of 6PBA-siNFKBIZ nucleic acid pharmaceutical composition

### The synthetic route of 6PBA-N₃ in this example is shown in FIG. 40.

### 1.1 Synthesis of compound 13

Compound 12 (200 mg) and azidohexanoic acid (467 mg) were added to a 100 mL flask, and 20 mL of methanol was added with stirring until dissolved. DMTMM (688 mg) and NMM (248 mg) were added, and the reaction was performed at room temperature for 20 h under nitrogen atmosphere. After the reaction was completed, methanol was removed by distillation under reduced pressure. The residue was separated and purified by silica gel column chromatography using an eluent of ethyl acetate: methanol = 30: 1 to finally obtain 356 mg of a white solid, i.e., compound 13, yield: about 83%.

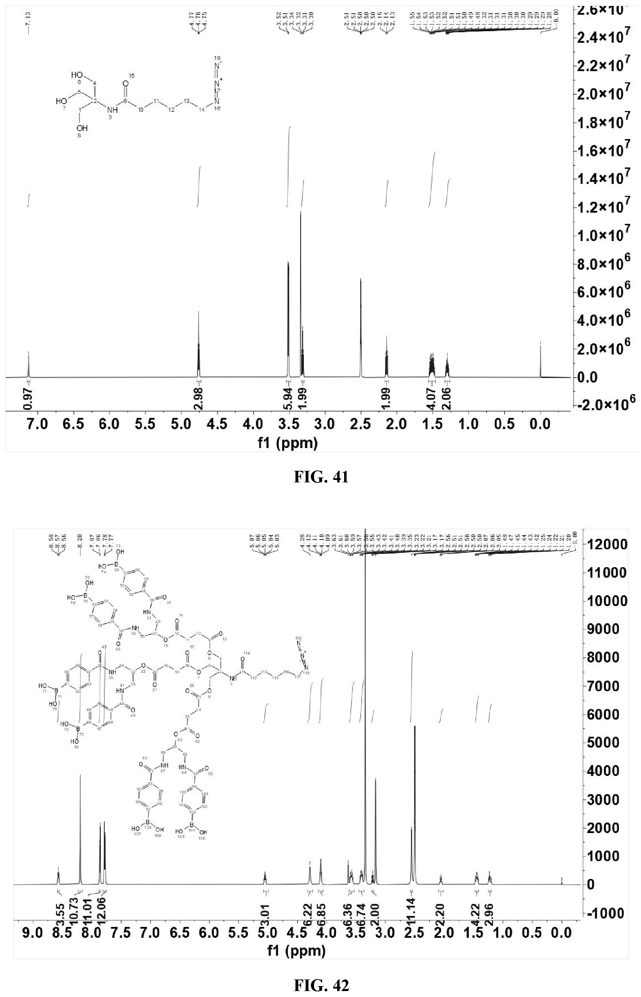

The ¹H NMR spectrum of compound 13 is shown in FIG. 41. The solvent used for the nuclear magnetic spectroscopy test was CDCl₃, and the assignment of the proton peaks was as follows: ¹H NMR (700 MHz, DMSO-d6) δ 7.13 (s, 1H), 4.76 (t, J = 5.8 Hz, 3H), 3.51 (d, J = 5.7 Hz, 6H), 3.31 (t, J = 6.9 Hz, 2H), 2.14 (t, J = 7.4 Hz, 2H), 1.51 (ddt, J = 21.1, 15.1, 7.3 Hz, 4H), 1.33 - 1.26 (m, 2H).

### 1.2 Synthesis of compound 14

Compound 13 (356mg) and compound 8 (3560 mg) were added to a 250 mL flask, and 100 mL of dichloromethane was added with stirring until dissolved. EDCI (849 mg) and DMAP (100 mg) were added, and the reaction was performed at room temperature for 20 h under nitrogen atmosphere. After the reaction was completed, dichloromethane was removed by distillation under reduced pressure. 100 mL of ethyl acetate was added, and the mixture was washed twice with 100 mL of water (pH = 3), twice with 100 mL of saturated sodium bicarbonate solution, and then once with 100 mL of saturated sodium chloride solution. The organic phase was collected, dried over anhydrous sodium sulfate, and then distilled under reduced pressure to obtain a white foamy solid, which was directly used for the subsequent reaction.

### 6.1.3 Synthesis of compound 15

Compound 14 (1000 mg) was added to a dry 100 mL round-bottom flask, and 30 mL of acetone was added with stirring until dissolved. Sodium periodate (2379 mg) and ammonium carbonate (857 mg) were dissolved in 30 mL of water, and the solution was then added to the acetone solution. The reaction was performed at room temperature for 10 h under nitrogen atmosphere. After the reaction was completed, acetone was removed by distillation under reduced pressure. 100 mL of ethyl acetate was added, and the mixture was washed twice with 100 mL of water, and then once with 100 mL of saturated sodium chloride solution. The organic phase was collected, dried over anhydrous sodium sulfate, and then distilled under reduced pressure to obtain a white solid, which was dissolved in 10 mL of ethyl acetate. 10 mL of dichloromethane followed by 20 mL of petroleum ether were slowly added with stirring, and the mixture was filtered under vacuum to obtain a white solid, which was washed with 20 mL of petroleum ether and then dried to obtain 702 mg of a white solid, i.e., compound 15, yield: about 91%.

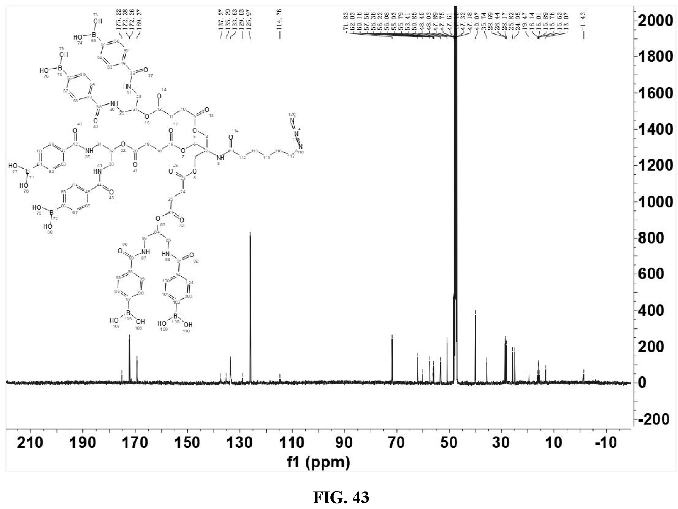

The ¹H NMR spectrum of compound 15 is shown in FIG. 42. The solvent used for the nuclear magnetic spectroscopy test was Methanol-d4, and the assignment of the proton peaks was as follows: ¹H NMR (600 MHz, Methanol-d4) δ 7.78 (t, J = 15.6 Hz, 24H), 5.22 - 5.18 (m, 3H), 4.32 (s, 6H), 3.71 (dd, J = 14.2, 4.8 Hz, 6H), 3.55 (dd, J = 14.3, 6.5 Hz, 6H), 3.19 (t, J = 6.8 Hz, 2H), 2.64 - 2.56 (m, 13H), 2.15 (t, J = 7.4 Hz, 2H), 1.51 (dq, J = 22.3, 7.4 Hz, 4H), 1.29 (ddt, J = 8.3, 5.9, 2.5 Hz, 2H). The ¹³C NMR spectrum of compound 15 is shown in FIG. 43. The solvent used for the nuclear magnetic spectroscopy test was Methanol-*d4*, and the assignment of the characteristic carbons contained in the product was as follows: ¹³C NMR (151 MHz, Methanol-*d4*) δ 175.22, 172.27 (d, J = 3.1 Hz), 169.37, 137.37, 135.29, 133.63, 125.97, 71.83, 62.03, 60.16, 57.56, 53.41, 50.85, 48.45, 40.07, 35.74, 25.82, 24.95, 19.47, 13.07.

### 1.4 Synthesis of 6PBA-siNFKBIZ nucleic acid pharmaceutical composition

The small interfering RNAs targeting the *NFKBIZ* gene used in this example were all purchased from Suzhou Biosyntech Co., Ltd. A dibenzocyclooctyne (DBCO) modification was introduced at the 3' end of the small interfering RNAs, enabling efficient conjugation with the azido group modified on 6PBA-N₃ via a click chemical reaction. The DBCO group modification at the 3' end is represented as 3'-DBCO in all the examples below. The modified structure is represented by formula 62: 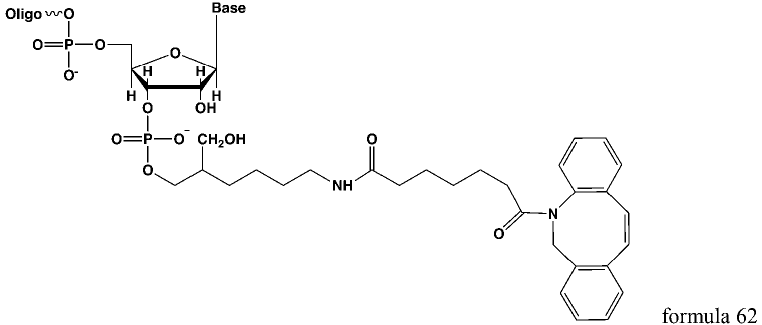 where **Oligo** 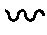 represents an attached nucleic acid molecule. Base represents any type of base.

The sequences of the *siNFKBIZ* nucleic acid drugs used in this example are as follows:
The sequence of small interfering RNA targeting the human *NFKBIZ* gene (si*NFKBIZ*) includes:
Sense: 5'-AGUUGUUUCUAUGAAACAA-3' (SEQ ID NO: 101); and
Antisense: 5'-UUGUUUCAUAGAAACAACUua-3' (SEQ ID NO: 434);
Further, in this example, the sequences used were subjected to stabilizing modifications, specifically as follows:
   Sense: 5'-AmsGmsUmUmGfUmUfUfCfUmAmUmGmAmAmAmCmAmAm-3' (SEQ ID NO: 101); and
   Antisense: 5'-UmsUfsGmUmUmUfCmAfUfAmGmAmAmAfCmAfAmCmUmsUmsAm-3' (SEQ ID NO: 434),
   where m represents a 2'-O-methylation modification of the ribose backbone, s represents a phosphorothioate modification of the backbone, and f represents a 2'-fluoro modification of the ribose backbone.

The sequence of small interfering RNA targeting the mouse *NFKBIZ* gene (si*NFKBIZ*-M) includes:
Sense: 5'-GCGUCAAUGUACCAGUAUU-3' (SEQ ID NO: 421); and
Antisense: 5'-AAUACUGGUACAUUGACGCCU-3' (SEQ ID NO: 422).

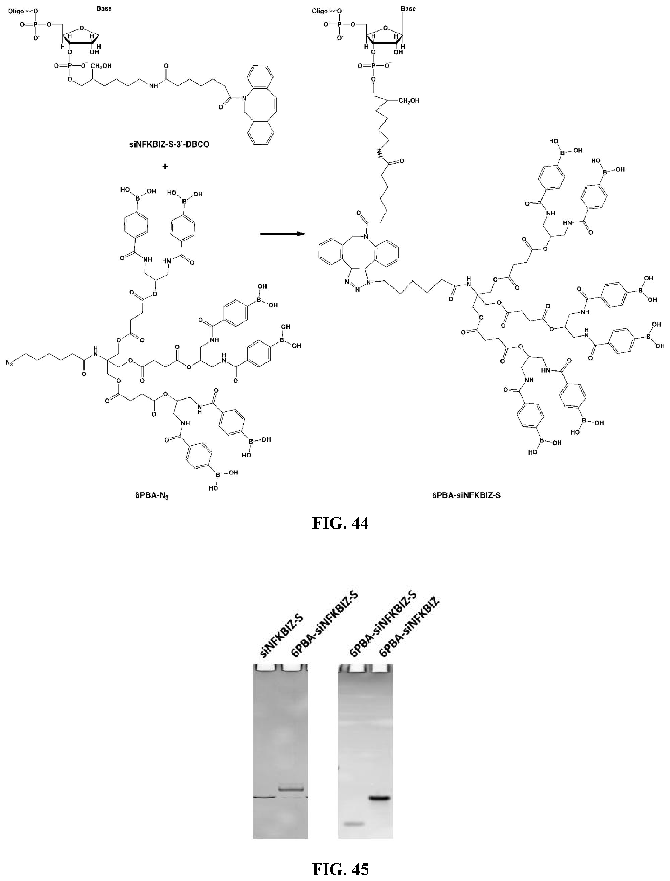

The synthetic route of 6PBA-si*NFKBIZ*-S is shown in FIG. 44.

The specific synthesis method was as follows: 0.15 mg of 6PBA-N₃ (130 nmol) was dissolved in 0.13 mL of DMSO. Then, 5 OD (26 nmol) of the sense strand (si*NFKBIZ*-S) of DBCO-siNFKBIZ was added. The mixture was reacted with shaking at 50°C for 24 h. After 5 mL of water was added, excess 6PBA-N₃ in the reaction was removed by extraction with ethyl acetate, and then the aqueous solution was concentrated to dryness to obtain a PBA-si*NFKBIZ*-S conjugate molecule. Then, 6PBA-si*NFKBIZ*-S and the antisense strand (si*NFKBIZ-*A) of *siNFKBIZ* were mixed at a molar ratio of 1: 1 via quantitative operations. The mixture was incubated at 65°C for 10 min, and then annealed for 10 min to 25 °C to obtain the 6PBA-si*NFKBIZ* pharmaceutical composition. As shown in FIG. 45, the successful conjugation of 6PBA to *siNFKBIZ* was verified by 20% denaturing PAGE, with a conjugation efficiency greater than 90%.

### 2. Verification of cellular uptake capacity of 6PBA-siNFKBIZ nucleic aicd pharmaceutical composition

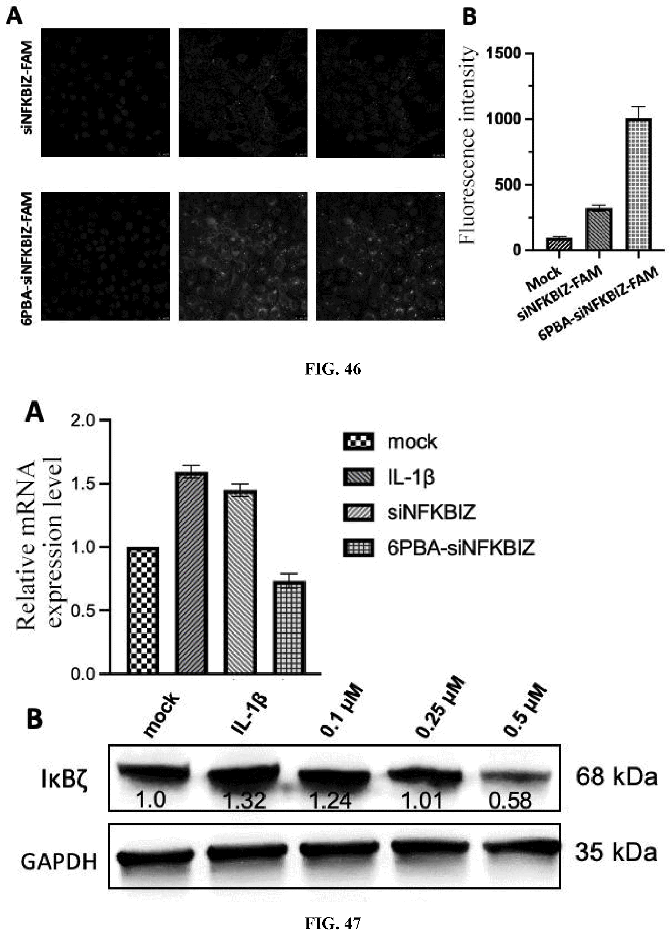

HCEC cells were seeded in a 24-well culture plate at a density of 4 × 10⁴ cells/well, with a clean coverslip placed in each well. After overnight culture, the cells were respectively cultured with 6PBA-si*NFKBIZ*-FAM and si*NFKBIZ*-FAM (at an equivalent FAM concentration of 0.5 µM) for 6 h. Subsequently, the medium was removed, and the cells were washed 3 times with PBS and fixed with 4% paraformaldehyde at room temperature for 15 min. Then, the cells were stained with DAPI for 15 min. After washing with PBS, the cells were observed using a laser scanning confocal microscope. The results are shown in panel A of FIG. 46. The green fluorescence intensity of cells in the 6PBA-si*NFKBIZ*-FAM group was significantly higher than that in the si*NFKBIZ*-FAM-FAM group.

HCEC cells were seeded at 4.0 × 10⁵ cells/well in a 12-well plate and cultured overnight in DMEM. 6PBA-si*NFKBIZ*-FAM (with an equivalent FAM concentration of 0.5 µM) was added to the cells, and the mixture was incubated in Opti-MEM at 37°C for 6 h. si*NFKBIZ*-FAM was used as a control. The cells were collected using trypsin and used for flow cytometry analysis. The results are shown in panel B of FIG. 46. The mean fluorescence intensity of the 6PBA-si*NFKBIZ*-FMA group was about 3 times that of the si*NFKBIZ*-FAM group. The above results indicate that the 6PBA-si*NFKBIZ* pharmaceutical composition has more PBA that can better interact with the glycosyl groups on the cell surface, thereby enhancing the cellular uptake capacity of the si*NFKBIZ* oligonucleotide drug and significantly increasing the intracellular efficiency.

### 3. Evaluation of gene regulatory capacity of 6PBA-siNFKBIZ nucleic acid pharmaceutical composition

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing si*NFKBIZ* and the 6PBA-si*NFKBIZ* pharmaceutical composition for 6 h. After that, the media were replaced with complete DMEM medium. The cells were further cultured at 37°C for 48 h. Next, the cells were lysed using RNAiso reagent to extract mRNA, and the concentration of mRNA was measured by Nanodrop. cDNA was obtained by reverse transcription and the qPCR experiment was performed. The results are shown in panel A of FIG. 47. After stimulation with IL-1β, the expression of the *NFKBIZ* gene in HCEC cells was significantly upregulated to around 1.6 times that of normal cells, while the si*NFKBIZ* nucleic acid drug alone could downregulate the expression to around 1.5 times that of normal cells. In contrast, the 6PBA-si*NFKBIZ* pharmaceutical composition was capable of inhibiting the expression of the *NFKBIZ* gene to around 0.7 times that of normal cells.

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing the 6PBA-si*NFKBIZ* pharmaceutical composition at different concentrations for 6 h. After that, the media were replaced with complete medium, and the cells were further cultured for 48 h. Next, the protein was extracted and the concentration of the protein was measured. The Western-blot experiment was performed. The results are shown in panel B of FIG. 47. The 6PBA-si*NFKBIZ* pharmaceutical composition was capable of effectively inhibiting the expression of IκBζ protein.

### 4. Therapeutic effect of 6PBA-siNFKBIZ nucleic acid pharmaceutical composition on dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 15 specific pathogen-free (SPF) C57BL/6 mice (30 eyes), aged 6-8 weeks, were selected from Shanghai Slac Laboratory Animal Co., Ltd. and housed in an environmentally controlled room with adequate food and water. The mice were randomly divided into 3 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, cyclosporine CsA eye drops (Shenyang Xingqi Pharmaceutical Co., Ltd.), 6PBA-si*NFKBIZ* (at an equivalent siRNA dose of 0.05 mg/kg) into both eyes twice daily (once in the morning and once in the evening) for 14 consecutive days. Tear secretion detection and corneal sodium fluorescein staining assessment were performed on day 0, day 7 and day 14. The specific procedures were performed according to Example 2.

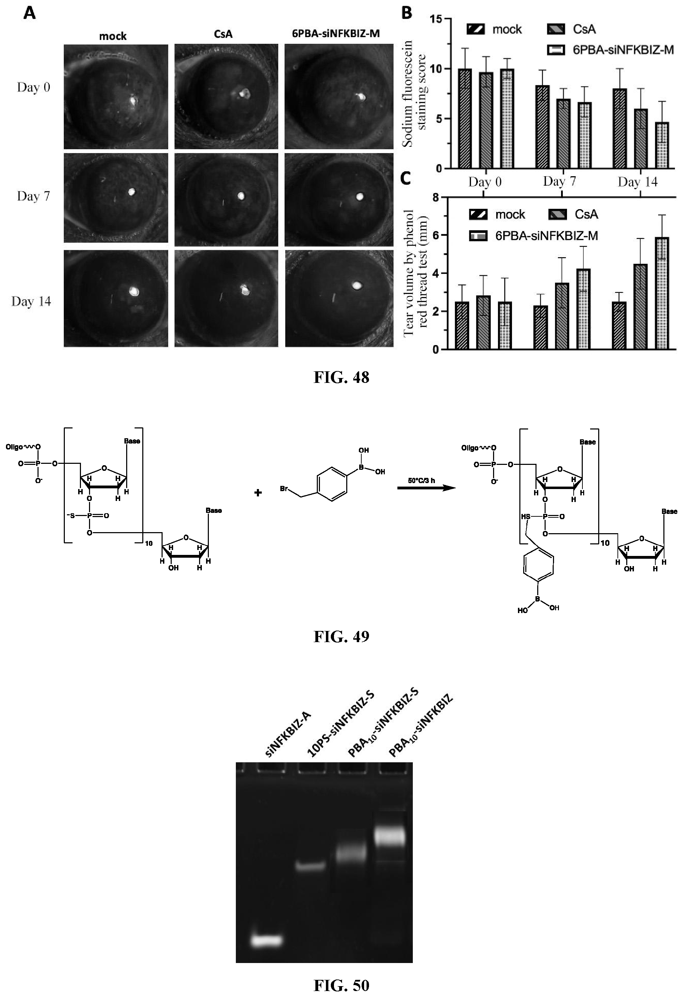

The results are shown in FIG. 48. The results of sodium fluorescein staining showed that, compared to the PBS group, the treatment groups all showed improvement on day 7. On day 14, the ocular score of the CsA eye drop treatment group decreased from around 10 to 5, and the results of the PRTT showed a significant increase in tear secretion. In contrast, the sodium fluorescein score of the 6PBA-si*NFKBIZ*-M treatment group decreased from 10 to around 3.5 , and the wetting length of the phenol red thread increased from 2 mm to around 5.7 mm. In summary, compared to CsA eye drops, the 6PBA-si*NFKBIZ*-M treatment group has a significantly better therapeutic effect within two weeks.

### Example 7

### Decaphenylboronic acid-mediated targeted delivery of siNFKBIZ nucleic acid pharmaceutical composition for the treatment of dry eye disease

### 1. Synthesis and characterization of PBA₁₀-siNFKBIZ nucleic acid pharmaceutical composition

The phosphorothioate-modified small interfering RNA (siRNA) targeting the *NFKBIZ* gene used in this example was purchased from Suzhou Biosyntech Co., Ltd. Twelve T sequences were introduced at the 3' end of the sense strand of the siRNA, and 10 phosphorothioate (PS) backbone modifications were incorporated (10PS-si*NFKBIZ*-S), enabling conjugation with the bromoacetyl bromide group modified on 4-bromomethyl-phenylboronic acid (PBA-Br). The PS modifications on the 10 T sequences extended at the 3' end of the sense strand of *siNFKBIZ* are represented as -10PS in all the examples below. The specific structure of PS is represented by formula 63: 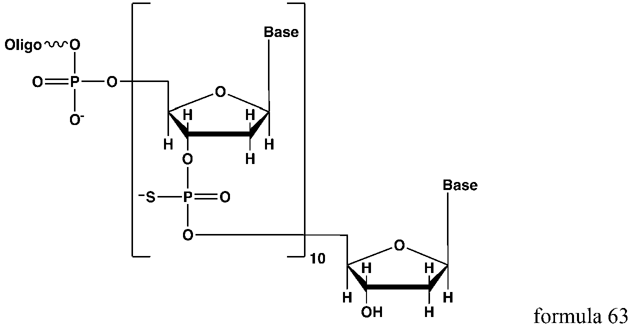 where **Oligo** 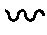 represents an attached nucleic acid molecule.

The sequences of the si*NFKBIZ* nucleic acid drugs used in this example are as follows:
The sequence of small interfering RNA targeting the human *NFKBIZ* gene (si*NFKBIZ*) includes:
Sense: 5'-GCCCGAUUCGUUGUCUGAUTT*T*T*T*T*T*T*T*T*T*T-3' (SEQ ID NO: 435), where * represents a phosphorothioate modification; and
Antisense: 5'-AUCAGACAACGAAUCGGGCcc-3' (SEQ ID NO: 433);
The sequence of small interfering RNA targeting the mouse *NFKBIZ* gene (si*NFKBIZ*-M) includes:
   Sense: 5'-GCGUCAAUGUACCAGUAUUTT*T*T*T*T*T*T*T*T*T*T-3' (SEQ ID NO: 436), where * represents a phosphorothioate modification; and
   Antisense: 5'-AAUACUGGUACAUUGACGCCU-3' (SEQ ID NO: 422).

The synthetic routes of PS and Br-PBA in the synthesis of PBA₁₀-siNKKBIZ are shown in FIG. 49.

Specific synthetic steps: 4-bromomethyl-phenylboronic acid was taken to prepare a 60 mM solution in DMSO. Then, the solution was mixed with 200 µM 10PS-si*NFKBIZ* at a volume ratio of 1 : 1, and the mixture was reacted in a metal bath at 50°C for 3 h. Then, small molecules were removed by extraction with ethyl acetate, and dimethyl sulfoxide was removed by dialysis to obtain the product PBA₁₀-si*NFKBIZ*-S, which was then quantified and concentrated to dryness by rotary evaporation. Finally, PBA₁₀-si*NFKBIZ*-S and si*NFKBIZ*-A were subjected to complementary base pairing in 1× PBS to assemble into PBA₁₀-siNKKBIZ. Verification was performed using a non-denaturing 10% polyacrylamide gel. The results are shown in FIG. 50. The results showed that PBA was successfully modified on the si*NFKBIZ*-S strand, which successfully hybridized with si*NFKBIZ*-A.

### 2. Cellular uptake capacity of PBA₁₀-siNFKBIZ nucleic acid pharmaceutical composition

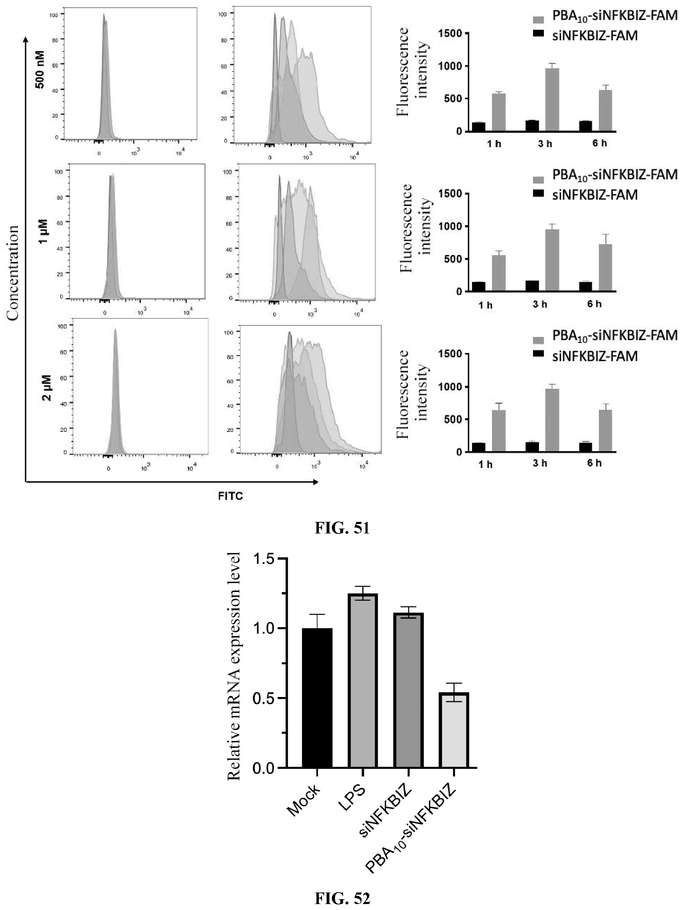

HCEC cells were seeded at 4.0 × 10⁵ cells/well in a 12-well plate and cultured overnight in DMEM. PBA₁₀-si*NFKBIZ*-FAM (with an equivalent FAM concentration of 0.5 µM) was added to the cells, and the mixture was incubated in Opti-MEM at 37°C for 1 h, 3 h and 6 h. si*NFKBIZ*-FAM was used as a control. The cells were collected using trypsin and used for flow cytometry analysis. The results are shown in FIG. 51. The uptake of the PBA₁₀-si*NFKBIZ*-FAM pharmaceutical composition by HCEC cells was time-dependent at 1 h and 3 h, with the uptake amount at 3 h being about 6 times that of the si*NFKBIZ*-FAM drug. In summary, the results indicate that extending and modifying one of the strands of the si*NFKBIZ* nucleic acid drug with PBA can still enhance the uptake capacity of the si*NFKBIZ* oligonucleotide drug and improve the cellular delivery efficiency.

### 3. Regulatory effect of PBA₁₀-siNFKBIZ nucleic acid pharmaceutical composition on the NFKBIZ gene

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing si*NFKBIZ* and the PBA₁₀-si*NFKBIZ* nucleic acid pharmaceutical composition (with an equivalent siRNA concentration of 400 nM) for 6 h. After that, the media were replaced with complete medium, and the cells were further cultured for 48 h. Next, the total RNA was extracted and the concentration of the mRNA was measured. cDNA was obtained by reverse transcription and the qPCR experiment was performed. The results are shown in FIG. 52. After stimulation with LPS, the expression of the *NFKBIZ* gene in HCEC cells was significantly upregulated to around 1.2 times that of normal cells. The PBA₁₀-si*NFKBIZ* pharmaceutical composition was capable of effectively inhibiting the expression of the *NFKBIZ* gene in a concentration-dependent manner. At a concentration of 400 nM, the *NFKBIZ* gene was inhibited to around 50% relative to the LPS-stimulated group. The above results indicate that the PBA₁₀-si*NFKBIZ* pharmaceutical composition can effectively promote the intracellular delivery of the si*NFKBIZ* nucleic acid drug, thereby exerting a regulatory effect on the *NFKBIZ* gene, and achieving a good gene inhibitory effect.

### 4. Therapeutic effect of PBA₁₀-siNFKBIZ nucleic acid pharmaceutical composition on dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 15 specific pathogen-free (SPF) C57BL/6 mice (30 eyes), aged 6-8 weeks, were selected from Shanghai Slac Laboratory Animal Co., Ltd. and housed in an environmentally controlled room with adequate food and water. The mice were randomly divided into 3 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, CsA eye drops (Shenyang Xingqi Pharmaceutical Co., Ltd.), PBA₁₀-si*NFKBIZ*-M (at an equivalent siRNA dose of 0.05 mg/kg) into both eyes twice daily (once in the morning and once in the evening) for 14 consecutive days. Tear secretion detection and corneal sodium fluorescein staining assessment were performed on day 0, day 7 and day 14. The specific procedures were performed according to Example 2.

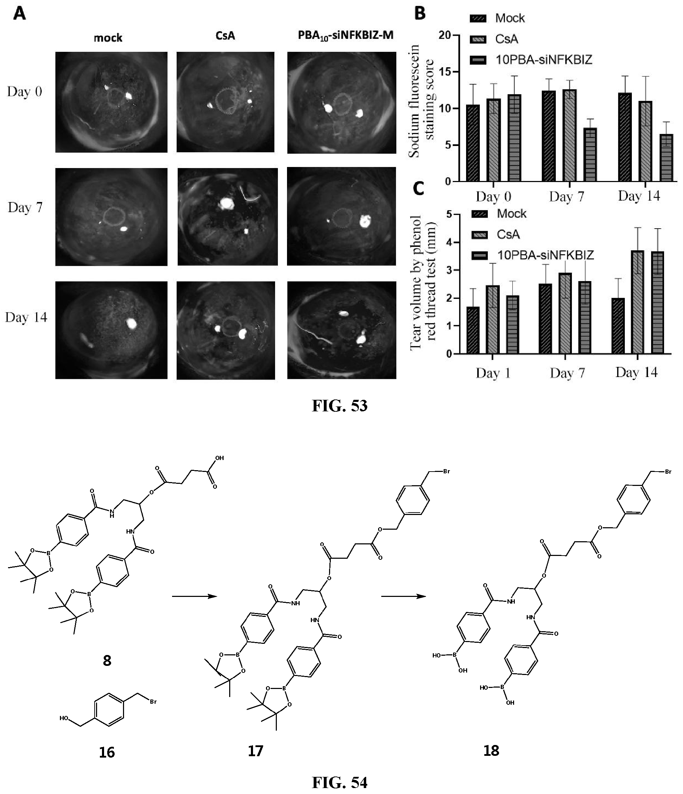

The results are shown in FIG. 53. After 14 days of treatment, significant changes were observed in ocular damage in both the CsA eye drops group and the PBA₁₀-si*NFKBIZ*-M pharmaceutical composition group. The ocular score of the mice in the CsA treatment group decreased from 12 to around 8, and the results of the PRTT showed an increase from 2.4 mm to around 3.25 mm. The ocular sodium fluorescein score of the mice in the PBA₁₀-si*NFKBIZ*-M pharmaceutical composition group was decreased from 11 to around 5.4, and the wetting length of the phenol red thread increased from 2.0 mm to around 3.35 mm. The results of the animal experiments that, compared to the CsA eye drops group, the PBA₁₀-si*NFKBIZ*-M pharmaceutical composition group has a more significant therapeutic effect on dry eye disease in mice.

### Example 8

### Hexaphenylboronic acid-mediated targeted delivery of stem-loop siNFKBIZ-based nucleic acid pharmaceutical composition for the treatment of dry eye disease

### 1. Synthesis and characterization of PBA₆-siNFKBIZ nucleic acid pharmaceutical composition

The synthetic route of 2PBA-Br in this example is shown in FIG. 54.

### 1.1 Synthesis of compound 17

Compound 8 (776 mg) and compound 16 (200 mg) were added to a 100 mL flask, and 20 mL of dichloromethane was added with stirring until dissolved. EDCI (285 mg) and DMAP (20 mg) were added, and the reaction was performed at room temperature for 20 h under nitrogen atmosphere. After the reaction, dichloromethane was removed by distillation under reduced pressure. 30 mL of ethyl acetate was added, and the mixture was washed twice with 30 mL of water (pH = 3), twice with 30 mL of saturated sodium bicarbonate solution, and then once with 30 mL of saturated sodium chloride solution. The organic phase was collected, dried over anhydrous sodium sulfate, and then distilled under reduced pressure to obtain a white foamy solid, which was directly used for the subsequent reaction.

### 1.2 Synthesis of compound 18

Compound 17 was added to a dry 100 mL round-bottom flask, and 20 mL of acetone was added with stirring until dissolved. Sodium periodate (852 mg) and ammonium acetate (310 mg) were dissolved in 20 mL of water, and the solution was then added to the acetone solution. The reaction was performed at room temperature for 10 h under nitrogen atmosphere. After the reaction, acetone was removed by distillation under reduced pressure. Next, 100 mL of ethyl acetate was added, and the mixture was washed twice with 100 mL of water, and then once with 100 mL of saturated sodium chloride solution. The organic phase was collected, dried over anhydrous sodium sulfate, and then distilled under reduced pressure to obtain a pale yellow oily liquid. Purification was performed by reversed-phase column chromatography to obtain 486 mg of a white solid, i.e., compound 18, two-step yield: about 73%.

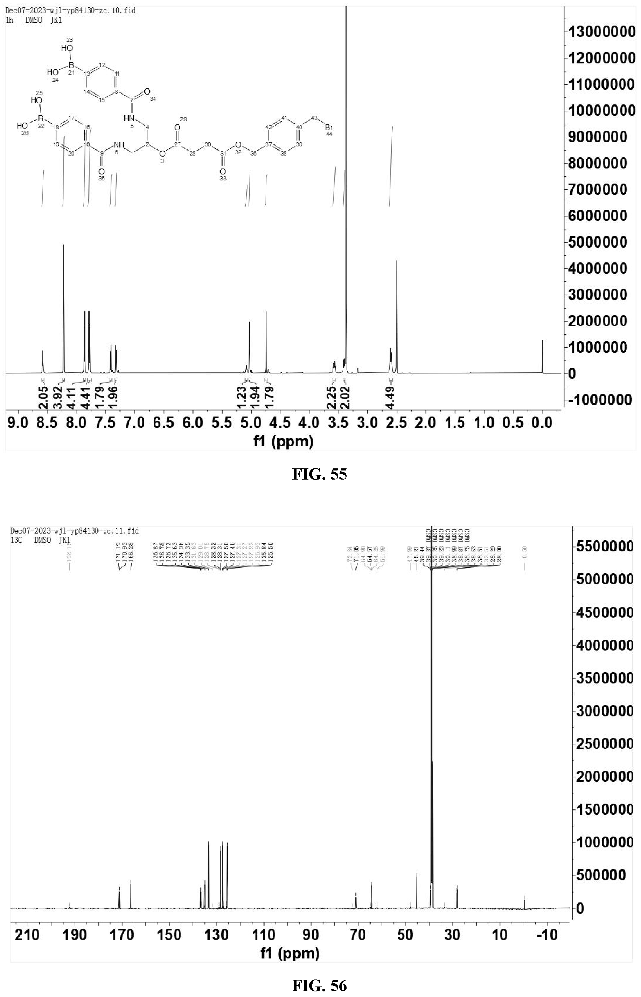

The ¹H NMR spectrum of compound 18 is shown in FIG. 55. The solvent used for the nuclear magnetic spectroscopy test was DMSO-d6, and the assignment of the proton peaks was as follows: ¹ H NMR (700 MHz, DMSO-d6) δ 8.58 (t, J = 6.0 Hz, 2H), 8.22 (s, 4H), 7.87 (d, J = 7.7 Hz, 4H), 7.81 - 7.74 (m, 4H), 7.41 (d, J = 7.9 Hz, 2H), 7.32 (d, J = 8.0 Hz, 2H), 5.09 (qd, J = 6.4, 3.2 Hz, 1H), 5.03 (s, 2H), 4.74 (s, 2H), 3.58 (dt, J = 13.9, 5.5 Hz, 2H), 3.42 - 3.39 (m, 2H), 2.63 - 2.57 (m, 4H). The ¹³C NMR spectrum of compound 18 is shown in FIG. 56. The solvent used for the nuclear magnetic spectroscopy test was DMSO-D6, and the assignment of the characteristic carbons contained in the product was as follows: ¹³C NMR (176 MHz, DMSO-d6) δ 166.28, 136.87, 134.96, 133.35, 128.32, 127.50, 125.50, 71.05, 64.57, 45.21, 39.44, 38.63, 38.51, 28.29, 28.00.

### 1.3 Preparation of PBA₆-siNFKBIZ nucleic acid pharmaceutical composition

The phosphorothioate-modified small interfering RNA (siRNA) targeting the *NFKBIZ* gene used in this example was purchased from Suzhou Biosyntech Co., Ltd. A stem-loop structure was formed by 16 bases at the 3' end of the sense strand of the siRNA, and three phosphorothioate (PS) backbone modifications were incorporated at the loop structure site (3PS-si*NFKBIZ*-S), enabling the conjugation of the RNA with a bromo group modified on bromo-diphenylboronic acid (2PBA-Br).

The sequences of the si*NFKBIZ* nucleic acid drugs used in this example are as follows:
The sequence of small interfering RNA targeting the human *NFKBIZ* gene (si*NFKBIZ*) includes:
Sense: 5'-GCCCGAUUCGUUGUCUGAUGCAGCCG*A*A*AGGCUGC-3' (SEQ ID NO: 437); and
Antisense: 5'-AUCAGACAACGAAUCGGGCcc-3' (SEQ ID NO: 433).

The sequence of small interfering RNA targeting the mouse *NFKBIZ* gene (si*NFKBIZ*-M) includes: Sense: 5'-GCGUCAAUGUACCAGUAUUGCAGCCG*A*A*AGGCUGC-3' (SEQ ID NO: 438); and Antisense: 5'-AAUACUGGUACAUUGACGCCU-3' (SEQ ID NO: 422).

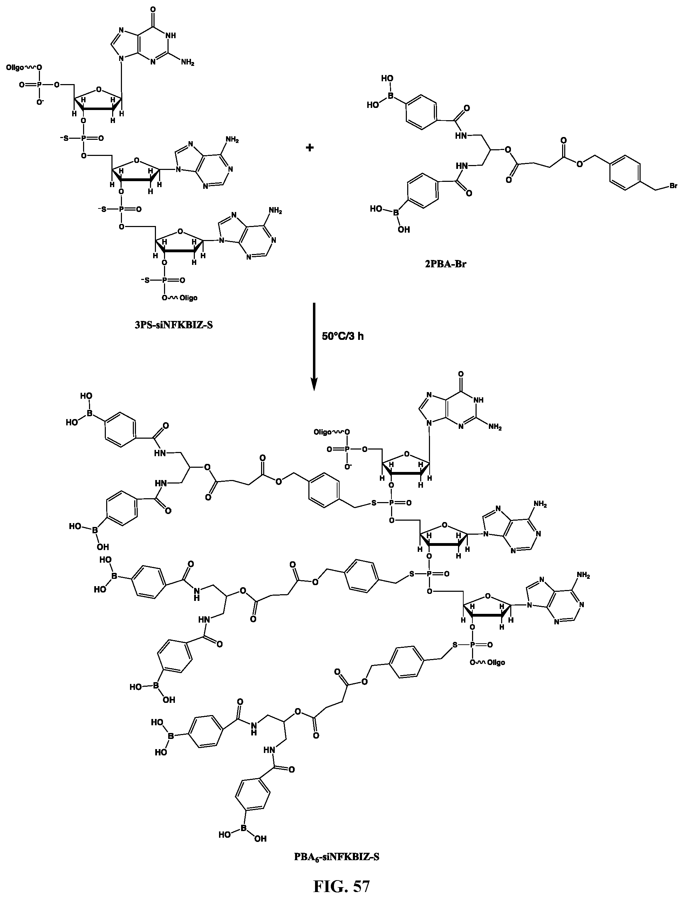

The synthetic route of PBA₆-siNKKBIZ is shown in FIG. 57.

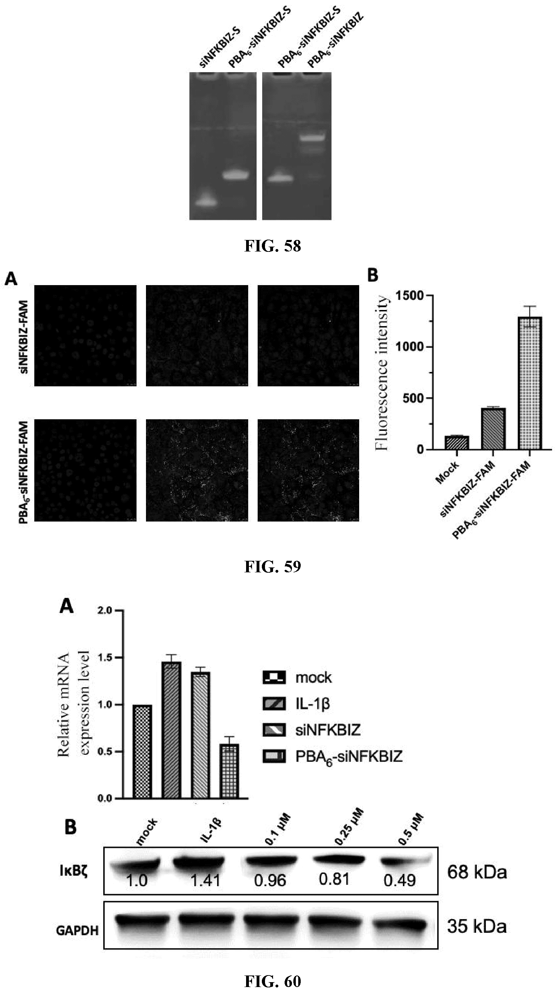

Specific synthetic steps: Compound 18 was taken to prepare a 20 mM solution in DMSO. Then, the solution was mixed with 200 µM 3PS-si*NFKBIZ* at a volume ratio of 1 : 1, and the mixture was reacted in a metal bath at 50°C for 3 h. Then, small molecules were removed by extraction with ethyl acetate, and dimethyl sulfoxide was removed by dialysis to obtain the product PBA₆-si*NFKBIZ*-S, which was then quantified and concentrated to dryness by rotary evaporation. Finally, PBA₆-si*NFKBIZ*-S and si*NFKBIZ*-A were subjected to complementary base pairing in 1× PBS to assemble into PBA₆-siNKKBIZ. Verification was performed using a non-denaturing 20% polyacrylamide gel. The results are shown in FIG. 58. The results showed that PBA was successfully modified on the si*NFKBIZ*-S strand, which successfully hybridized with si*NFKBIZ*-A.

### 2. Cellular uptake capacity of PBA₆-siNFKBIZ nucleic acid pharmaceutical composition

HCEC cells were seeded in a 24-well culture plate at a density of 4 × 10⁴ cells/well, with a clean coverslip placed in each well. After overnight culture, the cells were respectively cultured with PBA₆-si*NFKBIZ*-FAM and siRNA-FAM (at an equivalent FAM concentration of 0.5 µM) for a total of 4 h. Subsequently, the medium was removed, and the cells were washed 3 times with PBS and fixed with 4% paraformaldehyde at room temperature for 15 min. Then, the cells were stained with DAPI for 15 min. After washing with PBS, the cells were observed using a laser scanning confocal microscope. The results are shown in panel A of FIG. 59. The green fluorescence intensity of cells in the PBA₆-si*NFKBIZ*-FAM group was significantly higher than that in the si*NFKBIZ*-FAM group.

HCEC cells were seeded at 4.0 × 10⁵ cells/well in a 12-well plate and cultured overnight in DMEM. PBA₆-si*NFKBIZ*-FAM was added to the cells at a final FAM concentration of 0.5 µM per well, and the mixture was incubated in Opti-MEM at 37°C for 6 h. si*NFKBIZ*-FAM was used as a control. The cells were collected using trypsin and used for flow cytometry analysis. The results are shown in panel B of FIG. 59. The uptake of PBA₆-si*NFKBIZ*-FAM by HCEC cells was about 3.2 times that of the si*NFKBIZ*-FAM drug. In summary, the results indicate that the PBA modification on the stem-loop structure of the si*NFKBIZ* nucleic acid drug enhances the uptake capacity of the si*NFKBIZ* oligonucleotide drug and improves the cellular delivery efficiency.

### 3. Regulatory effect of PBA₆-siNFKBIZ nucleic acid pharmaceutical composition on the NFKBIZ gene

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing si*NFKBIZ* and the PBA₆-si*NFKBIZ* nucleic acid pharmaceutical composition for 6 h. After that, the media were replaced with complete medium, and the cells were further cultured for 48 h. Next, the total RNA was extracted and the concentration of the mRNA was measured. cDNA was obtained by reverse transcription and the qPCR experiment was performed. The results are shown in panel A of FIG. 60. After stimulation with IL-1β, the expression of the *NFKBIZ* gene in HCEC cells was significantly upregulated to around 1.5 times that of normal cells. The PBA₆-si*NFKBIZ* nucleic acid pharmaceutical composition was capable of effectively inhibiting the expression of the *NFKBIZ* gene, which could be inhibited by around 50%.

HCEC cells were seeded into a 6-well plate at a density of 1 × 10⁵ cells/well and cultured overnight. Then, after 12 h of stimulation with IL-1β, the cells were washed twice with PBS. Next, the cells were incubated in Opti-MEM media respectively containing the PBA₆-si*NFKBIZ* pharmaceutical composition at different concentrations for 6 h. After that, the media were replaced with complete medium, and the cells were further cultured for 48 h. Total protein was extracted from the cells using RIPA buffer containing a protease inhibitor cocktail, the protein concentration was determined, and the Western-blot experiment was performed. The results are shown in panel B of FIG. 60. The PBA₆-si*NFKBIZ* nucleic acid pharmaceutical composition had a concentration-dependent inhibitory effect on the IκBζ protein, and could effectively inhibit the expression of the IκBζ protein at a concentration of 500 nM. The above results indicate that the PBA modification at the end of the stem-loop structure can effectively promote the intracellular delivery of the si*NFKBIZ* nucleic acid drug, thereby enabling the regulatory effect of the *NFKBIZ* gene, inhibiting the expression of IκBζ protein, and achieving a good anti-inflammatory effect.

### 4. Therapeutic effect of PBA₆-siNFKBIZ nucleic acid pharmaceutical composition on dry eye disease

All animal experiments in this example were performed according to the guidelines in the "Statement for the Use of Animals in ocular and Vision Research" by the Association for Research in Vision and Ophthalmology (ARVO), and complied with relevant operational standards. All animal experiments were performed according to the guidelines established by the Ethics Committee of Shanghai Jiao Tong University. In this example, 15 specific pathogen-free (SPF) C57BL/6 mice (30 eyes), aged 6-8 weeks, were selected from Shanghai Slac Laboratory Animal Co., Ltd. and housed in an environmentally controlled room with adequate food and water. The mice were randomly divided into 3 groups and acclimated to a low-humidity environment (RH = 18.5% ± 5.1%, AF = 15 L/min, T = 21-23°C) for 4 days. Then, 5 µL of 0.2% benzalkonium chloride was administered to each eye twice daily for 14 consecutive days to establish a dry eye disease mouse model. The groups of mice respectively received topical instillation of PBS, cyclosporine CsA eye drops (Shenyang Xingqi Pharmaceutical Co., Ltd.), PBA₆-si*NFKBIZ* (at an equivalent siRNA dose of 0.05 mg/kg) into both eyes twice daily (once in the morning and once in the evening) for 14 consecutive days. Tear secretion detection and corneal sodium fluorescein staining assessment were performed on day 0, day 7 and day 14. The specific procedures were performed according to Example 2.

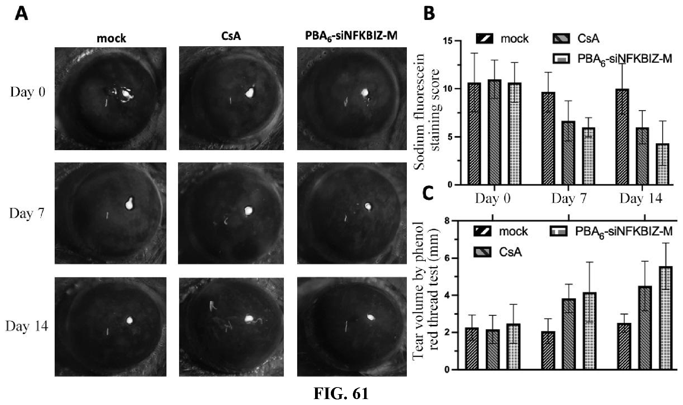

The results are shown as in FIG. 61. After 14 days of treatment, significant changes were observed in ocular damage in both the CsA eye drops group and the PBA₆-si*NFKBIZ* pharmaceutical composition group. The ocular score of the mice in the CsA treatment group decreased from 11 to around 6, and the results of the PRTT showed an increase from 2.1 mm to around 4.5 mm. The ocular sodium fluorescein score of the mice in the PBA₆-si*NFKBIZ* pharmaceutical composition group was decreased from 11 to around 4, and the wetting length of the phenol red thread increased from 2.6 mm to around 5.6 mm. It can be seen from the results of the animal experiments that, compared to the model group and the CsA eye drops group, the PBA₆-si*NFKBIZ* pharmaceutical composition group has a more significant therapeutic effect on dry eye disease in mice.

The foregoing descriptions are merely preferred embodiments of the present disclosure, and it should be noted that for those of ordinary skill in the art, without departing from the principles of the present disclosure, some improvements and refinements may also be made, which should also be considered as the scope of protection of the present disclosure.

## Claims

1. A pharmaceutical composition for inhibiting nuclear factor of kappa light polypeptide gene enhancer in B-cells inhibitor, zeta (*NFKBIZ*) gene expression, comprising a nucleic acid molecule inhibiting *NFKBIZ* gene expression and a targeting ligand targeting an ocular tissue-specific protein.

2. The pharmaceutical composition according to claim 1, wherein the nucleic acid molecule is a double-stranded nucleic acid molecule composed of a sense strand and an antisense strand;
the nucleic acid molecule comprises at least one nucleotide sequence of the following items A and B:
A: a sense strand selected from at least one of the following numbered nucleotide sequences: SEQ ID NO: 1 to SEQ ID NO: 18, SEQ ID NO: 25 to SEQ ID NO: 49, SEQ ID NO: 51 to SEQ ID NO: 89, SEQ ID NO: 93 to SEQ ID NO: 108, SEQ ID NO: 113 to SEQ ID NO: 132, SEQ ID NO: 134 to SEQ ID NO: 135, SEQ ID NO: 138, SEQ ID NO: 143 to SEQ ID NO: 152, SEQ ID NO: 154 to SEQ ID NO: 161, SEQ ID NO: 169 to SEQ ID NO: 185, and SEQ ID NO: 421; and
an antisense strand selected from at least one of the following numbered nucleotide sequences: SEQ ID NO: 211 to SEQ ID NO: 228, SEQ ID NO: 235 to SEQ ID NO: 259, SEQ ID NO: 261 to SEQ ID NO: 299, SEQ ID NO: 303 to SEQ ID NO: 318, SEQ ID NO: 323 to SEQ ID NO: 342, SEQ ID NO: 344 to SEQ ID NO: 345, SEQ ID NO: 348, SEQ ID NO: 353 to SEQ ID NO: 362, SEQ ID NO: 364 to SEQ ID NO: 371, SEQ ID NO: 379 to SEQ ID NO: 395, and SEQ ID NO: 422; and
B: nucleotide sequences having 2-3 consecutive or non-consecutive bases added, deleted or substituted based on the nucleotide sequences in item A, and targets the *NFKBIZ* gene to exert an inhibitory effect.

3. The pharmaceutical composition according to claim 2, wherein the sense strand or the antisense strand of the nucleic acid molecule further comprises an overhang at an end.

4. The pharmaceutical composition according to claim 3, wherein the overhang is 1-3nt in length.

5. The pharmaceutical composition according to any one of claims 1-4, wherein the nucleic acid molecule comprises any one or more of the following modified nucleotides: deoxynucleotides, 3'-deoxythymidine nucleotides, 2'-O-methyl modified nucleotides, 2'-fluoro-modified nucleotides, 2'-deoxy-modified nucleotides, locked nucleic acids, conformationally restricted nucleotides, constrained ethyl nucleotides, abasic nucleotides, 2'-amino-modified nucleotides, 2'-O-allyl-modified nucleotides, 2'-C-alkyl-modified nucleotides, 2'-hydroxy-modified nucleotides, 2'-methoxyethyl-modified nucleotides, 2'-O-alkyl-modified nucleotides, morpholino nucleotides, phosphoramidate-modified nucleotides, nucleotides containing unnatural bases, tetrahydropyran-modified nucleotides, 1,5-anhydrohexitol-modified nucleotides, cyclohexenyl-modified nucleotides, nucleotides containing phosphorothioate linkages, nucleotides containing methylphosphonate linkages, nucleotides containing 5'-phosphate groups, and nucleotides containing 5'-phosphate mimics.

6. The pharmaceutical composition according to claim 1, wherein the targeting ligand is selected from the group consisting of an aptamer targeting an ocular tissue-specific protein, a compound modified with boronic acid or the derivative thereof, and a compound modified with phenylboronic acid or the derivative thereof.

7. The pharmaceutical composition according to claim 6, wherein the ocular tissue-specific protein comprises at least one of: mucin, integrin, and CD44.

8. The pharmaceutical composition according to claim 7, wherein the mucin comprises at least one of: MUC-1, MUC-4, and MUC-16; and
the integrin comprises at least one of: α_{V}β₁, α_{V}β₃, α_{V}β₆, α₅β₁, α₆β₁, α_{V}β₁, α_{V}β₅, α₆β₄, and α₁β₁.

9. The pharmaceutical composition according to claim 8, wherein the aptamer targeting the mucin MUC-1 has the nucleotide sequence of SEQ ID NO: 423;
the aptamer targeting the mucin MUC-16 has the nucleotide sequence of SEQ ID NO: 424;
the aptamer targeting the integrin α_{V}β₃ has the nucleotide sequence of SEQ ID NO: 425; and
the aptamer targeting CD44 has the nucleotide sequence of SEQ ID NO: 426.

10. The pharmaceutical composition according to claim 1, wherein the aptamer further comprises a stability-enhancing modification;
the stability-enhancing modification comprises at least one of: phosphorothioate backbone modification, 2'-O-methyl modification, 2'-methoxyethyl modification, 2'-fluoro modification, inverted deoxythymidine (dT) modification, dT modification, and terminal conjugation with polyethylene glycol.

11. The pharmaceutical composition according to claim 6, wherein the compound modified with boronic acid or the derivative thereof, or the compound modified with phenylboronic acid or the derivative thereof, is a linear or branched compound terminally modified with boronic acid or phenylboronic acid or a derivative thereof;
the linear or branched compound is modified with 1-40 moieties of boronic acid or the derivative thereof, or phenylboronic acid or the derivative thereof.

12. The pharmaceutical composition according to claim 11, wherein the compound modified with boronic acid or the derivative thereof, or the compound modified with phenylboronic acid or the derivative thereof, comprises any one of formulas 1-10: 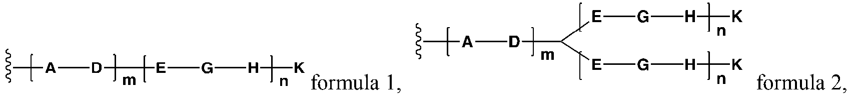 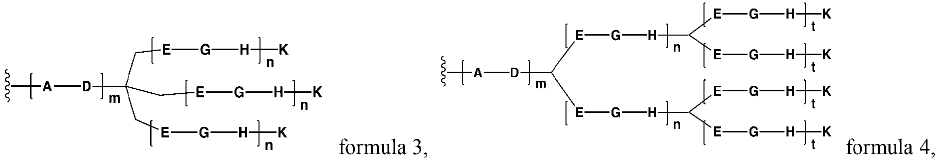 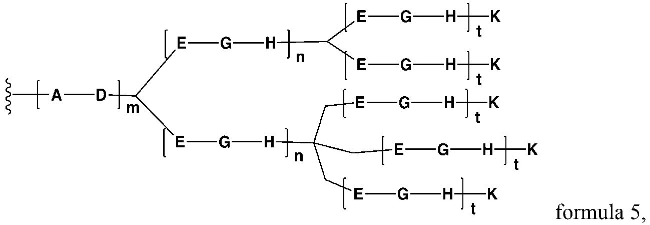 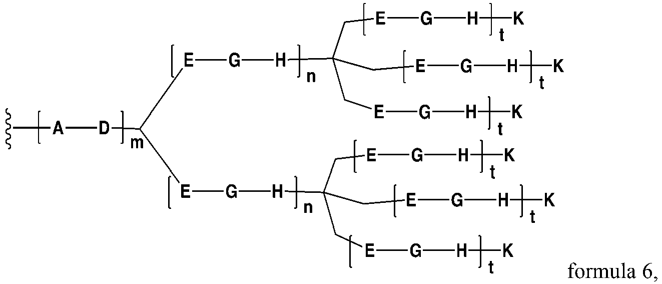 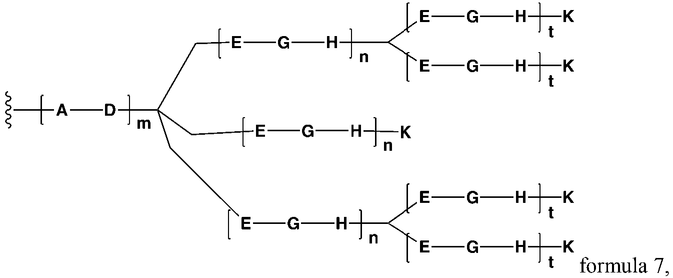 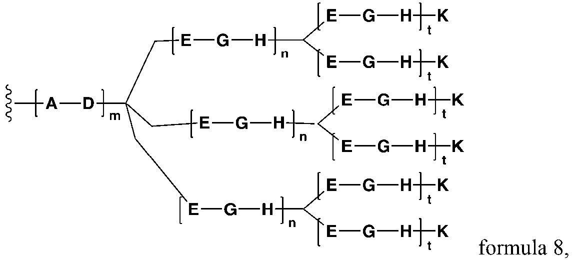 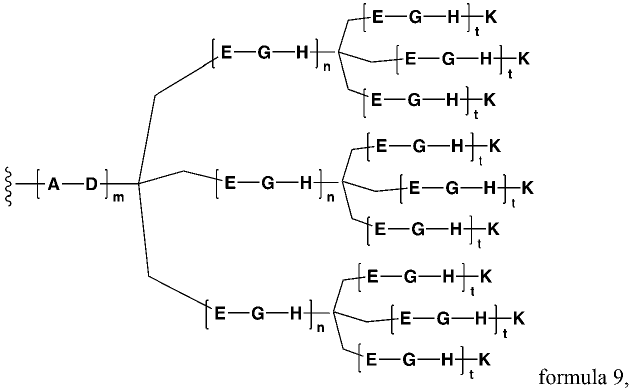 and 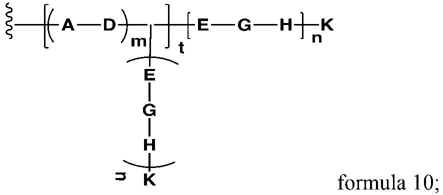
in formula 1 to formula 10, A and G are each independently absent, -(CH₂)ₕ-, or -(CH₂)ₕ- having one or more methylene groups substituted with an M group; the h is 0-15; the M group comprises: one or more of -O-, -S-, -C(O)-, -C(O)O-, -C(O)N_{H}-, -CH(T^{C})-, -C(T') (T")-, -NH-, -N(T^{N})-, -S-S-, -C(T')=C(T")-, -C≡C-, 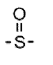 and 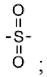 T^{C}, T^{N}, T' and T" in the M group each indicate that any one or more hydrogen atoms on a specified atom, comprising a carbon atom or a nitrogen atom, are replaced by an L group, provided that the normal valency of the specified atom is not exceeded and the substitution results in a stable compound; the L group comprises any one of: C1-C6 alkyl, C2-C6 alkenyl, C2-C6 alkynyl, cyano, hydroxyl, carboxyl, cycloalkyl, cycloalkenyl, heterocyclyl, heteroaryl, aryl, ketone, alkoxycarbonyl, aryloxycarbonyl, heteroaryloxycarbonyl or halogen; the halogen comprises F, Cl, Br or I; (O) in the A represents a carbonyl oxygen atom;
D, E, and H are each independently absent or one or more of -O- , -S-, -C(O)-, -NH-, -CH₂-, -C(O)NH-, -NHC(O)-, -C(O)O-, -OC(O)-, -OC(O)O-, -OC(O)NH-, -NHC(O)O-, 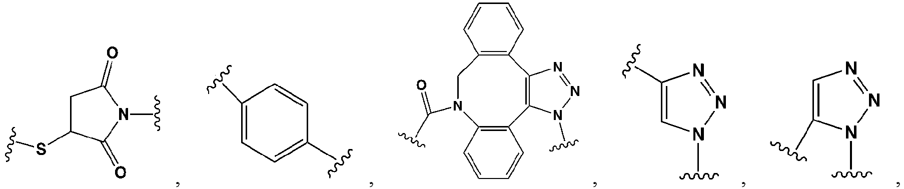 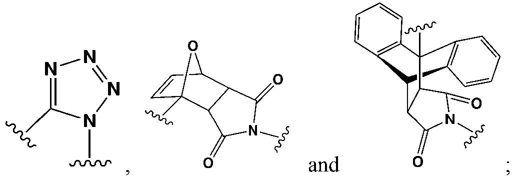 (O) in the D, E, and H groups represents a carbonyl oxygen atom; the 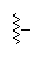 represents an attachment position of the corresponding formula;the A, G, D, E, and H are not simultaneously absent; and
K represents boronic acid or a derivative thereof, or phenylboronic acid and a derivative thereof; m, n and t are independently 1-15.

13. The pharmaceutical composition according to any one of claims 6-12, wherein the targeting ligand is directly covalently conjugated to an end of the nucleic acid molecule.

14. The pharmaceutical composition according to claim 13, wherein when the targeting ligand is an aptamer, a phosphate group of the aptamer is covalently conjugated to a ribose of the nucleic acid molecule.

15. The pharmaceutical composition according to claim 13, wherein when the targeting ligand is a compound modified with boronic acid or a derivative thereof, or a compound modified with phenylboronic acid or a derivative thereof, the targeting ligand is covalently conjugated to a phosphate group or base at an end of the nucleic acid molecule;
when covalently conjugated to the phosphate group at the end of the nucleic acid molecule, the targeting ligand is conjugated via an X group; the X group represents -O- or -S-.

16. The pharmaceutical composition according to any one of claims 6-12, wherein the targeting ligand is covalently conjugated to the nucleic acid molecule via an extension sequence linked to a terminus of the nucleic acid molecule.

17. The pharmaceutical composition according to claim 16, wherein the extension sequence comprises an extension sequence having only one end linked to a terminus of the nucleic acid molecule and/or an extension sequence that forms a stem-loop structure by being linked to the 3' end of one strand of the nucleic acid molecule at one end, while being linked or not linked to the 5' end of the complementary strand of the nucleic acid molecule at the other end.

18. The pharmaceutical composition according to claim 17, wherein the extension sequence is 1-40nt in length; the extension sequence is a random nucleotide sequence.

19. The pharmaceutical composition according to claim 18, wherein when the targeting ligand is an aptamer, a phosphate group of the aptamer is covalently conjugated to a ribose of the extension sequence.

20. The pharmaceutical composition according to claim 18, wherein when the targeting ligand is a compound modified with boronic acid or a derivative thereof, or a compound modified with phenylboronic acid or a derivative thereof, the targeting ligand is covalently coupled to one or more phosphate groups or bases on the extension sequence;
when covalently conjugated to the phosphate groups of the extension sequence, the targeting ligand is conjugated via an X group; the X group represents -O- or -S-.

21. A drug for treating an ocular disease, comprising the pharmaceutical composition according to any one of claims 1 to 20 as an active ingredient.

22. Use of the pharmaceutical composition according to any one of claims 1 to 20 in the preparation of a drug for preventing and/or treating an ocular disease.

23. Use of the pharmaceutical composition according to any one of claims 1 to 20 in the prevention and/or treatment of an ocular disease.

24. The use according to claim 22 or 23, wherein the ocular disease comprises one or more of: dry eye, keratitis, conjunctivitis and blepharitis.

25. A method for treating an ocular disease, comprising instilling the pharmaceutical composition according to any one of claims 1 to 20 or a drug prepared from the pharmaceutical composition into the eye of a patient.

26. The method according to claim 24, wherein the pharmaceutical composition or drug is instilled twice daily; the two instillations have a time interval of 10-14 h.
